# EUROPEAN PATENT APPLICATION

(11) **EP 2 743 232 A1**
(43) Date of publication of application: **18.06.2014**
(21) Application number: 12821931.8
(22) Date of filing: 08.08.2012
(51) Int. Cl.: C01B 31/02, C01G 9/02, C01G 23/04, C01G 49/02, C07K 19/00, C12N 15/09, H01L 31/04, H01M 4/96, H01M 14/00

(54) **POROUS STRUCTURE AND METHOD FOR PRODUCING SAME**

(30) Priority: 08.08.2011 JP 2011173229; 20.12.2011 JP 2011278816
(71) Applicant: Ajinomoto Co., Inc., Tokyo 104-8315 (JP)
(72) Inventor: INOUE, Ippei, Kanagawa 210-8681 (JP); YAMASHITA, Ichiro, Nara 630-0192 (JP); ZHENG, Bin, Nara 630-0192 (JP); YASUEDA, Hisashi, Kanagawa 210-8681 (JP); URAOKA, Yukiharu, Nara 630-0192 (JP); ISHIKAWA, Yasuaki, Nara 630-0192 (JP)
(74) Representative: Nicholls, Kathryn Margaret
(86) International application number: PCT/JP2012/070280
(87) International publication number: WO 2013/022051

(57) **Abstract**

A functional material having excellent photocatalytic activity, electric characteristics and the like is provided. A porous structure body 10 comprises a first target material 20 and an aggregate body 30 formed by aggregation of the first material. The aggregate body 30 adheres to the first target material and is located so as to surround the first target material. The aggregate body has a plurality of first pores 32 unevenly distributed near the first target material in the aggregate body and a plurality of second pores 34 scattered over the aggregate body.

## Description

### TECHNICAL FIELD

The present invention relates to a porous structure body. More specifically, the present invention relates to a porous structure body having useful characteristics such as conductivity and photocatalytic characteristics, to an electronic element using the porous structure body as a functional material, and to methods for producing the same.

### BACKGROUND ART

For example, when titanium oxide is irradiated with light, the light energy causes electrons with reducing power and holes with oxidizing power to be generated on the surface of titanium oxide particles. There has been an attempt to use titanium oxide as a functional material that utilizes the reducing power of electrons and the oxidizing power of holes, as a functional material having functions such as an antimicrobial function, a deodorization function, an air purification function, an antifouling function, a hydrogen generation function, and an electric charge separation function (Non Patent Document 1).

For example, by oxidizing hydroxide ions using the oxidizing power generated on the surface of titanium oxide particles irradiated with light, radicals with strong oxidizing power can be generated. Such radicals can have, because of their oxidizing power, a sterilization function, a deodorization function of decomposing odorants such as acetaldehyde and ammonia, an air purification function of decomposing hazardous materials such as NOx and formaldehyde in the air, and an antifouling function of decomposing dust and the like.

There has also been an attempt to electrolyze water using the generated oxidizing-reducing power to generate oxygen and hydrogen so that the hydrogen is used as clean energy. In addition, by extracting the electrons generated in the titanium oxide particles irradiated with light to the outside, the functional material can operate as a solar cell.

To further improve the performance of the functional material that uses titanium oxide, it is contemplated that the surface area of the functional material that uses titanium oxide is increased or its electric characteristic are improved.

More specifically, by increasing the surface area of the functional material that uses titanium oxide, the total number of electrons having reducing power and holes having oxidizing power that are generated by the energy of the applied light can be increased. By improving the electrical characteristics such as conductivity, the probability of recombination of electrons and holes excited by the applied light can be reduced. As a result, a larger amount of electric charge can be obtained, so that stronger oxidizing power can be achieved.

One known technique for increasing the surface area of a functional material that uses titanium oxide is to use titanium oxide formed into fine particles. More specifically, a titanium ion complex is hydrolyzed to produce nanoparticles of titanium oxide (Patent Document 1). Another known method is to form titanium oxide nanoparticles or a film of titanium oxide nanoparticles using a pulsed laser (Patent Document 3). In addition, a technique is known in which titanium oxide nanoparticles are deposited using an atmospheric pressure atomic layer deposition method, thus forming a porous layer (Patent Document 4).

Nano-graphite structures (carbon nanomaterials) having carbon crystal structures such as carbon nanotube (hereinafter referred to as CNT) and carbon nanohorn are expected to be applied, in the form of a complex with another nanomaterial in consideration of electric characteristics and structures, to electric functional materials, catalysts, optical functional materials and the like, and also to medical technologies and the like.

For example, there is a report that the electric characteristics of a functional material that uses titanium oxide are improved by forming a hybrid material of titanium oxide and CNT having a carbon crystal structure. A known method for binding titanium oxide to the surface of CNT is, for example, to subject the CNT to acid treatment (Non Patent Documents 2 and 3).

One reported method for binding titanium oxide to the surface of CNT is to precipitate titanium oxide on the surface of a carbon nanomaterial through a precipitation reaction using, for example, a titanium fluoro complex. In addition, one method known as a technique for binding CNT and titanium oxide without acid treatment is to use a material bondable to CNT and a material bondable to titanium oxide. More specifically, in one reported technique, a mixed polymer material comprising styrene and maleic acid is used to bind CNT to particles of a metal oxide such as titanium oxide (Patent Document 2). In another reported technique, a polypeptide comprising 35 amino acid residues in which CNT-binding peptides and titanium oxide-binding peptides are fused is used to coat the surface of CNT with titanium oxide, thus changing the electric characteristics of the CNT (Non Patent Document 4).

Particularly, when peptides are used as disclosed in Non Patent Document 4, first, CNT is coated with CNT-binding peptides, and the activity of titanium-binding peptides is utilized to facilitate deposition of titanium from the titanium complex on the surface of the CNT, thus circumferentially coating the peptides with titanium. Moreover the deposited titanium is oxidized by heating, thus forming titanium oxide, whereby the CNT is coated with the titanium oxide.

In one known example, to form a titanium film that encapsulates metal nanoparticles, ferritin having a polypeptide capable of forming an internal cavity, and having a 24-meric structure capable of storing a metal in its internal cavity, is formed, and a protein in which a titanium-binding peptide is fused with the ferritin having a 24-meric structure is used (Non Patent Document 5).

A dye-sensitized solar cell, which is one of solar cells that use an organic material, is watching because of its high photoelectric conversion efficiency. Examples of the material having a photoelectric conversion function and used for such a dye-sensitized solar cell may include a material in which a spectral sensitizing dye functioning as a photosensitizing dye having absorption in the visible light range is adsorbed on the surface of a semiconductor material.

For example, in known examples (Patent Documents 5, 6, 7, and 8), a structure body in which the surface of CNT is coated with titanium oxide through a precipitation reaction that uses a titanium fluoro complex or a structure body in which only the CNT in the above structure body are burned down is used as a semiconductor material for a dye-sensitized solar cell.

Also, in one reported technique, polyoxometalate is used to coat carbon nanotube (Non Patent Document 6).

Moreover, in one known example (Non Patent Document 7), a nano-complex in which titanium dioxide is precipitated on a plurality of CNTs bundled with a virus is used as the material of an optical electrode of a dye-sensitized solar cell.

### RELATED ART DOCUMENTS

### Patent Document

Patent Document 1: International Publication No. WO2007/074436.
Patent Document 2: International Publication No. WO2008/127396.
Patent Document 3: International Publication No. WO2008/118533.
Patent Document 4: International Publication No. WO2009/040499.
Patent Document 5: JP 2010-24133 A
Patent Document 6: JP 2010-24134 A
Patent Document 7: JP 2010-208941 A
Patent Document 8: JP 2010-24135 A

### Non Patent Document

Non-patent Document 1: M.A.Fox and M.T.Dulay,Chem.Rev.,1993,vol.93,p.341.
Non-patent Document 2: D. Eder, Chem. Rev., 2010, Vol. 110, p. 1348.
Non-patent Document 3: A. Kongkanand et al., Nano Lett., 2007 Vol. 7 p.676.
Non-patent Document 4: M.J.Pender et al.,Nano Lett.,2006,vol.6,No.1,p.44.
Non-patent Document 5: K. Sano et al.,Nano Lett.,2007,vol.7,No.10,p.3200.
Non-patent Document 6: Bin Fei et al.,Nanotechnology,2006,vol.17,p.1589.
Non-patent Document 7: X. Dang et al., Nature Nanotechnology., 2011, Vol. 6, p. 377.

### DISCLOSURE OF INVENTION

### PROBLEM TO BE SOLVED BY THE INVENTION

However, with the functional materials disclosed in the above conventional technologies, functional materials using titanium oxide, their photocatalytic activity and electric characteristics and the like are insufficient.

More specifically, when chemical treatment such as acid treatment is performed during production of a functional material, the electric characteristics of CNT deteriorate, so that the desired electric characteristics may not be obtained even when, for example, a structure bound to titanium oxide is formed.

When a polypeptide in which CNT-binding peptides and titanium oxide-binding peptides are fused is used, chemical treatment and the like of the CNT is not necessary, so that the electric characteristics of the CNT themselves do not deteriorate. However, since the size of the polypeptide is small, it is difficult to further increase the surface area of titanium oxide, and to further improve photocatalytic activity, electric characteristics and the like.

To produce a dye-sensitized solar cell, when a sensitizing dye is adsorbed on a semiconductor material (functional material), a structure having a thickness of about several µm to several tens of µm and formed from a porous semiconductor material is immersed in a dye-adsorption solution prepared by dissolving the sensitizing dye in an organic solvent such as ethanol. Therefore, even when the structure body formed from the semiconductor material has a specific surface area and a surface roughness coefficient enough to allow a sufficient amount of the sensitizing dye to be adsorbed, the structure formed from the porous semiconductor material cannot actually support a sufficient amount of the sensitizing dye unless the structure formed from the porous semiconductor material has inner pores that can contribute to an increase in the surface area.

Therefore, there is a demand for a functional material that is excellent in photocatalytic activity, electric characteristics and the like, and, for example, can support a larger amount of a sensitizing dye.

### MEANS FOR SOLVING PROBLEM

The present invention has been made in view of the foregoing problem. The present inventors have conducted extensive studies and found that a functional material having excellent photocatalytic activity, electric characteristics, etc. can be produced by using a multimer of a fusion protein that has a prescribed structure. Thus, the present invention has been completed.

The present invention provides the following [1] to [40].
[1] A porous structure body comprising: a first target material; and an aggregate body formed by aggregation of a second target material, the aggregate body adhering to the first target material and being located so as to surround the first target material, wherein
   the aggregate body has a plurality of first pores unevenly distributed near the first target material in the aggregate body and a plurality of second pores scattered over the aggregate body.
[2] The porous structure body according to claim 1, wherein the first target material is a carbon material selected from the group consisting of carbon nanotubes, carbon nanohorns, graphene sheets, fullerenes, and graphite.
[3] The porous structure body according to above [1] or [2], wherein the second target material is titanium oxide or zinc oxide.
[4] The porous structure body according to any one of above [1] to [3], further comprising metal particles put in the first pores and different from the second target material.
[5] The porous structure body according to above [4], wherein the metal particles are nanoparticles of a metal selected from the group consisting of iron oxide, nickel, cobalt, manganese, phosphorus, uranium, beryllium, aluminum, cadmium sulfide, cadmium selenide, palladium, chromium, copper, silver, a gadolium complex, platinum cobalt, silicon oxide, cobalt oxide, indium oxide, platinum, gold, gold sulfide, zinc selenide, and cadmium selenide.
[6] The porous structure body according to [5], wherein the metal particles are iron oxide nanoparticles.
[7] The porous structure body according to any one of above [1] to [5], wherein the first pores have a diameter within a range of 5 nm to 15 nm.
[8] The porous structure body according to any one of above [1] to [7], wherein distances to each the first pore from a surface of the first target material are identical and within a range of 1 nm to 500 nm.
[9] An electronic device comprising, as a functional material, the porous structure body according to any one of above [1] to [8].
[10] The electronic device according to above [9], wherein the electronic device is a solar cell.
[11] The electronic device according to above [10], wherein the solar cell comprises the porous structure body as a material for an electrode or as a functional material placed on the electrode.
[12] The electronic device according to above [11], wherein the electronic device is a dye-sensitized solar cell.
[13] A method for producing a porous structure body, comprising the steps of:
   preparing a complex in which a first target material and a second target material or a precursor of the second target material are bound to a multimer of a fusion protein, the complex being prepared by binding the multimer of the fusion protein to the first target material and binding the second target material or the precursor of the second target material to the multimer of the fusion protein, wherein the multimer of the fusion protein has an internal cavity and is formed from the fusion protein that contains a first peptide portion capable of binding to the first target material, a second peptide portion capable of binding to the second target material, and a polypeptide portion capable of forming a multimer having an internal cavity; and
   forming an aggregate body by burning the complex to consume the multimer of the fusion protein, the aggregate body adhering to the first target material and being located so as to surround the first target material, the aggregate body having a plurality of first pores unevenly distributed near the first target material and a plurality of second pores scattered over the aggregate body.
[14] The method for producing the porous structure body according to above [13],
   wherein the step of preparing the complex is the step of preparing the complex in which the first target material and the precursor of the second target material are bound to the multimer of the fusion protein, and the step of forming the aggregate body is the step of burning the complex to consume the multimer of the fusion protein and to convert the precursor of the second target material to the second target material, thereby forming the aggregate body.
[15] The method for producing the porous structure body according to above [14], wherein the step of preparing the complex is the step of binding the precursor of the second target material to the multimer of the fusion protein and depositing the second target material around the first target material.
[16] The method for producing the porous structure body according to any one of above [13] to [15], wherein the first target material is a carbon material selected from the group consisting of carbon nanotubes, carbon nanohorns, graphene sheets, fullerenes, and graphite.
[17] The method for producing the porous structure body according to any one of above [13] to [16], wherein the second target material is titanium oxide or zinc oxide.
[18] The method for producing the porous structure body according to any one of above [13] to [17],
   wherein the multimer of the fusion protein further comprises metal particles different from the first target material, each of the metal particles being encapsulated in the internal cavity thereof, and the aggregate body formed further comprises the metal particles in the first pores.
[19] The method for producing a porous structure body according to claim 18, wherein the metal particles are nanoparticles of a metal selected from the group consisting of iron oxide, nickel, cobalt, manganese, phosphorus, uranium, beryllium, aluminum, cadmium sulfide, cadmium selenide, palladium, chromium, copper, silver, a gadolium complex, platinum cobalt, silicon oxide, cobalt oxide, indium oxide, platinum, gold, gold sulfide, zinc selenide, and cadmium selenide.
[20] The method for producing the porous structure body according to above [19], wherein the metal particles are iron oxide nanoparticles.
[21] A method for producing an electronic device, comprising the step of forming a functional structure using the porous structure body according to any one of above [1] to [8] as a functional material.
[22] A method for producing a dye-sensitized solar cell, comprising the steps of:
   preparing a substrate comprising a transparent electrode;
   obtaining a complex by binding a multimer of a fusion protein to a first target material, wherein the multimer of the fusion protein has an internal cavity and is formed from the fusion protein that contains a first peptide portion capable of binding to the first target material, a second peptide portion capable of binding to the second target material, and a polypeptide portion capable of forming a multimer having an internal cavity, thereby forming a combination body and binding the combination body to a second target material or a precursor of the second target material;
   placing a material comprising the complex on the transparent electrode;
   burning the material on the transparent electrode to consume the multimer of the fusion protein, thereby forming a structure having a porous structure body on the transparent electrode, the porous structure body comprising an aggregate body that comprises the second target material, adheres to the first target material, and is located so as to surround the first target material, the aggregate body having a plurality of first pores unevenly distributed near the first target material and a plurality of second pores scattered over the aggregate body;
   supporting a sensitizing dye by the porous structure body, thereby forming a photoelectrode; and
   pouring an electrolyte and sealing the photoelectrode and a counter electrode.
[23] The method for producing the dye-sensitized solar cell according to above [22], wherein the structure comprising the porous structure body is formed such that a final concentration of the complex is less than 1% by weight.
[24] The method for producing the dye-sensitized solar cell according to above [23], wherein the structure comprising the porous structure body is formed such that the final concentration of the complex is within a range of 0.06% by weight to 0.5% by weight.
[25] A method for producing a dye-sensitized solar cell, comprising the steps of:
   preparing a substrate comprising a transparent electrode;
   forming a porous structure body comprising an aggregate body by binding a multimer of a fusion protein, wherein the multimer of the fusion protein has an internal cavity and is formed from the fusion protein that contains a first peptide portion capable of binding to the first target material, a second peptide portion capable of binding to the second target material, and a polypeptide portion capable of forming a multimer having an internal cavity, to a first target material, thereby forming a combination body, binding the combination body to a second target material or a precursor of the second target material, thereby forming a complex body, and burning the complex body to consume the multimer of the fusion protein, the aggregate body comprising the second target material, adhering to the first target material, and being located so as to surround the first target material, the aggregate body having a plurality of first pores unevenly distributed near the first target material and a plurality of second pores scattered over the aggregate body;
   placing a material comprising the porous structure body and the second target material or the precursor of the second target material on the transparent electrode;
   heating the material on the transparent electrode, thereby forming a structure body comprising the porous structure body by the transparent electrode;
   supporting a sensitizing dye by the porous structure body, thereby forming a photoelectrode; and
   pouring an electrolyte and sealing the photoelectrode and a counter electrode.
[26] The method for producing the dye-sensitized solar cell according to any one of above [22] to [25], wherein the first target material is a carbon material selected from the group consisting of carbon nanotubes, carbon nanohorns, graphene sheets, fullerenes, and graphite.
[27] A method for producing a dye-sensitized solar cell, comprising the steps of:
   preparing a substrate comprising a transparent electrode;
   arranging at least one carbon nanotube used as a first target material on the transparent electrode so as to extend in a direction of a thickness of the substrate;
   forming a complex by binding a multimer of a fusion protein to the carbon nanotube, wherein the multimer of the fusion protein has an internal cavity and is formed from the fusion protein that contains a first peptide portion capable of binding to the carbon nanotube, a second peptide portion capable of binding to a precursor of the second target material, and a polypeptide portion capable of forming a multimer having an internal cavity, thereby forming a combination body and binding the combination body to a second target material or a precursor of the second target material;
   burning the mixture to consume the multimer of the fusion protein, whereby a structure body having a porous structure body comprising an aggregate body is formed on the transparent electrode, the aggregate body comprising the second target material, adhering to the carbon nanotube, and being located so as to surround the carbon nanotube, the aggregate body having a plurality of first pores unevenly distributed near the carbon nanotube and a plurality of second pores scattered over the aggregate body;
   supporting a sensitizing dye by the porous structure body, thereby forming a photoelectrode; and
   pouring an electrolyte and sealing the photoelectrode and a counter electrode.
[28] The method for producing a dye-sensitized solar cell according to above [27], wherein the step of arranging the at least one carbon nanotube used as the first target material on the transparent electrode so as to extend in the direction of the thickness of the substrate comprises the step of adsorbing an inorganic material on a substrate, the step of growing a carbon nanotube from the inorganic material as a seed, thereby obtaining a carbon nanotube-arranged substrate, and the step of transferring the carbon nanotube on the carbon nanotube-arranged substrate to the transparent electrode.
[29] The method for producing the dye-sensitized solar cell according to above [28], wherein the step of adsorbing the inorganic material on the substrate is the step of adsorbing and arranging at least two types of inorganic material-encapsulating proteins with different sizes on the substrate.
[30] The method for producing the dye-sensitized solar cell according to above [29], wherein the step of adsorbing the inorganic material on the substrate is the step of adsorbing and arranging the inorganic material-encapsulating proteins on a silicon oxide film provided on the substrate used.
[31] The method for producing the dye-sensitized solar cell according to above [29] or [30], wherein the inorganic material-encapsulating proteins comprise at least one type of protein selected from ferritin protein, Dps protein, CDT protein, and modified proteins thereof.
[32] The method for producing the dye-sensitized solar cell according to any one of above [22] to [31], wherein the second target material is titanium oxide or zinc oxide.
[33] A dye-sensitized solar cell obtainable by the method for producing according to any one of above [22] to [32].
[34] A dye-sensitized solar cell comprising:
   a substrate comprising a transparent electrode;
   a photoelectric conversion layer provided on the transparent electrode, the photoelectric conversion layer comprising a porous structure body, the porous structure body supporting a sensitizing dye, the porous structure body comprising a first target material and an aggregate body comprising a second target material, the aggregate body adhering to the first target material and being located so as to surround the first target material, the aggregate body having a plurality of first pores unevenly distributed near the first target material and a plurality of second pores scattered over the aggregate body; and
   a sealing portion that seals the dye-sensitized solar cell such that the photoelectrode faces a counter electrode and the photoelectrode and the counter electrode are in contact with an electrolyte.
[35] A dye-sensitized solar cell comprising:
   a substrate comprising a transparent electrode;
   a photoelectric conversion layer provided on the transparent electrode, the photoelectric conversion layer comprising a porous structure body, the porous structure body supporting a sensitizing dye, the porous structure body comprising at least one carbon nanotube used as a first target material and an aggregate body comprising a second target material, the at least one carbon nanotube being arranged to extend in a direction of a thickness of the substrate, the aggregate body adhering to the carbon nanotube and being located so as to surround the carbon nanotube, the aggregate body having a plurality of first pores unevenly distributed near the carbon nanotube and a plurality of second pores scattered over the aggregate body; and
   a sealing portion that seals the dye-sensitized solar cell such that the photoelectrode faces a counter electrode and the photoelectrode and the counter electrode are in contact with an electrolyte.
[36] The dye-sensitized solar cell according to claim 33, wherein the first target material is a carbon material selected from the group consisting of carbon nanotubes, carbon nanohorns, graphene sheets, fullerenes, and graphite.
[37] The dye-sensitized solar cell according to any one of above [34] to [36], wherein the second target material is titanium oxide or zinc oxide.
[38] A method for producing a carbon nanotube-arranged substrate in which at least one carbon nanotube is arranged to extend in a thickness direction, the method comprising the steps of:
   adsorbing and arranging at least two types of inorganic material-encapsulating proteins with different sizes on the substrate; and
   growing a carbon nanotube from an inorganic material encapsulated in the inorganic material-encapsulating proteins as a seed.
[39] The method for producing a carbon nanotube-arranged substrate according to above [38], wherein the step of adsorbing and arranging the at least two types of inorganic material-encapsulating proteins with different sizes on the substrate is performed by adsorbing the inorganic material-encapsulating proteins on a silicon oxide film provided on the substrate used.
[40] The method for producing the carbon nanotube-arranged substrate according to above [38] or [39], wherein the inorganic material-encapsulating proteins comprise at least one type of protein selected from ferritin protein, Dps protein, CDT protein, and modified proteins thereof.

### EFFECT OF THE INVENTION

The porous structure body of the present invention has excellent performance in terms of photocatalytic activity, electric characteristics and the like. The porous structure body of the present invention can be preferably used as a functional material having an antimicrobial function, a deodorization function, an air purification function, an antifouling function, a hydrogen generation function and the like for various electronic devices. Therefore, higher performance solar cells, semiconductor devices and the like can be provided. The porous structure body of the present invention is also useful as a functional material used in the fields of medicine, biological research and the like.

### BRIEF DESCRIPTION OF DRAWINGS

FIG. 1A is a schematic plain illustration of a porous structure body.
FIG. 1B is a schematic plain illustration (1) showing a cut-end face of the porous structure body cut at a position shown by a long dashed and short dashed line of IB-IB in FIG. 1A.
FIG. 1C is a schematic plain illustration (2) showing a cut-end face of the porous structure body cut at the same position as in FIG. 1B.
FIG. 1D is a schematic plain illustration (3) showing a cut-end face of the porous structure body cut at the same position as in FIG. 1B.
FIG. 1E is a schematic plain illustration (4) showing a cut-end face of the porous structure body cut at the same position as in FIG. 1B.
FIG. 2A is a schematic plain illustration (1) showing an embodiment of a dye-sensitized solar cell.
FIG. 2B is a schematic plain illustration (2) showing an embodiment of a dye-sensitized solar cell.
FIG. 3A is a schematic plain illustration (1) showing a step of producing a dye-sensitized solar cell.
FIG. 3B is a schematic plain illustration (2) showing a step of producing a dye-sensitized solar cell.
FIG. 3C is a schematic plain illustration (3) showing a step of producing a dye-sensitized solar cell.
FIG. 3D is a schematic plain illustration (4) showing a step of producing a dye-sensitized solar cell.
FIG. 4 is a view of a transmission electron microscopic image of a complex of CNT and CNHBP-Dps-TBP (CDT).
FIG. 5 is a view of a transmission electron microscopic image of a black precipitate obtained by adding a titanium precursor to a complex of CNT and CNHBP-Dps-TBP (CDT).
FIG. 6 is a diagram showing peaks obtained by EDS analysis performed on regions surrounded by Box 004 and Box 005 illustrated in FIG. 5.
FIG. 7 is a view of a transmission electron microscopic image of a black precipitate obtained by adding a titanium precursor to a complex of CNHBP-Dps-TBP (CDT) and CNT.
FIG. 8 is a view of a transmission electron microscopic image of a black precipitate obtained by adding a titanium precursor to a complex of CNHBP-Dps-TBP (CDT) and CNT.
FIG. 9 is a view of a transmission electron microscopic image of a black precipitate obtained by adding a titanium precursor into CNT dispersing solution.
FIG. 10 is a view of a transmission electron microscopic image of a complex of CNHBP-Dps-TBP (CDT) and CNT having an iron oxide nanoparticle in an internal cavity.
FIG. 11 is a view of a transmission electron microscopic image of a black precipitate obtained by adding a titanium precursor to a complex of CNHBP-Dps-TBP (CDT) and CNT having an iron oxide nanoparticle in an internal cavity.
FIG. 12 is a view of a transmission electron microscopic image of a structure obtained by burning a complex of CNT and CNHBP-Dps-TBP (CDT) coated with titanium.
FIG. 13A is a view of a transmission electron microscopic image of a structure obtained by burning at 500°C a complex of CNT and CNHBP-Dps-TBP (CDT) coated with titanium.
FIG. 13B is a view of a transmission electron microscopic image of a structure obtained by burning at 600°C a complex of CNT and CNHBP-Dps-TBP (CDT) coated with titanium.
FIG. 13C is a view of a transmission electron microscopic image of a structure obtained by burning at 700°C a complex of CNT and CNHBP-Dps-TBP (CDT) coated with titanium.
FIG. 13D is a view of a transmission electron microscopic image of a structure obtained by burning at 800°C a complex of CNT and CNHBP-Dps-TBP (CDT) coated with titanium.
FIG. 14 is a view of a result showing XRD analysis of a structure obtained by burning a complex of CNT and CNHBP-Dps-TBP (CDT) coated with titanium.
FIG. 15 is a graph (1) showing the current-voltage characteristics of dye-sensitized solar cells.
FIG. 16 is a graph (2) showing the current-voltage characteristics of dye-sensitized solar cells.
FIG. 17 is a graph (3) showing the current-voltage characteristics of dye-sensitized solar cells.
FIG. 18 is a view of a transmission electron microscopic image of obtained CcDT.
Fig 19 is a graph showing the result of confirmation of binding between CcDT and CNT.
Fig 20 is a graph showing the result of confirmation of binding between CcDT and a titanium oxide.
FIG. 21 is a view showing a transmission electron microscopic image of CDZ.
FIG. 22 is a graph showing facilitation of formation of a white precipitate from an aqueous solution of zinc sulfate by CDZ.
FIG. 23 is a view showing a transmission electron microscopic image of a complex of CDZ and CNT.
FIG. 24 is a graph (4) showing the current-voltage characteristics of dye-sensitized solar cells.
FIG. 25 is a graph (5) showing the current-voltage characteristics of dye-sensitized solar cells.
FIG. 26 is a photograph of CNT synthesized on a silicon substrate subjected to SEM analysis.
FIG. 27A is a view showing a transmission electron microscopic image of a mixed solution of CDT and CNT.
FIG. 27B is a view showing a transmission electron microscopic image of a mixed solution of CDT and CNT.
FIG. 28 is a photograph of CNT synthesized on a silicon substrate subjected to SEM analysis.
FIG. 29A is a photograph of CNT synthesized using CDT and TBF at a ratio of 1:1.
FIG. 29B is a photograph of CNT synthesized using CDT and TBF at a ratio of 1:2.
FIG. 29C is a photograph of CNT synthesized using CDT and TBF at a ratio of 2:1.
FIG. 30 is a photograph obtained by SEM analysis on a complex of CNT and Fe-CD.

### DESCRIPTION OF EMBODIMENTS

Embodiments of the present invention will next be described with reference to the drawings. In the drawings, the shape, size, and arrangement of each component are shown only schematically to such an extent that the invention can be understood. The present invention is not limited to the following description, and each component may be appropriately modified without departing from the scope of the present invention.

In the drawings used for the following description, similar components may be denoted by the same numerals, and redundant description may be omitted.

### 1. Configuration of porous structure body

### (1) Embodiment 1 of porous structure body

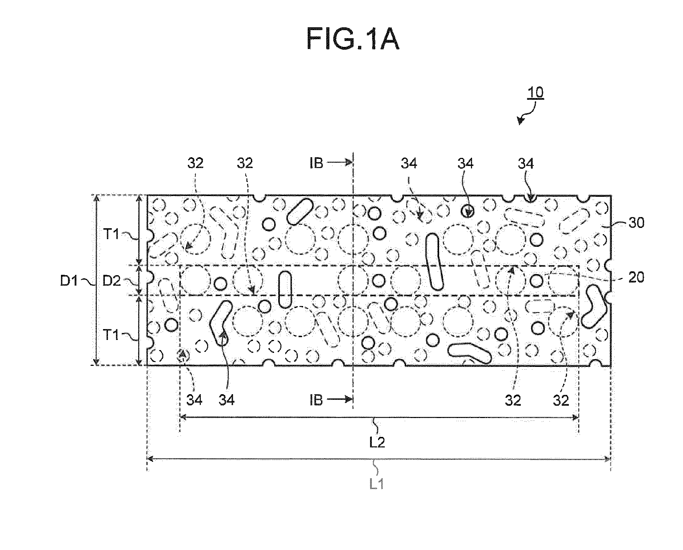

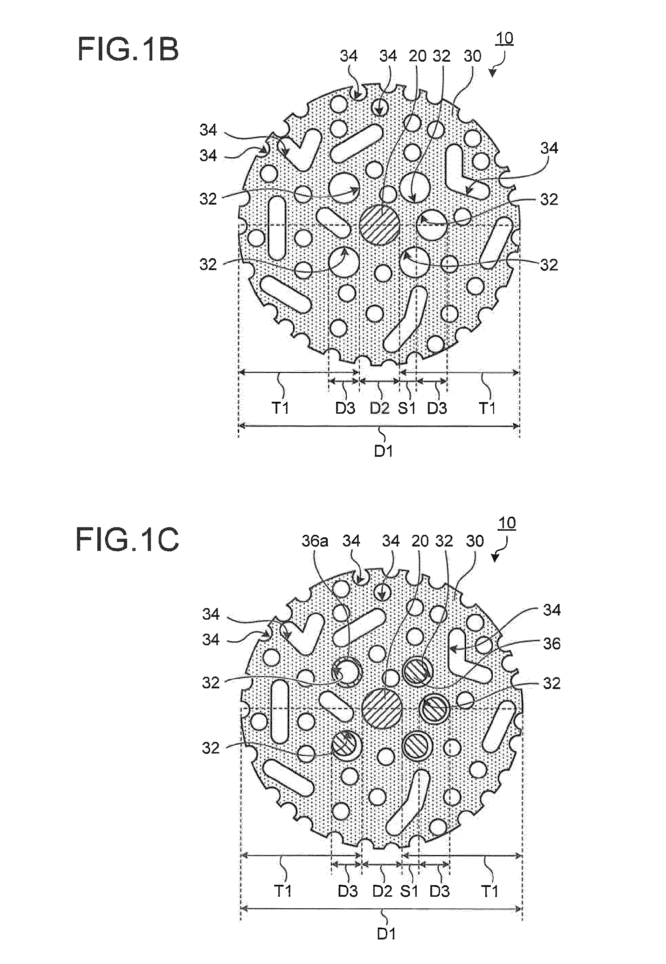

Embodiment 1 of the porous structure body will be described with reference to FIGs. 1A and 1B. FIG. 1A is a schematic plain illustration of the porous structure body. FIG. 1B is a schematic illustration showing a cut-end face of the porous structure body cut at a position shown by a dash-dot line IB-IB in FIG. 1A.

As shown in FIGs. 1A and 1B, the porous structure body 10 comprises: a first target material 20 selected from the group consisting of metal materials, silicon materials, and carbon materials; and an aggregate body 30 formed by aggregation of a second target material, adhering to the first target material 20, and located so as to surround the first target material 20. The aggregate body 30 has a plurality of first pores 32 unevenly distributed near the first target material 20 in the aggregate body 30 and a plurality of second pores 34 scattered over the aggregate body 30.

The porous structure body 10 has an elongated rod-like (bar-like) shape. In consideration of application to, for example, electronic devices, the average length L1 of the porous structure body 10 in its elongation direction is preferably about 10 nm to about 100 µm and particularly preferably about 50 nm to about 20 µm, from the viewpoint of electric characteristics and optical characteristics.

The porous structure body 10 comprises the first target material 20. The first target material 20 is located, as a core, substantially at the radial center of the porous structure body 10 in a direction orthogonal to the elongation direction of the porous structure body 10.

Examples of the first target material 20 may include inorganic materials and organic materials, or conductive materials, semiconductor materials, and magnetic materials. Specifically, the first target material 20 may include metal materials, silicon materials, carbon materials, low molecular compounds (e.g., biological substances such as porphyrin, radioactive substances, fluorescent substances, dyes, and medicines), polymers (e.g., hydrophobic organic polymers and conductive polymers such as poly(methyl methacrylate), polystyrene, polyethylene oxide, or poly(L-lactic acid)), proteins (e.g., oligopeptides or polypeptides), nucleic acids (DNA or RNA, or nucleosides, nucleotides, oligonucleotides or polynucleotides), carbohydrates (e.g., monosaccharides, oligosaccharides or polysaccharides), and lipids.

Examples of the metal materials as the first target material 20 may include metals and metal compounds. Examples of the metal may include titanium, chromium, zinc, lead, manganese, calcium, copper, calcium, germanium, aluminium, gallium, cadmium, iron, cobalt, gold, silver, platinum, palladium, hafnium, and tellurium. Examples of the metal compounds may include oxide, sulfide, carbonate, arsenide, chloride, fluoride and iodide of the metals, and intermetallic compounds. Oxide of the metal may include various oxides. Describing such an oxide using the oxide of titanium as one example, examples of the oxide of titanium may include titanium monoxide (CAS No. 12137-20-1), titanium dioxide (CAS No. 13463-67-7), titanium dioxide (anatase, CAS No. 1317-70-0), titanium dioxide (rutile, CAS No. 1317-80-2), and titanium trioxide (CAS No. 1344-54-3). More specifically, the metal compounds may include oxides of titanium as described above, chromium oxide, zinc oxide, lead oxide, manganese oxide, zeolite, calcium carbonate, copper oxide, manganese-calcium oxide, germanium oxide, aluminium oxide, hafnium oxide, lead titanium zirconate, gallium arsenide, zinc sulfide, lead sulfide, cadmium sulfide, iron platinum, cobalt platinum, and cadmium tellurium.

Examples of the silicon materials as the first target material 20 may include silicon and silicon compounds. Examples of the silicon compounds may include oxides of silicon (e.g., silicon monoxide (SiO), silicon dioxide (SiO₂)), silicon carbide (SiC), silane (SiH₄), and silicone rubbers.

Examples of the carbon materials as the first target material 20 may include carbon nanomaterials (e.g., carbon nanotube (CNT), carbon nanohorn (CNH)), fullerene (C₆₀ fullerene), graphene sheet, and graphite.

In consideration of application of the porous structure body 10 to electronic devices, the first target material 20 is preferably, for example, a material selected from the group consisting of metal materials, silicon materials, and carbon materials. The first target material 20 is preferably a carbon material selected from the group consisting of carbon nanotube, carbon nanohorn, graphene sheets, fullerenes, and graphite and is particularly preferably carbon nanotube (CNT).

In this embodiment and embodiments described later, a description will be specifically given of an example in which CNT is used as the first target material 20. CNT is classified in terms of its shape into single layer carbon nanotube, multilayer carbon nanotube, armchair carbon nanotube, chiral carbon nanotube, zigzag carbon nanotube and the like. CNT is classified in terms of its electric characteristics into metal type, and semiconductor type. No particular limitation is imposed on the CNT usable for the porous structure body 10, and any suitable CNT may be selected in consideration of the desired characteristics of the porous structure body 10.

The CNT used may be a combination of different types of CNT having an average length of D2 and different diameters (thicknesses). No particular limitation is imposed on the diameter D2 and aspect ratio of the CNT used for the porous structure body 10. In consideration of application to, for example, electronic devices, the CNT used for the porous structure body 10 has an average length L2 in the elongation direction of preferably about 10 nm to about 100 µm and particularly preferably about 50 nm to about 20 µm, from the viewpoint of electric characteristics and optical characteristics. The aspect ratio of the CNT used for the porous structure body 10 (the average length L2 in the elongation direction / the average radial length D2 in a direction orthogonal to the elongation direction) is preferably 5 to 50,000. The average length L2 in the elongation direction and the average radial length D2 in the direction orthogonal to the elongation direction can be measured, for example, by observation under an electron microscope.

The porous structure body 10 comprises the aggregate body 30. The aggregate body 30 is formed by aggregating (binding) many pieces of the second target material. The aggregate body 30 has a porous sponge-like shape.

Examples of the second target material may include inorganic materials and organic materials or may include conductive materials, semiconductor materials, and magnetic materials. Specific examples of such a target material may include metal materials, silicon materials, carbon materials, low molecular compounds (e.g., biological substances such as porphyrin, radioactive substances, fluorescent substances, dyes, and medicines), polymers (e.g., hydrophobic organic polymers and conductive polymers such as polymethyl methacrylate, polystyrene, polyethylene oxide, and poly(L-lactic acid)), proteins (e.g., oligopeptides and polypeptides), nucleic acids (e.g., DNA, RNA, nucleosides, nucleotides, oligonucleotides, and polynucleotides), carbohydrates (e.g., monosaccharides, oligosaccharides, and polysaccharides), and lipids.

Examples of the metal materials may include metals and metal compounds. Examples of the metals may include titanium, chromium, zinc, lead, manganese, calcium, copper, calcium, germanium, aluminum, gallium, cadmium, iron, cobalt, gold, silver, platinum, palladium, hafnium, and tellurium. Examples of the metal compounds may include oxides, sulfides, carbonates, arsenides, chlorides, fluorides, and iodides of metals, and intermetallic compounds. Examples of such oxides of metals include various types of oxides.

A description will be given of oxide of titanium (titanium oxide) as an example of such an oxide. Examples of the oxide of titanium may include titanium monoxide (CAS No. 12137-20-1), titanium dioxide (CAS No. 13463-67-7), titanium dioxide (anatase, CAS No. 1317-70-0), titanium dioxide (rutile, 1317-80-2), and dititanium trioxide (CAS No. 1344-54-3). More specifically, examples of the metal compounds may include oxides of titanium described above, chromium oxide, zinc oxide, lead oxide, manganese oxide, zeolite, calcium carbonate, copper oxide, manganese-calcium oxide, germanium oxide, aluminum oxide, hafnium oxide, lead titanium zirconate, gallium arsenide, zinc sulfide, lead sulfide, cadmium sulfide, iron platinum, cobalt platinum, and cadmium tellurium.

The metal material used is preferably a metal material that can be deposited through the deposition action of a fusion protein (a multimer of the fusion protein) used for a method for producing the porous structure body. (The details will be described later.)

Examples of the silicon materials may include silicon and silicon compounds. Examples of the silicon compounds may include silicon oxides (e.g., silicon monoxide (SiO) and silicon dioxide (SiO₂)), silicon carbide (SiC), silane (SiH₄), and silicone rubber.

When the porous structure body 10 is used as a functional material for a dye-sensitized solar cell, the second target material is preferably titanium oxide or zinc oxide.

No particular limitation is imposed on the crystal structures of titanium oxide and zinc oxide used as the second target material.

When the porous structure body 10 comprising titanium oxide as the second target material is used for a dye-sensitized solar cell, it is preferable that the porous structure body 10 comprising at least one selected from the group consisting of anatase-type titanium oxide, rutile-type titanium oxide, and brookite-type titanium oxide. In terms of photoactivity, anatase-type titanium oxide is more preferred. The crystal structure of titanium oxide can be measured by, for example, an X-ray diffraction method, Raman spectroscopic analysis and the like.

When the porous structure body 10 is used for a dye-sensitized solar cell, the average particle diameter of the titanium oxide used as the second target material is preferably 10 nm to 100 nm and more preferably 10 nm to 20 nm, from the viewpoint of ease of adsorption of the sensitizing dye and efficient absorption of light.

When the porous structure body 10 is used for a dye-sensitized solar cell and zinc oxide is used as the second target material, the average particle diameter of the zinc oxide is preferably 20 nm to 500 nm, from the viewpoint of ease of adsorption of the sensitizing dye and efficient absorption of light.

The average particle diameter can be measured by, for example, electron microscopic (SEM) observation.

The number of types of second target materials that can be comprised in the porous structure body 10 is not only one but also two or more.

When the porous structure body 10 is used, for example, as a functional material for a rechargeable battery, nickel, iron, cadmium, lithium, or a metal that can form a compound with any of the above metals (e.g., a nickel compound or an iron compound) may be used as the second target material, from the viewpoint of electric characteristics.

When the porous structure body 10 is used, for example, as a functional material for a capacitor, any of titanium, compounds thereof, tantalum, compounds thereof, aluminum, compounds thereof and the like may be used as the second target material, from the viewpoint of electric characteristics.

When the porous structure body 10 is used, for example, as a functional material for a transparent electrode, any of indium tin oxide (ITO), zinc tin oxide (ZTO), fluorine-doped tin oxide (FTO) and the like may be used as the second target material, from the viewpoint of electric characteristics.

When the porous structure body 10 is used, for example, as a functional material for a fuel cell, any of platinum, compounds thereof and the like may be used as the second target material, from the viewpoint of electric characteristics and catalytic activity.

The aggregate body 30 is adhering to the first target material 20 and located so as to surround the first target material 20. The external shape of the aggregate body 30 corresponds with the external shape of the porous structure body 10. In other words, the aggregate body 30 (the porous structure body 10) has an elongated rod-like shape with the first target material 20 serving as a core.

The thickness T1 of the aggregate body 30, namely, the distance from the interface between the first target material 20 and the aggregate body 30 to the surface of the aggregate body 30 (the porous structure body 10) in a direction orthogonal to the elongation direction of the porous structure body 10, may be any suitable distance (thickness T1) in consideration of, for example, the required characteristics of the porous structure body 10 or a device that uses the porous structure body 10 (e.g., a storage battery or a catalyst). The thickness T1 of the aggregate body 30 is preferably 5 nm to 1,000 nm and more preferably 10 nm to 100 nm.

Particularly, when the porous structure body 10 is used as a functional material for a solar cell, the thickness T1 of the aggregate body 30 is preferably 5 nm to 500 nm and more preferably 10 nm to 100 nm from the viewpoint of prevention of recombination of carriers.

Therefore, the aspect ratio of the porous structure body 10 (the average length L1 in the elongation direction / the average radial length D1 in a direction orthogonal to the elongation direction) is preferably about 1 to about 10,000.

The aggregate body 30 has a plurality of first pores 32. The plurality of first pores 32 are unevenly distributed near the first target material 20. In other words, the plurality of first pores 32 are located specifically in the vicinity of the first target material 20. The plurality of first pores 32 are located and spaced such that the distances S1 from the plurality of first pores 32 to the surface of the first target material 20 (the distances from the contours of the first pores 32 to the surface of the first target material 20 in directions orthogonal to the surface of the first target material 20 and passing through the centers of the first pores 32) are identical (equal) to each other. In addition, the plurality of first pores 32 are arranged so as to surround the first target material 20.

The term "identical" as used in the present specification means substantially identical and is meant to allow an unavoidable and unintended slight difference to be included to the extent that it does not cause any essential functional impairment.

The phrase "the distances are identical" means that the distances are substantially identical and also means that, when the distances S1 from the first pores 32 are compared with each other, differences that cause no impairment of the function of the porous structure body 10 and occur unavoidably in its producing step are allowed to exist.

The first pores 32 have a substantially spherical shape. The plurality of first pores 32 have substantially identical shapes and diameters D3. The shape and diameter of each first pore 32 result from the shape etc. of a multimer of a fusion protein used in the step of producing the porous structure body 10 (the details will be described later). The diameters D3 of the first pores 32, for example, their diameters when the pores are assumed to be spherical, are within the range of about 5 nm to about 15 nm as measured using a transmission electron microscope or a scanning electron microscope.

The plurality of first pores 32 are generally present within the thickness of the aggregate body 30. However, part of or all the plurality of first pores 32 may open at at least one of the surface of the aggregate body 30 and the surface of the first target material 20.

The distances S1 from the first pores 32 to the first target material 20, namely, the distances S1 from the first pores 32 to the interface between the first target material 20 and the aggregate body 30 in a direction orthogonal to the elongation direction of the porous structure body 10, may be any suitable distances, in consideration of the required characteristics of the porous structure body 10. The distances S1 are preferably, for example, 1 nm to 500 nm and more preferably 5 nm to 100 nm in consideration of electric characteristics such as conductivity and adhesion to the first target material 20.

Particularly, when the porous structure body 10 is used for a dye-sensitized solar cell, the distances S1 are preferably 1 nm to 500 nm and more preferably 5 nm to 100 nm, in consideration of the life and migration distance of carries generated by irradiation with light.

The aggregate body 30 has a plurality of second pores 34. The plurality of second pores 34 are scattered over the aggregate body 30. In other words, the plurality of second pores 34 are distributed randomly over the entire aggregate body 30.

The shapes of the plurality of second pores 34 are not uniform, and the second pores 34 may have various shapes such as a rough spherical shape, a rod-like shape, etc. The dimensions (diameter and length) of the plurality of second pores 34 are also not constant and uniform. The second pores 34 and the first pores 32 can be distinguished from each other because of the following reason. The second pores 34 have nonuniform outline shapes and sizes and are scattered over the entire aggregate body 30. However, the outline shapes of the first pores 32 are rough spherical and substantially identical, and all the first pores 32 are unevenly distributed specifically at positions spaced apart from the surface of the first target material 20 by substantially the same distance.

The second pores 34 and the first pores 32 can be distinguished from each other by, for example, electron microscopic (SEM) observation and the like.

The plurality of second pores 34 are generally present within the thickness of the aggregate body 30. However, part of the plurality of second pores 34 may open at at least one of the surface of the aggregate body 30 and the surface of the first target material 20. Second pores 34 may be in communication with first pores 32.

The porous structure body 10 in embodiment 1 has the plurality of first pores 32 within the aggregate body 30. The presence of the first pores 32 allows the surface area of the porous structure body 10 to be significantly increased, particularly, within the thickness of the porous structure body 10.

When such a porous structure body 10 is used particularly as a functional material for a dye-sensitized solar cell, a larger amount of sensitizing dye can be supported by the first pores 32 particularly within the thickness of the porous structure body 10. Therefore, the photoelectric conversion efficiency of the dye-sensitized solar cell can be further improved.

For example, when a porous structure body 10 in which the aggregate body 30 is formed from titanium oxide or zinc oxide and CNT is used as the first target material 20 is used particularly as a functional material for a dye-sensitized solar cell, a large number of electrons separated by incident light in the porous structure body 10 supporting the sensitizing dye are transmitted to the CNT without recombination and can be extracted to the outside from the CNT rapidly.

### (2) Embodiment 2 of porous structure body

Embodiment 2 of the porous structure body will be described with reference to FIG. 1C. FIG. 1C is a schematic illustration showing a cut end face of the porous structure body, as in FIG. 1B.

As shown in FIG. 1C, the porous structure body 10 in embodiment 2 further comprises metal particles 36 that are put in the first pores 32 and may be different from the second target material. The metal particles 36 may be joined to wall surfaces of the aggregate body 30 defining the first pores 32 or may be spaced apart from the wall surfaces.

Part of or all the metal particles 36 comprised in the porous structure body 10 may be thin films, namely, metal thin films 36a. The metal thin films 36a may coat part of or all the wall surfaces defining the first pores 32 in the aggregate body 30.

The porous structure body 10 comprises a first target material 20 and a porous sponge-like aggregate body 30 formed from a second target material. The aggregate body 30 has a plurality of first pores 32 and a plurality of second pores 34. The components other than the metal particles 36 are as described in (1) Embodiment 1 of the porous structure body, and their detailed description will be omitted.

Preferably, the metal particles 36 are nanoparticles of a metal selected from the group consisting of iron oxide, nickel, cobalt, manganese, phosphorus, uranium, beryllium, aluminum, cadmium sulfide, cadmium selenide, palladium, chromium, copper, silver, a gadolium complex, platinum cobalt, silicon oxide, cobalt oxide, indium oxide, platinum, gold, gold sulfide, zinc selenide, and cadmium selenide. No particular limitation is imposed on the metal particles 36 usable for the porous structure body 10, and any suitable metal particles 36 may be selected in consideration of the required characteristics of the porous structure body 10.

When the porous structure body 10 in embodiment 2 is used as, for example, a functional material for a solar cell, quantum dots, such as cadmium selenide, cadmium sulfide, or zinc oxide, may be used as the metal particles 36 having the ability to absorb light. When the porous structure body 10 is used as, for example, a functional material for a semiconductor memory, nickel, cobalt, or iron oxide may be used as the metal particles 36 that can change a charged state.

The number of types of metal particles 36 that can be comprised in the porous structure body 10 may be not only one but also two or more.

In the porous structure body 10 in embodiment 2, the aggregate body 30 comprises the metal particles 36 provided in the plurality of first pores 32. This porous structure body 10 has the above-described operation and effect of the porous structure body 10 in embodiment 1, and the metal particles 36 can further improve the functions of the porous structure body 10 and can add a new function such as the ability to absorb visible light, the ability to absorb infrared light, or the ability to retain electric charge.

Particularly, when the porous structure body 10 in embodiment 2 is used as a functional material for a quantum dot solar cell, an electrode having absorption in a wide wavelength range can be obtained. Therefore, the photoelectric conversion efficiency can be further improved.

### (3) Embodiment 3 of porous structure body

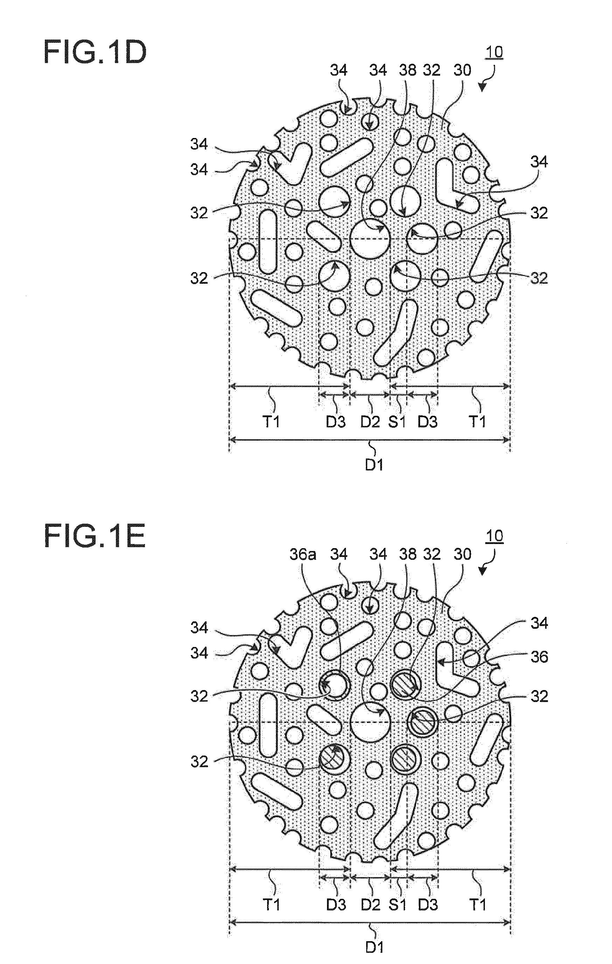

Embodiment 3 of the porous structure body will be described with reference to FIG. 1D. FIG. 1D is a schematic illustration showing a cut-end face of the porous structure body, as is FIG. 1B.

As shown in FIG. 1D, the porous structure body 10 in embodiment 3 comprises no first target material 20, and the region in which the first target material 20 is present in the above-described porous structure body 10 in embodiment 1 is a third pore 38.

In other words, the porous structure body 10 comprises a porous sponge-shaped aggregate body 30 formed from a second target material. The aggregate body 30 comprises a plurality of first pores 32 and a plurality of second pores 34.

Components other than the third pore 38 are as described above and denoted by the same numerals, and their detailed description will be omitted.

The third pore 38 is located, as a core, substantially at the radial center of the porous structure body 10 in a direction orthogonal to the elongation direction of the porous structure body 10 and has a rod-like shape extending in the elongation direction of the porous structure body 10.

No particular limitation is imposed on the diameter D2 and aspect ratio of the third pore 38. The diameter D2 of the third pore 38 is preferably 1 nm to 20 nm and particularly preferably 5 nm to 10 nm, and the average length in the elongation direction is preferably 10 nm to 100 µm and particularly preferably 50 nm to 20 µm. The length L2 in the elongation direction and diameter D2 of the third pore 38 can be measured, for example, by observation under an electron microscope.

In the porous structure body 10 in embodiment 3, the aggregate body 30 has the third pore 38 in addition to the first pores 32 and the second pores 34. Therefore, in the porous structure body 10 in embodiment 3, the surface area of the porous structure body 10 can be further increased. With the porous structure body 10 in embodiment 3, light transmittance can be further improved. Therefore, the porous structure body 10 can be used, for example, as a conductive material for a transparent electrode. Particularly, when the porous structure body 10 in embodiment 3 is used as a functional material for an electrode of a fuel cell or a solar cell, a porous electrode with higher light transmittance can be realized. Therefore, catalytic activity and photoelectric conversion efficiency can be further improved.

### (4) Embodiment 4 of porous structure body

Embodiment 4 of the porous structure body will be described with reference to FIG. 1E. FIG. 1E is a schematic illustration showing a cut-end face of the porous structure body, as is FIG. 1B.

As shown in FIG. 1E, the porous structure body 10 in embodiment 4 comprises no first target material 20, as does the porous structure body 10 in embodiment 3, and the region in which the first target material 20 is present in the porous structure body 10 in embodiment 1 is a third pore 38.

As does the porous structure body 10 in embodiment 2, the porous structure body 10 in embodiment 4 further comprises metal particles 36 that are put in the first pores 32 and different from the second target material.

The third pore 38 is as described in embodiment 3, and the metal particles 36 are as described in embodiment 2. Therefore, their detailed description will be omitted.

In the porous structure body 10 in embodiment 4, the aggregate body 30 has the third pore 38 and also has the metal particles 36 in the first pores 32. Therefore, particularly, when the porous structure body 10 in embodiment 4 is used as a functional material for a semiconductor memory or a capacitor, the storage capacity of the semiconductor memory can be further increased, or the electric capacity of the capacitor can be further increased.

### 2. Embodiments of use of the porous structure body

The porous structure body 10 can be used as catalysts, antimicrobial materials, deodorizing materials, and antifouling materials. The porous structure body 10 can be used also as functional materials for functional layers of solar cells such as dye-sensitized solar cells, fuel cells, hydrogen generators, memory elements, capacitors, and electronic devices (electronic elements) such as transistors. More specifically, the porous structure body 10 is useful as materials of electrodes of solar cells such as a functional material for a photoelectric conversion layer (photoelectrode) of a dye-sensitized solar cell, a functional material for a dielectric layer of a capacitor, a functional material for a semiconductor layer of a transistor, and functional materials for electrodes (transparent electrodes) of these electronic devices.

The porous structure body 10 according to the above-described embodiments can be used as a main component of the functional structure of any of the above-described electronic devices. The porous structure body 10 can also be used as an additional (supplementary) component of the functional structure.

When the porous structure body is used as the material of an electrode of a solar cell, the porous structure body may be used as a component comprised in the electrode formed as a layer or may be provided on the electrode by, for example, joining (bonding), merely, electrically connecting, the porous structure body to the electrode.

When the porous structure body 10 is used, for example, as a functional material for a photoelectric conversion layer of a dye-sensitized solar cell, the photoelectric conversion layer may comprise the porous structure body 10 as a component in an amount of about 0.1% by weight to about 100% by weight.

When a porous structure body 10 is used, for example, as a functional material for a photocatalyst in an antimicrobial material, a deodorizing material, or an antifouling material, a photocatalyst layer may comprise the porous structure body 10 as a component in an amount of about 0.1% by weight to about 100% by weight.

When a porous structure body 10 is used as an additional component, a coexisting main component may be different from the second target material and a precursor of the second target material.

### 3. Method for producing the porous structure body

A method for producing the above-described porous structure body 10 will next be described. The components that have already been described are denoted by the same numerals, and their detailed description will be omitted.

The method for producing the porous structure body 10 comprises the step of: preparing a complex in which the first target material 20 and the second target material or a precursor of the second target material are bound to a multimer of a fusion protein, the complex being prepared by binding the multimer of the fusion protein to the first target material 20, wherein the multimer of the fusion protein has an internal cavity and is formed from the fusion protein that contains a first peptide portion capable of binding to the first target material 20, a second peptide portion capable of binding to the second target material, and a polypeptide portion capable of forming a multimer having an internal cavity, and binding the second target material or the precursor of the second target material to the multimer of the fusion protein; and forming an aggregate body 30 by burning the complex to consume the multimer of the fusion protein, the aggregate body 30 adhering to the first target material 20 and being located so as to surround the first target material 20, the aggregate body 30 having a plurality of first pores 32 unevenly distributed near the first target material 20 and a plurality of second pores 34 scattered over the aggregate body 30.

First, the first target material 20 and the multimer of the fusion protein are prepared. The first target material 20 used may be any of various suitable materials available from the market.

The fusion protein constituting the fusion protein multimer used for the method for producing the porous structure body 10 may comprise a polypeptide portion capable of forming a multimer having an internal cavity, a first peptide portion capable of binding to the first target material 20, and a second peptide portion capable of binding to the second target material.

A specific structure of the fusion protein comprised in the fusion protein multimer used for the method for producing the porous structure body 10 and a specific method for producing the fusion protein will next be described.

The term "polypeptide portion capable of forming a multimer having an internal cavity" refers to a polypeptide portion having an ability to form a multimer having a space inside thereof by association of the polypeptide portions. Several proteins are known as such a polypeptide portion. Examples of such a polypeptide portion may include ferritin capable of forming a 24-meric structure having an internal cavity and a ferritin-like protein capable of forming a multimer having an internal cavity. Examples of the ferritin-like protein capable of forming the multimer having the internal cavity may include Dps capable of forming a 12-meric structure having an internal cavity.

The polypeptide portion capable of forming the multimer having the internal cavity may be naturally occurring proteins derived from any organism such as microorganisms, plants and animals or mutants of the naturally occurring proteins. Hereinafter, the polypeptide portion capable of forming the multimer having the internal cavity may be simply referred to as the polypeptide portion.

In one embodiment, the polypeptide portion capable of forming the multimer having the internal cavity is Dps. The term "Dps (DNA-binding protein from starved cells)" as used herein refers to a protein capable of forming a 12-meric structure having an internal cavity, as described in BACKGROUND ART. The term "Dps" includes naturally occurring Dps or mutants thereof. For the mutants of the naturally occurring Dps, preferred are those exposing its N-terminal part and C-terminal part on the surface of the 12-meric structure upon formation of the 12-meric structure, as is similar to the naturally occurring Dps. Dps may be also referred to as NapA, bacterioferritin, Dlp or MrgA depending on a type of the microorganism from which Dps is derived. Subtypes such as DpsA, DpsB, Dps1 and Dps2 are also known for Dps (see, T. Haikarainen and A. C. Papageorgion, Cell. Mol. Life Sci., 2010 vol. 67, p.341). Therefore, the term "Dps" includes the proteins called by these other names.

The microorganism from which Dps is derived is not particularly limited as long as the microorganism produces Dps. Examples of the microorganism may include bacteria belonging to genera *Listeria, Staphylococcus, Bacillus, Streptococcus, Vibrio, Escherichia, Brucella, Borrelia, Mycobacterium, Gampylobacter, Thermosynechococcus* and *Deinococcus,* and *Corynebacterium*.

Examples of the bacteria belonging to genus Listeria may include *Listeria innocua* and *Listeria monocytogenes*. Examples of the bacteria belonging to genus *Staphylococcus* may include *Staphylococcus aureus*. Examples of the bacteria belonging to genus Bacillus may include *Bacillus subtilis.* Examples of the bacteria belonging to genus *Streptococcus* may include *Streptococcus pyogenes* and *Streptococcus suis*. Examples of the bacteria belonging to genus *Vibrio* may include *Vibrio cholerae*. Examples of the bacteria belonging to genus *Escherichia* may include *Escherichia coli*. Examples of the bacteria belonging to genus *Brucella* may include *Brucella melitensis*. Examples of the bacteria belonging to genus *Borrelia* may include *Borrelia burgdorferi*. Examples of the bacteria belonging to genus *Mycobacterium* may include *Mycobacterium smegmetis*. Examples of the bacteria belonging to genus *Campylobacter* may include *Campylobacter jejuni*. Examples of the bacteria belonging to genus *Thermosynechococcus* may include *Thermosynechococcus elongates*. Examples of the bacteria belonging to genus *Deinococcus* may include *Deinococcus radiodurans*. Examples of the bacteria belonging to genus *Corynebacterium* may include *Corynebacterium glutamicum*.

In preferred embodiments, Dps may be a protein consisting of an amino acid sequence having 70% or more similarity to an amino acid sequence of Dps derived from *Listeria innocua* or *Escherichia coli,* or *Corynebacterium glutamicum.* The percent similarity of the amino acid sequence of Dps to the amino acid sequence of Dps derived from *Listeria innocua* or *Escherichia coli*, or *Corynebacterium glutamicum* can be preferably 75% or more, more preferably 80% or more, still more preferably 85% or more, and most preferably 90% or more, 95% or more, 96% or more, 97% or more, 98% or more, or 99% or more.

Dps has five α-helical segments in its secondary structure (see, A. Ilari et al., Nat. Struct. Biol., 2000, Vol. 7, p.38., R. A. Grant et al. Nat. Struct. Biol. 1998, Vol. 5, p.294., and R. R. Crichton et al., 2010, Vol. 1800, p.706). In terms of retaining a function of Dps, it is important to keep the above secondary structure.

Therefore, when the protein consisting of the amino acid sequence having 70% or more similarity to the amino acid sequence of Dps derived from *Listeria innocua* or *Escherichia coli*, or *Corynebacterium glutamicum* is prepared, a desired mutation can be introduced by a well-known mutagenesis method such as a site-directed mutagenesis so as to keep the above secondary structure. For Dps derived from *Listeria innocua* as an example, the relationships between positions of amino acid residues in the amino acid sequence represented by SEQ ID NO:4 and the above secondary structure are specifically described below from its N-terminal side. (i) The amino acid residues at positions 1 to 8 (an N-terminal region exposed on the surface of a 12-meric structure); (ii) the amino acid residues at positions 9 to 33 (α-helix); (iii) the amino acid residues at positions 39 to 66 (α-helix); (iv) the amino acid residues at positions 75 to 81 (α-helix); (v) the amino acid residue at positions 95 to 122 (α-helix); (vi) the amino acid residue at positions 126 to 149 (α-helix); and (vii) the amino acid residues at positions 150 to 156 (a C-terminal region exposed on the surface of a 12-meric structure). Here, among (i) to (vii) above, (ii) to (vi) can be important for retaining the ability to form the multimer having the internal cavity. For exposing the N-terminal part of Dps on the surface of the 12-meric structure, (i) and (ii), particularly (ii) can be important, since it is required that the α-helix adjacent to the N-terminal part of Dps faces outward the 12-meric structure. For exposing the C-terminal part of Dps on the surface of the 12-meric structure, (vi) and (vii), particularly (vi) can be important, since it is required that the α-helix adjacent to the C-terminal part of Dps faces outward the 12-meric structure.

Therefore, conservative amino acid substitution is preferred when an amino acid residue present in the above important regions is mutated. On the other hand, any mutation may be introduced when an amino acid residue present in the regions other than the aforementioned important regions is mutated. A person skilled in the art can easily prepare the mutant of the naturally occurring Dps by introducing a desired mutation into naturally occurring Dps so as to retain its function based on these guidelines.

The position of the amino acid residue to which the mutation is to be introduced in the amino acid sequence is apparent to a person skilled in the art as described above. However, the mutant of naturally occurring Dps may be prepared further with reference to a sequence alignment. Specifically, a person skilled in the art can recognize correlation between structure and function because a person skilled in the art can 1) compare a plurality of amino acid sequences of Dps (e.g., the amino acid sequence represented by SEQ ID NO:4 and the amino acid sequence of other Dps), 2) demonstrate relatively conserved regions and relatively non-conserved regions, and then 3) predict regions capable of playing an important role for the function and regions incapable of playing an important role for the function from the relatively conserved regions and the relatively non-conserved regions, respectively. Therefore, a person skilled in the art can identify the position to which the mutation is to be introduced in the amino acid sequence of Dps by the aforementioned secondary structure alone, and also can identify the position of the amino acid residue to which the mutation is to be introduced in the amino acid sequence of Dps by combining the secondary structure information and the sequence alignment information.

In one embodiment, the protein consisting of the amino acid sequence having 70% or more similarity to the amino acid sequence of Dps derived from *Listeria innocua* or *Escherichia coli*, or *Corynebacterium glutamicum* may be a protein consisting of an amino acid sequence that comprises one or several mutations of amino acid residues (e.g., deletions, substitutions, additions, and insertions) in the amino acid sequence of Dps derived from *Listeria innocua* or *Escherichia coli*, or *Corynebacterium glutamicum*, and retaining the function of Dps. One or several mutations of the amino acid residues may be introduced into one region or a plurality of different regions in the amino acid sequence. For the mutation of the amino acid residues in Dps, the number represented by the term "one or several" is, for example, 1 to 50, preferably 1 to 30, more preferably 1 to 20, still more preferably 1 to 10, and particularly preferably 1, 2, 3, 4 or 5.

When the amino acid residue is mutated by substitution, the substitution of the amino acid residue may be the conservative substitution. The term "conservative substitution" as used herein refers to that a certain amino acid residue is substituted with an amino acid residue having a similar side chain. Families of the amino acid residues having the similar side chain are well-known in the art. Examples of such a family may include amino acids having a basic side chain (e.g., lysine, arginine, histidine), amino acids having an acidic side chain (e.g., aspartic acid, glutamic acid), amino acids having a non-charged polar side chain (e.g., glycine, asparagine, glutamine, serine, threonine, tyrosine, cysteine), amino acids having a nonpolar side chain (e.g., alanine, valine, leucine, isoleucine, proline, phenylalanine, methionine, tryptophan), amino acids having a branched side chain at position β (e.g., threonine, valine, isoleucine), amino acids having an aromatic side chain (e.g., tyrosine, phenylalanine, tryptophan, histidine), amino acids having a hydroxyl group (alcoholic, phenolic)-containing side chain (e.g., serine, threonine, tyrosine), and amino acids having a sulfur-containing side chain (e.g., cysteine, methionine). Preferably, the conservative substitution of the amino acids may be the substitution between aspartic acid and glutamic acid, the substitution among arginine, lysine and histidine, the substitution between tryptophan and phenylalanine, the substitution between phenylalanine and valine, the substitution among leucine, isoleucine and alanine, and the substitution between glycine and alanine.

In another embodiment, the protein consisting of the amino acid sequence having 70% or more similarity to the amino acid sequence of Dps derived from *Listeria innocua* or *Escherichia coli,* or *Corynebacterium glutamicum* may be a protein encoded by a polynucleotide that hybridizes under a stringent condition with a nucleotide sequence complementary to a nucleotide sequence represented by SEQ ID NO:3 or SEQ ID NO:28, and retains the function of Dps. The "stringent conditions" refers to a condition where a so-called specific hybrid is formed whereas a nonspecific hybrid is not formed. It is difficult to clearly quantify such a condition, but to cite a case, such a condition is a condition where polynucleotides having high homology (e.g., identity or similarity), for example, 70% or more, preferably 80% or more, more preferably 90% or more, still more preferably 95% or more and particularly preferably 98% or more homology are hybridized with each other and polynucleotides having the lower homology than that are not hybridized. Specifically, such a condition may include hybridization in 6×SSC (sodium chloride/sodium citrate) at about 45°C followed by washing once or twice or more with 0.2×SSC and 0.1% SDS at 50 to 65°C.

In a certain embodiment, Dps may be a protein consisting of an amino acid sequence having 70% or more identity to the amino acid sequence of Dps derived from *Listeria innocua* or *Escherichia coli*, or *Corynebacterium glutamicum*. The percent identity of the amino acid sequences of Dps may be preferably 75% or more, more preferably 80% or more, still more preferably 85% or more, most preferably 90% or more, 95% or more, 96% or more, 97% or more, 98% or more, or 99% or more.

The homology (e.g., identity or similarity) of the amino acid sequences and the nucleotide sequences can be determined, for example, using the algorithm BLAST (Pro. Natl. Acad. Sci. USA, 90, 5873 (1993)) by Karlin and Altschul or FASTA (Methods Enzymol., 183, 63 (1990)) by Pearson. The programs referred to as BLASTP and BLASTN were developed based on this algorithm BLAST (see http://www.ncbi.nlm.nih.gov). Thus, the homology of the amino acid sequences and the nucleotide sequences may be calculated using these programs with default setting. Also, for example, a numerical value obtained by calculating the similarity as a percentage at a setting of "unit size to compare=2" using the full length of the polypeptide portion encoded in ORF using software GENETYX Ver. 7.0.9 from Genetyx Corporation employing Lipman-Pearson method may be used as the homology of the amino acid sequences. The lowest value among the values derived from these calculations may be employed as the homology of the nucleotide sequences and the amino acid sequences.

The terms "first peptide portion capable of binding to the first target material" and "second peptide portion capable of binding to the second target material" refer to a portion that has a peptide having an affinity to any target material and can bind to the target material. The first peptide portion and the second peptide portion may be the same or different from each other. Since various peptides having the affinity to the target material are known, a portion having such a peptide can be used as the peptide portion in the present invention. Hereinafter, the first peptide portion and the second peptide portion may be simply referred to as the peptide portion capable of binding to the target material. The expression "the peptide portion capable of binding to the target material" is an expression including the terms "the first peptide portion capable of binding to the first target material" and "the second peptide portion capable of binding to the second target material", and thus, these expressions are interchangeably used. The peptide portion capable of binding to the target material may have only one peptide having the affinity to any target material or may have a plurality of same or different peptides (e.g., several such as 2, 3, 4, 5, or 6) having the affinity to any target material. For example, when the peptide portion capable of binding to the target material has a plurality of different peptides having the affinity to any target material, P1R5 peptide (SSKKSGSYSGSKGSKRRILGGGGHSSYWYAFNNKT [SEQ ID NO:21]) that is a fusion peptide of P1 peptide capable of binding to a carbon nanomaterial (SEQ ID NO:13) and R5 peptide capable of binding to a titanium material or a silicon material (SEQ ID NO:15) can be used as the peptide portion (see, e.g., M. J. Pender et al., Nano Lett., 2006, vol. 6, No.1, p.40-44). When the peptide portion capable of binding to the target material has a plurality of peptides as above, the plurality of peptides can be fused in any order in the peptide portion. The fusion can be accomplished via an amide bond. The fusion can be accomplished directly via the amide bond or via the amide bond through a peptide (a peptide linker) consisting of one amino acid residue (e.g., methionine) or several (e.g., 2 to 50, preferably 2 to 30, more preferably 2 to 20, still more preferably 2 to 15 or 2 to 10, and most preferably 2, 3, 4, or 5) amino acid residues. Since various peptide linkers are known, such a peptide linker can also be used in the present invention.

The peptide portion capable of binding to the target material is not particularly limited as long as it has an affinity to the target material as described above. Various peptides having the affinity to the target material is known and developed. For example, the peptide capable of binding to the inorganic material or the organic material is developed by a technique such as screening using a phage for the purpose of making a complex of the biomaterial and the inorganic material or the organic material. Examples of the peptides developed by such a technique may include peptides capable of binding to the metal materials such as titanium, oxides of titanium and silver (K. Sano et al., Langmuir, 2004, vol.21, p.3090., International Publication No. WO2005/010031), gold (S. Brown, Nat. Biotechnol., 1997, vol.15. p.269), zinc oxide (K. Kjaergaard et al., Appl. Environ. Microbiol., 2000, vol. 66. p.10., and Umetsu et al., Adv. Mater., 17, 2571-2575 (2005)), germanium oxide (M. B. Dickerson et al., Chem. Common., 2004, vol.15. p.1776), and zinc sulfide and cadmium sulfide (C. E. Flynn et al., J. Mater. Chem., 2003, vol.13. p.2414.); peptides capable of binding to the silicon materials such as silicon and oxides of silicon (H. Chen et al., Anal. Chem., 2006, vol.78, p.4872, M. J. Pender et al., Nano Lett., 2006, vol. 6, No.1, p.40-44, and K. Sano et al., Langmuir, 2004, vol.21, p.3090., and International Publication No. WO2005/010031); peptides capable of binding to the carbon materials such as carbon nanotube (CNT) and carbon nanohorn (CNH) (S. Wang et al., Nat. Mater., 2003, vol. 2, p.196. and JP 2004-121154 A); and peptides capable of binding to the polymers such as hydrophobic organic polymers (JP 2008-133194 A). Therefore, such a peptide can also be used as the peptide portion capable of binding to the target material in the present invention.

It is known that the peptide capable of binding to the metal is able to have an action for deposition of the metal, and the peptide capable of binding to the metal compound is able to have an action for deposition of the metal compound (K. Sano et al., Langmuir, 2004, vol. 21, p.3090., and M. Umetsu et al., Adv. Mater., 2005, vol. 17, p.2571.). Therefore, when a peptide capable of binding to the metal material (metal or metal compound) is used as the peptide capable of binding to the target material, the peptide capable of binding to the metal material can have such an action for the deposition.

The fusion of the polypeptide portion and the first and second peptide portions can be accomplished via amide bonds. The fusion can be accomplished directly via the amide bond or via the amide bond through a peptide (a peptide linker) consisting of one amino acid residue (e.g., methionine) or several (e.g., 2 to 50, preferably 2 to 30, more preferably 2 to 20, still more preferably 2 to 15 or 2 to 10, and most preferably 2, 3, 4, or 5) amino acid residues. Since various peptide linkers are known, such a peptide linker can also be used in the present invention.

The order of fusing the polypeptide portion and the first and second peptide portions in the fusion protein is not particularly limited, 1) the N-terminal part and the C-terminal part of the polypeptide portion may be fused to the C-terminal part and the N-terminal part (or the N-terminal part and the C-terminal part) of the first and second peptide portions, respectively, or 2) the N-terminal part of the polypeptide portion may be fused to the C-terminal part of the first peptide portion and the N-terminal part of the first peptide portion may further be fused to the C-terminal part of the second peptide portion, or 3) the C-terminal part of the polypeptide portion may be fused to the N-terminal part of the first peptide portion and the C-terminal part of the first peptide portion may further be fused to the N-terminal part of the second peptide portion. For example, when ferritin is used as the polypeptide portion, ferritin is preferably fused in the order of 2) above, since the N-terminal part of ferritin is exposed on the surface of the multimer whereas the C-terminal part is not exposed on the surface. On the other hand, when Dps is used as the polypeptide portion, Dps may be fused in any of the orders of 1) to 3) above, since both the N-terminal part and the C-terminal part of Dps can be exposed on the surface of the multimer.

In preferred embodiments, the fusion protein can have the first peptide portion and the second peptide portion (one or plurality, respectively) on an N-terminal side and on a C-terminal side of the polypeptide portion, respectively. In other words, the C-terminal part of the first peptide portion is fused to the N-terminal part of the polypeptide portion and the N-terminal part of the second peptide portion is fused to the C-terminal part of the polypeptide portion.

The first peptide portion can be designed so as to have methionine encoded by a translation initiation codon or a portion including methionine at its N-terminus on the N-terminal side of the first peptide portion. The translation of the fusion protein can be facilitated by such a design. The peptide portion including methionine at the N-terminus may be a peptide consisting of several (e.g., 2 to 50, preferably 2 to 30, more preferably 2 to 20, still more preferably 2 to 15 or 2 to 10, and most preferably 2, 3, 4, or 5) amino acid residues.

In preferred embodiments, the first peptide portion and the second peptide portion in the fusion protein can bind to the different target material. Examples of a combination of the target materials to which the first peptide portion and the second peptide portion are bound may include a combination of the inorganic material and the organic material, a combination of two inorganic materials, and a combination of two organic materials. More specifically, such combinations may include a combination of the metal material and the silicon material, a combination of the metal material and the carbon material, a combination of the silicon material and the carbon material, a combination of two metal materials, a combination of two silicon materials, and a combination of two carbon materials. Therefore, the combination of the first peptide portion and the second peptide portion may be a combination of the peptide portions capable of binding to the target materials described above.

In still preferred embodiments, one of the first and second peptide portions may bind to the carbon material and the other may bind to the metal material or the silicon material in the fusion protein. In other words, the fusion protein of the present invention has a peptide portion capable of binding to the carbon material as the first peptide portion and has a peptide portion capable of binding to the metal material or the silicon material as the second peptide portion, or alternatively, has the peptide portion capable of binding to the metal material or the silicon material as the first peptide portion and has the peptide portion capable of binding to the carbon material as the second peptide portion.

For the peptide portion capable of binding to the carbon material, a peptide portion capable of binding to a carbon nanomaterial such as carbon nanotube (CNT) or carbon nanohorn (CNH) is preferred. Examples of such a peptide may include DYFSSPYYEQLF (SEQ ID NO:6) disclosed in Examples described later and JP Publication No. 2004-121154, HSSYWYAFNNKT (SEQ ID NO:13) disclosed in M. J. Pender et al., Nano Lett., 2006, vol. 6, No.1, p.40-44, and YDPFHII (SEQ ID NO:14) disclosed in JP Publication No. 2004-121154, or mutant peptides thereof (e.g., mutation such as conservative substitution for 1, 2, 3, 4 or 5 amino acid residues), or peptides having one or a plurality of such amino acid sequences.

For the peptide portion capable of binding to the metal material, a peptide portion capable of binding to a titanium material such as titanium or a titanium compound (e.g., a titanium oxide), and a peptide portion capable of binding to a zinc material such as zinc or a zinc compound (e.g., a zinc oxide) are preferred. Examples of the peptide portion capable of binding to the titanium material may include RKLPDA (SEQ ID NO:8) disclosed in Examples described later and International Publication No. WO2006/126595, SSKKSGSYSGSKGSKRRIL (SEQ ID NO:15) disclosed in M. J. Pender et al., Nano Lett., 2006, vol. 6, No.1, p.40-44, and RKLPDAPGMHTW (SEQ ID NO:16) and LPDA (SEQ ID NO:17) disclosed in International Publication No. WO2006/126595 or mutant peptides thereof (e.g., mutation by conservative substitution of 1, 2, 3, 4 or 5 amino acid residues), or peptides having one or several such an amino acid sequence. Examples of the peptide portion capable of binding to the zinc material may include EAHVMHKVAPRPGGGSC (SEQ ID NO:30) disclosed in Example described later and Umetsu et al., Adv. Mater., 17, 2571-2575 (2005), or mutant peptides thereof (e.g., mutation such as conservative substitution for 1, 2, 3, 4 or 5 amino acid residues), or peptides having one or a plurality of such amino acid sequences.

For the peptide portion capable of binding to the silicon material, a peptide portion capable of binding to silicon or a silicon compound (e.g., an oxide of silicon) is preferred. Examples of such a peptide portion may include RKLPDA (SEQ ID NO:8) disclosed in Examples described later and International Publication No. WO2006/126595 SSKKSGSYSGSKGSKRRIL (SEQ ID NO:15) disclosed in M. J. Pender et al., Nano Lett., 2006, vol. 6, No.1, p.40-44, and MSPHPHPRHHHT (SEQ ID NO:18), TGRRRRLSCRLL(SEQ ID NO:19) and KPSHHHHHTGAN (SEQ ID NO:20) disclosed in International Publication No. WO2006/126595, or mutant peptides thereof (e.g., mutation such as conservative substitution for 1, 2, 3, 4 or 5 amino acid residues), or peptides having one or a plurality of such amino acid sequences.

In a specific embodiment, the fusion protein may be a protein consisting of, or comprising an amino acid sequence having 90% or more identity to an amino acid sequence represented by SEQ ID NO:2, SEQ ID NO:27, or SEQ ID NO:32. The percent identity of the amino acid sequence of the fusion protein to the amino acid sequence represented by SEQ ID NO:2, SEQ ID NO:27, or SEQ ID NO:32 may be preferably 95% or more, more preferably 96% or more, still more preferably 97% or more, and particularly preferably 98% or more or 99% or more.

The fusion protein can be obtained from a transformant that expresses the fusion protein. This transformant can be prepared by making an expression vector for the fusion protein comprising a polynucleotide encoding the fusion protein and then introducing this expression vector into a host. Examples of the host for expressing the fusion protein may include various prokaryotic cells including bacteria belonging to genera *Escherichia* (*Escherichia coli*) and *Corynebacterium*, and *Bacillus subtilis,* and various eukaryotic cells including *Saccharomyces cerevisiae, Pichia stipitis* and *Aspergillus* oryzae.

*E. coli* as the host to be transformed will be described in detail. Examples of *E. coli* may include *E. coli* JM109 strain, DH5α strain, HB101 strain, and BL21 (DE3) strain that are subtypes of *E. coli* K12 strain. Methods of transformation and methods of selecting the transformant are described in Molecular Cloning: A Laboratory Manual, 3rd edition, Cold Spring Harbor press (2001/01/15). A method of making transformed *E. coli* and producing the fusion protein using this will be specifically described below as one example.

For a promoter for expressing a DNA encoding the fusion protein, a promoter generally used for the production of a heterologous protein in *E. coli* can be used. Examples of the promoter may include strong promoters such as a T7 promoter, a lac promoter, a trp promoter, a trc promoter, a tac promoter, a PR promoter and a PL promoter of lambda phage, and a T5 promoter. Examples of a vector may include pUC19, pUC18, pBR322, pHSG299, pHSG298, pHSG399, pHSG398, RSF1010, pACYC177, pACYC184, pMW119, pMW118, pMW219, pMW218, pQE30, and derivatives thereof.

A terminator that is a transcription termination sequence may be ligated downstream of a gene encoding the fusion protein. Examples of such a terminator may include a T7 terminator, a fd phage terminator, a T4 terminator, a terminator of a tetracycline resistant gene, and a terminator of an *E. coli* trpA gene.

The vector for introducing a gene encoding the fusion protein is preferably a so-called multicopy type, and may include a plasmid having a replication origin derived from ColE1, for example, pUC-based plasmids and pBR322-based plasmids or derivatives thereof. Here, the "derivative" means a plasmid modified by substitution, deletion, insertion, addition, and/or inversion of a nucleotide(s). The "modification" referred to herein includes a mutation treatment by a mutating agent or irradiation with UV or the modification by natural mutation.

The vector preferably has a marker such as an ampicillin resistant gene for selecting the transformant. The expression vectors having the strong promoter are commercially available (e.g., pUC-based vectors manufactured by Takara Bio Inc., pPROK-based vectors manufactured by Clontech, and pKK233-2 manufactured by Clontech).

When *E. coli* is transformed with the obtained expression vector and the resulting *E. coli* is cultured, the fusion protein is expressed.

Examples of culture media may include media such as M9-casamino acid medium and LB medium generally used for culturing *E. coli.* Conditions on cultivation, production induction and the like can be selected appropriately depending on the types of the marker in the vector used, the promoter, the host microorganism and the like.

The following method is available for recovering the fusion protein. The fusion protein can be obtained as a disrupted product or a lysed product by collecting the transformant that produces the fusion protein followed by disrupting (e.g., sonication or homogenization) or lysing (e.g., a lysozyme treatment) the transformant. A purified protein, a crude purified protein, or a fraction containing the fusion protein can be obtained by subjecting such a disrupted product or lysed product to a technique such as extraction, precipitation, filtration, or column chromatography.

The following description relates to a polynucleotide encoding the fusion protein and an expression vector comprising the polynucleotide and a transformant comprising the expression vector, as described above, which can be used for preparing the fusion protein.

The polynucleotide which can be used for preparing the fusion protein (refer to "the polynucleotide") can comprise a polynucleotide portion encoding the polypeptide portion capable of forming the multimer having the internal cavity, and a polynucleotide portion encoding the first peptide portion capable of binding to the first target material, and a polynucleotide portion encoding the second peptide portion capable of binding to the second target material. The polynucleotide can be specified from various points based on the aforementioned descriptions on the fusion protein, since it encodes the fusion protein.

In a specific embodiment, the polynucleotide may be a polynucleotide consisting of, or comprising a nucleotide sequence having 90% or more identity to a nucleotide sequence represented by SEQ ID NO:1, SEQ ID NO:26, or SEQ ID NO:31. The percent identity of the nucleotide sequence of the polynucleotide to the nucleotide sequence represented by SEQ ID NO:1, SEQ ID NO:26, or SEQ ID NO:31 may be preferably 95% or more, more preferably 96% or more, still more preferably 97% or more, and particularly preferably 98% or more or 99% or more.

The following description relates to a multimer of the fusion protein. The multimer can have the internal cavity. The fusion protein that composes the multimer is as described above. The multimer can be formed autonomously by expressing the fusion protein. The number of monomer units that compose the multimer can be determined by the type of the polypeptide portion in the fusion protein. Preferably, the multimer may be a 12-meric structure, since it may have Dps as the polypeptide portion capable of forming the multimer having the internal cavity.

The multimer of the fusion protein may be a homomultimer composed of a single fusion protein as the monomer unit or may be a heteromultimer composed of a plurality of different fusion proteins (e.g., 2, 3, 4, 5, or 6). In the multimer of the fusion protein, the polypeptide portion in the fusion protein that composes the multimer of the fusion protein is preferably a single polypeptide portion in terms of forming the multimer of the fusion protein, but the first peptide portion and the second peptide portion may be different in the fusion proteins that compose the multimer of the fusion protein. For example, when the multimer of the fusion protein is composed of two types of the fusion proteins and the polypeptide portion in the fusion protein has the peptide portions fused on its N-terminal side and C-terminal side, respectively, examples of a combination of two types of the fusion proteins may include the followings:
(i) combination of the first peptide portion (a)-the polypeptide portion-the second peptide portion (b) and the first peptide portion (c)-the polypeptide portion-the second peptide portion (d);
(ii) combination of the first peptide portion (a)-the polypeptide portion-the second peptide portion (b) and the first peptide portion (a)-the polypeptide portion-the second peptide portion (c);
(iii) combination of the first peptide portion (a)-the polypeptide portion-the second peptide portion (b) and the first peptide portion (c)-the polypeptide portion-the second peptide portion (b);
(iv) combination of the first peptide portion (a)-the polypeptide portion-the second peptide portion (b) and the first peptide portion (c)-the polypeptide portion-the second peptide portion (a);
(v) combination of the first peptide portion (a)-the polypeptide portion-the second peptide portion (a) and the first peptide portion (b)-the polypeptide portion-the second peptide portion (c);
(vi) combination of the first peptide portion (a)-the polypeptide portion-the second peptide portion (b) and the first peptide portion (b)-the polypeptide portion-the second peptide portion (a);
(vii) combination of the first peptide portion (a)-the polypeptide portion-the second peptide portion (a) and the first peptide portion (a)-the polypeptide portion-the second peptide portion (b); and
(viii) combination of the first peptide portion (a)-the polypeptide portion-the second peptide portion (a) and the first peptide portion (b)-the polypeptide portion-the second peptide portion (a).
[Note that, (a) to (d) represent different peptide portions (e.g., peptide portions capable of binding to the different target material). (i) is a manner utilizing four types of the peptide portions, (ii) to (v) are manners utilizing three types of the peptide portions, and (vi) to (viii) are manners utilizing two types of the peptide portions.]

Specifically, when the multimer of the fusion protein is composed of two types of the fusion proteins and at least a peptide portion capable of binding to the carbon material and a peptide portion capable of binding to the metal material (e.g., titanium material, zinc material) or the silicon material are used as the peptide portions, in terms of changing the electric properties of a device to be made using the multimer of the fusion protein, examples of the combination of two types of the fusion proteins may include the followings:
(i-1) combination of the peptide portion capable of binding to the carbon material-the polypeptide portion-the peptide portion capable of binding to the metal material or the silicon material and a peptide portion capable of binding to a first other material-a peptide portion capable of binding to a second other material;
(i-2) combination of the peptide portion capable of binding to the metal material or the silicon material-the polypeptide portion-the peptide portion capable of binding to the carbon material and the peptide portion capable of binding to the first other material-the polypeptide portion-the peptide portion capable of binding to the second other material;
(i-3) combination of the peptide portion capable of binding to the carbon material-the polypeptide portion-the peptide portion capable of binding to the first other material and the peptide portion capable of binding to the metal material or the silicon material-the polypeptide portion-the peptide portion capable of binding to the second other material;
(i-4) combination of the peptide portion capable of binding to the carbon material-the polypeptide portion-the peptide portion capable of binding to the first other material and the peptide portion capable of binding to the second other material-the polypeptide portion-the peptide portion capable of binding to the metal material or the silicon material;
(i-5) combination of the peptide portion capable of binding to the first other material-the polypeptide portion-the peptide portion capable of binding to the carbon material and the peptide portion capable of binding to the metal material or the silicon material-the polypeptide portion-the peptide portion capable of binding to the second other material;
(i-6) combination of the peptide portion capable of binding to the first other material-the polypeptide portion-the peptide portion capable of binding to the second other material and the peptide portion capable of binding to the metal material or the silicon material-the polypeptide portion-the peptide portion capable of binding to the carbon material;
(ii-1) combination of the peptide portion capable of binding to the carbon material-the polypeptide portion-the peptide portion capable of binding to the metal material or the silicon material and the peptide portion capable of binding to the carbon material-the polypeptide portion-the peptide portion capable of binding to the first other material;
(ii-2) combination of the peptide portion capable of binding to the carbon material-the polypeptide portion-the peptide portion capable of binding to the first other material and the peptide portion capable of binding to the carbon material-the polypeptide portion-the peptide portion capable of binding to the metal material or the silicon material;
(ii-3) combination of the peptide portion capable of binding to the metal material or the silicon material-the polypeptide portion-the peptide portion capable of binding to the carbon material and the peptide portion capable of binding to the metal material or the silicon material-the polypeptide portion-the peptide portion capable of binding to the first other material;
(ii-4) combination of the peptide portion capable of binding to the metal material or the silicon material-the polypeptide portion-the peptide portion capable of binding to the first other material and the peptide portion capable of binding to the metal material or the silicon material-the polypeptide portion-the peptide portion capable of binding to the carbon material;
(ii-5) combination of the peptide portion capable of binding to the first other material-the polypeptide portion-the peptide portion capable of binding to the metal material or the silicon material and the peptide portion capable of binding to the first other material-the polypeptide portion-the peptide portion capable of binding to the carbon material;
(ii-6) combination of the peptide portion capable of binding to the first other material-the polypeptide portion-the peptide portion capable of binding to the carbon material and the peptide portion capable of binding to the first other material-the polypeptide portion-the peptide portion capable of binding to the metal material or the silicon material;
(iii-1) combination of the peptide portion capable of binding to the carbon material-the polypeptide portion-the peptide portion capable of binding to the metal material or the silicon material and the peptide portion capable of binding to the first other material-the polypeptide portion-the peptide portion capable of binding to the metal material or the silicon material;
(iii-2) combination of the peptide portion capable of binding to the carbon material-the polypeptide portion-the peptide portion capable of binding to the first other material and the peptide portion capable of binding to the metal material or the silicon material-the polypeptide portion-the peptide portion capable of binding to the first other material;
(iii-3) combination of the peptide portion capable of binding to the metal material or the silicon material-the polypeptide portion-the peptide portion capable of binding to the carbon material and the peptide portion capable of binding to the first other material-the polypeptide portion-the peptide portion capable of binding to the carbon material;
(iii-4) combination of the peptide portion capable of binding to the metal material or the silicon material-the polypeptide portion-the peptide portion capable of binding to the first other material and the peptide portion capable of binding to the carbon material-the polypeptide portion-the peptide portion capable of binding to the first other material;
(iii-5) combination of the peptide portion capable of binding to the first other material-the polypeptide portion-the peptide portion capable of binding to the metal material or the silicon material and the peptide portion capable of binding to the carbon material-the polypeptide portion-the peptide portion capable of binding to the metal material or the silicon material;
(iii-6) combination of the peptide portion capable of binding to the first other material-the polypeptide portion-the peptide portion capable of binding to the carbon material and the peptide portion capable of binding to the metal material or the silicon material-the polypeptide portion-the peptide portion capable of binding to the carbon material;
(iv-1) combination of the peptide portion capable of binding to the carbon material-the polypeptide portion-the peptide portion capable of binding to the metal material or the silicon material and the peptide portion capable of binding to the first other material-the polypeptide portion-the peptide portion capable of binding to the carbon material;
(iv-2) combination of the peptide portion capable of binding to the carbon material-the polypeptide portion-the peptide portion capable of binding to the first other material and the peptide portion capable of binding to the metal material or the silicon material-the polypeptide portion-the peptide portion capable of binding to the carbon material;
(iv-3) combination of the peptide portion capable of binding to the metal material or the silicon material-the polypeptide portion-the peptide portion capable of binding to the carbon material and the peptide portion capable of binding to the first other material-the polypeptide portion-the peptide portion capable of binding to the metal material or the silicon material;
(iv-4) combination of the peptide portion capable of binding to the metal material or the silicon material-the polypeptide portion-the peptide portion capable of binding to the first other material and the peptide portion capable of binding to the carbon material-the polypeptide portion-the peptide portion capable of binding to the metal material or the silicon material;
(iv-5) combination of the peptide portion capable of binding to the first other material-the polypeptide portion-the peptide portion capable of binding to the metal material or the silicon material and the peptide portion capable of binding to the carbon material-the polypeptide portion-the peptide portion capable of binding to the first other material;
(iv-6) combination of the peptide portion capable of binding to the first other material-the polypeptide portion-the peptide portion capable of binding to the carbon material and the peptide portion capable of binding to the metal material or the silicon material-the polypeptide portion-the peptide portion capable of binding to the first other material;
(v-1) combination of the peptide portion capable of binding to the carbon material-the polypeptide portion-the peptide portion capable of binding to the carbon material and the peptide portion capable of binding to the metal material or the silicon material-the polypeptide portion-the peptide portion capable of binding to the first other material;
(v-2) combination of the peptide portion capable of binding to the carbon material-the polypeptide portion-the peptide portion capable of binding to the carbon material and the peptide portion capable of binding to the first other material-the polypeptide portion-the peptide portion capable of binding to the metal material or the silicon material;
(v-3) combination of the peptide portion capable of binding to the metal material or the silicon material-the polypeptide portion-the peptide portion capable of binding to the metal material or the silicon material and the peptide portion capable of binding to the carbon material-the polypeptide portion-the peptide portion capable of binding to the first other material;
(v-4) combination of the peptide portion capable of binding to the metal material or the silicon material-the polypeptide portion-the peptide portion capable of binding to the metal material or the silicon material and the peptide portion capable of binding to the first other material-the polypeptide portion-the peptide portion capable of binding to the carbon material;
(v-5) combination of the peptide portion capable of binding to the first other material-the polypeptide portion-the peptide portion capable of binding to the first other material and the peptide portion capable of binding to the metal material or the silicon material-the polypeptide portion-the peptide portion capable of binding to the carbon material;
(v-6) combination of the peptide portion capable of binding to the first other material-the polypeptide portion-the peptide portion capable of binding to the first other material and the peptide portion capable of binding to the carbon material-the polypeptide portion-the peptide portion capable of binding to the metal material or the silicon material;
(vi) combination of the peptide portion capable of binding to the carbon material-the polypeptide portion-the peptide portion capable of binding to the metal material or the silicon material and the peptide portion capable of binding to the metal material or the silicon material-the polypeptide portion-the peptide portion capable of binding to the carbon material;
(vii-1) combination of the peptide portion capable of binding to the carbon material-the polypeptide portion-the peptide portion capable of binding to the carbon material and the peptide portion capable of binding to the carbon material-the polypeptide portion-the peptide portion capable of binding to the metal material or the silicon material;
(vii-2) combination of the peptide portion capable of binding to the metal material or the silicon material-the polypeptide portion-the peptide portion capable of binding to the metal material or the silicon material and the peptide portion capable of binding to the metal material or the silicon material-the polypeptide portion-the peptide portion capable of binding to the carbon material;
(viii-1) combination of the peptide portion capable of binding to the carbon material-the polypeptide portion-the peptide portion capable of binding to the carbon material and the peptide portion capable of binding to the metal material or the silicon material-the polypeptide portion-the peptide portion capable of binding to the carbon material; and
(viii-2) combination of the peptide portion capable of binding to the metal material or the silicon material-the polypeptide portion-the peptide portion capable of binding to the metal material or the silicon material and the peptide portion capable of binding to the carbon material-the polypeptide portion-the peptide portion capable of binding to the metal material or the silicon material.

The multimer of the fusion protein composed of a plurality of different types of the fusion proteins can be obtained by, for example, introducing a plurality of vectors expressing the different types of the fusion proteins or a single vector expressing the different types of the fusion proteins (e.g., vector capable of expressing polycistronic mRNA) into a single host cell and then expressing the different types of the fusion proteins in the single host cell. Such a multimer of the fusion protein can also be obtained by allowing a first monomer composed of a single fusion protein and a second monomer composed of a single fusion protein (different from the fusion protein that composes the first monomer) to coexist and be left stand in the same vehicle (e.g., buffer solution). The monomer of the fusion protein can be prepared by, for example, leaving stand the multimer of the fusion protein in buffer solution at low pH. For details, see, for example, B. Zheng et al., Nanotechnology, 2010, vol. 21, p.445602.

The multimer of the fusion protein may comprise a substance in its internal cavity. The substance in a form of a complex or a particle (e.g., nanoparticle, magnetic particle) may be encapsulated in the multimer of the fusion protein. A person skilled in the art can appropriately select a substance that can be encapsulated in the multimer of the fusion protein by considering a size of the internal cavity of the multimer of the fusion protein, a charge property of the amino acid residues in regions involved in encapsulation of the substance in the multimer of the fusion protein (e.g., C-terminal region: see R. M. Kramer et al., 2004, J. Am. Chem. Soc., vol. 126, p.13282), and the like. For example, when the multimer of the fusion protein has Dps as the polypeptide portion, Dps has an internal cavity of about 40 to 60 nm³ (diameter: about 5 nm).

Therefore, the size of the substance that can be encapsulated in such a multimer of the fusion protein can be, for example, 60 nm³ or less, preferably 40 nm³ or less, more preferably 20 nm³ or less, still more preferably 10 nm³ or less, and most preferably 5 nm³ or less.

It is also reported that the encapsulation of the substance into the internal cavity of the multimer of the fusion protein can further be facilitated by changing the charge property in the region that can be involved in the encapsulation of the substance in the multimer (e.g., type and number of amino acid residues having a side chain that can be charged positively or negatively) (see, .g., R. M. Kramer et al., 2004, J. Am. Chem. Soc., vol. 126, p.13282). Therefore, the multimer of the fusion protein having the region in which the charge property is changed can also be used in the present invention. Examples of the substance that can be encapsulated in the multimer of the fusion protein may include inorganic materials as is similar to the aforementioned target materials. Specifically, the substances that can be encapsulated in the multimer of the fusion protein may include the metal materials and the silicon materials as described above. The substance that can be encapsulated in the multimer of the fusion protein is preferably metal particles (metal nanoparticles) different from the second target material. More specifically, such a substance may include iron oxides, nickel, cobalt, manganese, phosphorus, uranium, beryllium, aluminium, cadmium sulfide, cadmium selenide, palladium, chromium, copper, silver, gadolium complex, platinum cobalt, silicon oxide, cobalt oxide, indium oxide, platinum, gold, gold sulfide, zinc selenide, and cadmium selenium.

The encapsulation of the substance in the internal cavity of the multimer of the fusion protein can be carried out by known methods. For example, it can be carried out in the same manner as in the method of encapsulating the substance in the internal cavity of the multimer of ferritin or a ferritin-like protein such as Dps (see, e.g., I. Yamashita et al., Chem., Lett., 2005. vol. 33, p.1158). Specifically, the substance can be encapsulated in the internal cavity of the multimer by allowing the multimer of the fusion protein (or fusion protein) and the substance to be encapsulated to coexist in the buffer solution such as HEPES buffer solution and then leaving them stand at an appropriate temperature (e.g., 0°C to 37°C).

The multimer of the fusion protein may be provided as a set of a plurality of different types of the multimers comprising a plurality (e.g., 2, 3, 4, 5 or 6) of different types of the substances when comprising the substance in the internal cavity. For example, when the multimer of the fusion protein is provided as a set of two types of the multimers comprising two types of the substances, such a set can be obtained by combining a first multimer of the fusion protein encapsulating a first substance and a second multimer of the fusion protein encapsulating a second substance (different from the first substance), which are each prepared separately. Highly diverse multimers of the present invention can be obtained by appropriately combining diversified patterns of the fusion proteins with diversified patterns of the substances to be encapsulated, as described above.

The above-described multimer of the fusion protein can be preferably used for the method for producing the porous structure body 10.

The production method will next be specifically described.

### (1) Step of preparing complex in which first target material and second target material are bound to multimer of fusion protein.

First, the multimer of the fusion protein is prepared. When the multimer of the fusion protein further comprises the metal particles 36 described above with reference to FIGs. 1C and 1E, the metal particles 36 are encapsulated in the internal cavities of the multimer of the fusion protein in this stage.

As described above, the metal particles 36 can be encapsulated in the internal cavities of the multimer of the fusion protein by allowing the multimer of the fusion protein (or the fusion protein) and the substance to be encapsulated to coexist in a buffer solution such as a HEPES buffer solution and then leaving them to stand at a suitable temperature (e.g., 0°C to 37°C).

Next, the multimer of the fusion protein and the first target material 20 are bound to each other to obtain a combination body in which the multimer of the fusion protein and the first target material 20 are bound to each other. The binding of the multimer of the fusion protein to the first target material 20 can be performed by a step suitable for the selected first target material 20.

This step may be performed using, for example, a multimer of a fusion protein that binds to a plurality of different types of second target materials.

The multimer of the fusion protein and the first target material 20 may be bound to each other by, for example, mixing the multimer of the fusion protein and the first target material 20 in water or a buffer solution.

Examples of the buffer solution that can be used to bind the multimer of the fusion protein to the first target material 20 may include a phosphate buffer solution and a Good's buffer solution.

The hydrogen-ion concentration exponent (pH) of the buffer solution may be adjusted to any suitable range according to the selected material. Particularly, when CNT is used as the first target material 20, the pH is preferably within the range of about 6 to about 9 and more preferably within the range of about 6 to about 7 because the binding between the multimer of the fusion protein and the first target material 20 can be favorable.

The buffer solution used for binding may further comprise a component such as sodium chloride (NaCl) or ammonium sulfate for the purpose of, for example, changing the surface charge or hydrophilicity of the multimer of the fusion protein.

When a nanomaterial such as CNT is used as the first target material 20, the protein component in the multimer of the fusion protein functions as a dispersant, so that an additional component such as a surfactant may not be required to be added.

To facilitate mixing and binding, additional treatment such as sonication or stirring may be performed.

When the first target material 20 is, for example, CNT, water or a buffer solution containing the CNT and the multimer of the fusion protein at preferred concentrations is prepared and subjected to sonication for binding, whereby the multimer of the fusion protein and the first target material 20 can be bound to each other.

Next, the second target material is further bound to the multimer of the fusion protein to which the first target material 20 has been bound.

The binding of the second target material further to the multimer of the fusion protein to which the first target material 20 has been bound may be performed by a step suitable for the selected second target material.

The second target material may be bound to the multimer of the fusion protein by, for example, mixing the second target material and the multimer of the fusion protein to which the first target material 20 has been bound in water, an aqueous ethanol solution, or a buffer solution.

Examples of the buffer solution used to bind the second target material to the multimer of the fusion protein may include a phosphate buffer solution and a Good's buffer solution.

The binding of the second target material to the multimer of the fusion protein may be performed by controlling conditions such as pH within a suitable range in consideration of the selected material, the catalytic activity of the multimer of the fusion protein and the like. Particularly, when a metal is used as the second target material or a precursor thereof, it is preferable to perform the binding under the conditions under which the catalytic activity of the multimer of the fusion protein becomes higher.

The buffer solution used to bind the second target material to the multimer of the fusion protein may further comprise an additional component such as NaCl, polyethylene glycol, or tween (registered trademark) 20 for the purpose of, for example, controlling the charged states of the multimer of the fusion protein and the second target material and controlling the dispersibility of the second target material. To facilitate mixing and binding, additional treatment such as sonication, stirring, heating, or cooling may be performed.

When the first target material 20 is, for example, CNT, the second target material is added to water or a buffer solution comprising a suitable concentration of a combination body of the CNT and the multimer of the fusion protein bound to each other to further bind the second target material to the combination body, whereby a complex in which the first target material 20 and the second target material are bound to the multimer of the fusion protein can be obtained.

This step may be performed using a precursor of the second target material instead of the second target material. The precursor of the second target material is a material that is a selected precursor and can be converted to the second target material by a catalyst such as a protein (a multimer of a fusion protein), a functional peptide, or an acid or a material that can be converted to the second target material by subjecting the selected precursor to any suitable treatment such as burning (heat treatment).

When the second target material is, for example, titanium oxide, any of, for example, titanium(IV) bis(ammonium lactato)dihydroxide, titanium(IV) 2-ethylhexyloxide, titanium ethoxide, titanium isopropoxide, and titanium n-butoxide may be used as the precursor of the second target material.

The precursors of the second target material, namely, titanium(IV) bis(ammonium lactato)dihydroxide, titanium ethoxide, titanium 2-ethylhexyloxide, titanium isopropoxide, and titanium n-butoxide can be converted to titanium oxide, namely, the second target material, by interaction with biomolecules such as proteins or functional peptides, acid treatment, or heat treatment.

In the above-described step, internal cavities (and the metal particles 36 encapsulated in the internal cavities) are fixed in the second target material or the precursor of the second target material bound to the multimer of the fusion protein at a prescribed distance from the surface of the first target material 20 through the multimer of the fusion protein. In other words, the plurality of internal cavities (the metal particles 36) comprised in the multimer of the fusion protein are fixed at positions spaced substantially equal distance (corresponding to the distance S1 in FIGs. 1B to 1E) from the surface of the first target material 20.

For example, the distance S1 can be controlled as follows. (1) Deposition conditions (reaction time, pH, etc.) for the second target material are controlled. (2) The length of a binding peptide to be bound to the first target material 20 and/or the length of a linker between the binding peptide and the polypeptide portion capable of forming an internal cavity is changed (the type of fusion protein is changed). Alternatively, (3) first, the second target material is deposited around the first target material 20 using a fusion protein and the like (the second target material deposited in this stage may not form first pores 32). Secondly, a fusion protein capable of binding to the second target material and capable of forming an internal cavity is bound to the deposited second target material, and a new second target material that encapsulates the fusion protein capable of forming an internal cavity is deposited around the first deposited second target material.

To increase the thickness T1 of the aggregate body 30 to be formed, the precursor of the second target material and/or the second target material itself may be mixed with a solvent such as water or a buffer solution to form a paste-like composition, and the paste-like composition may be supplied in the step of binding the second target material to the multimer of the fusion protein, thus depositing the precursor of the second target material and/or the second target material itself around the first target material 20.

It is known that, in the fusion protein (the multimer of the fusion protein), a peptide portion capable of binding to, for example, a metal serving as the second target material can have an action of depositing the metal and a peptide portion capable of binding to a metal compound can have an action of depositing the metal compound, as described above.

Therefore, when a peptide portion capable of binding to a metal material (a metal or a metal compound) and having the above-described structure is used at the peptide portion capable of binding to the second target material, the peptide portion capable of binding to a metal material can have the deposition action of depositing the metal material around the vicinity of the peptide portion.

When the second target material or the precursor of the second target material is a metal material, the thickness T1 can be increased by further supplying the precursor of the second target material and/or the second target material itself to deposit it and performing a burning step described later.

Since the fusion protein (the multimer of the fusion protein) used has the above-described deposition action, no additional means for deposition is necessary, and the porous structure body and also a functional structure comprising the porous structure body can be formed with a simpler step.

Deposition can be performed under any known suitable conditions. When the second target material and/or the precursor of the second target material is deposited, the thickness T1 of the aggregate body 30 can be controlled by controlling, for example, reaction time, the concentration of the second target material and/or the precursor of the second target material and the like.

When the peptide portion capable of binding to a metal material (a metal or a metal compound) and having the above-described structure is used, a metal material used as the second target material can be deposited around the first target material 20 at room temperature (about 20°C), thus forming the aggregate body 30.

### (2) Step of burning complex body to form aggregate body

Next, the obtained complex is subjected to burning (heat treatment) to consume the multimer of the fusion protein. In this step, the aggregate body 30 is formed which comprises the second target material located so as to surround the first target material 20 and adhering to the first target material 20.

To burn the complex to form the aggregate body 30, any known suitable method may be used. Specific examples of such a method may include a method comprising applying a coating solution (a solution or a suspension), which is a composition containing water or a buffer solution containing the complex, to a region in which the aggregate body 30 is to be formed, thus forming a film and burning the formed film. Examples of the buffer solution used to obtain the coating solution containing the complex may include an acetate buffer solution, a citrate buffer solution, and a glycine buffer solution.

The coating solution used to form a film may be prepared as a composition additionally containing the second target material and/or the precursor of the second target material that do not form the complex, in order to, for example, control the thickness T1 of the aggregate body 30. The coating solution used to form a film may further contain, as a component, a surfactant such as tween (registered trademark) 20, for the purpose of, for example, dispersing the complex in a more effective manner.

The burning of the complex may be performed by appropriately controlling temperature, time, atmosphere and the like. according to the region in which the aggregate body 30 is formed, the type of second target material and others.

The burning of the complex may be performed, for example, under the conditions of about 50°C to about 800°C in the air or an inert gas atmosphere for about 10 seconds to about 12 hours. The complex may be burned once at a single temperature or two or more times at different temperatures.

Titanium(IV) bis(ammonium lactato)dihydroxide, which is one of the precursors of the second target material described above, can become target titanium oxide composed mainly of anatase particles when, for example, burned at a temperature of about 450°C to about 600°C.

In this step, the multimer of the fusion protein is consumed, and a plurality of first pores 32 unevenly distributed near the first target material 20 and a plurality of second pores 34 are thereby formed. In other words, the plurality of first pores 32 (and the metal particles 36) are arranged so as to surround the first target material 20 such that the distances S1 from the plurality of first pores 32 to the surface of the first target material 20 (see FIGs. 1B to 1E) are substantially identical.

The size and shape of the second pores 34 can be controlled by, for example, appropriately changing burning temperature, burning time, and the particle diameter of the supplied second target material and/or the precursor of the second target material. The size of the second pores 34 can be reduced by, for example, adding a surfactant as a template or performing hydrothermal treatment.

When the porous structure body 10 configured to comprise the metal particles 36 in the first pores 32 as described with reference to FIGs. 1C and 1E are produced, heat treatment may be performed at a temperature equal to or higher than the melting point of the selected metal particles 36 to melt the metal particles 36. In this case, metal thin films 36a that can cover part of or the entire wall surfaces defining the first pores 32 in the aggregate body 30 may be formed.

When silver particles, for example, are used to form the metal thin films 36a, the heat treatment for the formation of the metal thin films 36a may be performed at a temperature of about 150°C to about 300°C.

The porous structure body 10 having the third pore 38 instead of the first target material 20 and described above with reference to FIGS. 1D and 1E may be produced by consuming the first target material 20 in the burning step. For example, when the first target material 20 is CNT and the second target material is titanium oxide, the CNT can be consumed by performing the burning step at about 600°C to about 700°C, whereby an aggregate body 30 having the third pore 38 and comprising particles of the second target material mainly of the anatase type can be formed.

The porous structure body 10 formed through the above steps may be stored alone after dissolved or suspended in a medium such as ethanol and may be used for a subsequent step.

The porous structure body 10 is produced through the above steps. When the metal particles 36 are encapsulated in the multimer of the fusion protein, the porous structure body 10 produced further comprises the metal particles 36 in the first pores 32.

Next, the configuration of a dye-sensitized solar cell and a method for producing the same will be described as examples of an electronic device having a functional structure using the above-described porous structure body 10 as a functional material and a method for producing the electronic device.

### 4. Configuration of dye-sensitized solar cell

### (1) Embodiment 1 of dye-sensitized solar cell

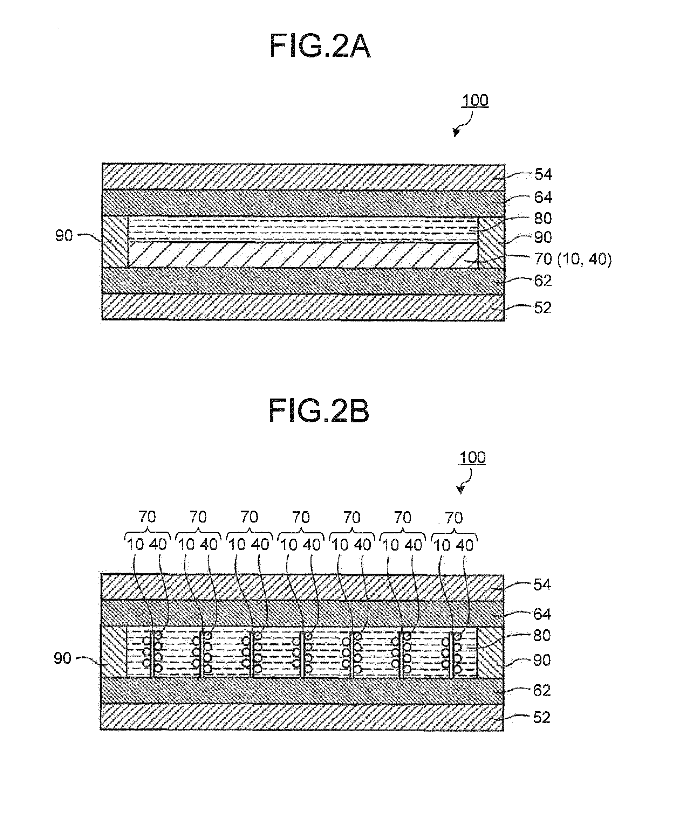

Referring to FIG. 2A, embodiment 1 of a dye-sensitized solar cell will be described. FIG. 2A is a schematic illustration showing a cut-end face of the dye-sensitized solar cell.

As shown in FIG. 2A, the dye-sensitized solar cell 100 according to embodiment 1 comprises a first substrate 52, a transparent electrode 62 provided on the first substrate 52, and a photoelectric conversion layer 70 provided on the transparent electrode 62. The photoelectric conversion layer 70 is combined with the transparent electrode 62, thus forming a photoelectrode.

The photoelectric conversion layer 70 comprises the above-described porous structure body 10 as a functional material. The porous structure body 10 used in embodiment 1 is preferably a porous structure body 10 in which CNT, for example, is used as the first target material 20 and titanium oxide, for example, is used as the second target material, namely, the material of the aggregate body 30. Many pieces of the sensitizing dye 40 are supported on the surface of the porous structure body 10.

The dye-sensitized solar cell 100 further comprises a second substrate 54 and a counter electrode 64 provided on the second substrate 54. The counter electrode 64 is provided so as to face the photoelectric conversion layer 70, in other words, the photoelectrode.

The photoelectric conversion layer 70 and the counter electrode 64 are provided so as to be spaced apart from each other, and the gap between the photoelectric conversion layer 70 and the counter electrode 64 is filled with an electrolyte 80. The electrolyte 80 is sealed by a sealing portion 90 provided in contact with the transparent electrode 62 and the counter electrode 64 so as to surround the gap, in other words, the electrolyte. More specifically, the gap is defined by the first substrate 52 on which the photoelectric conversion layer 70 is provided, the second substrate 54 on which the counter electrode 64 is provided, and the sealing portion 90.

No particular limitation is imposed on the first substrate 52 and the second substrate 54, so long as they can support the entire dye-sensitized solar cell 100. For example, any single one of glass, heat resistant polymer films such as polyimide, PET, PEN, PES, and Teflon (registered trademark), metals such as stainless steel (SUS) and an aluminum plate, and ceramics or a stacked structure thereof may be used for the first substrate 52 and the second substrate 54. Since the first substrate 52 is provided on a light incident side, the first substrate 52 is preferably transparent or semi-transparent and more preferably has high transparency.

Examples of the material of the transparent electrode 62 may include indium tin oxide (ITO), zinc tin oxide (ZTO), fluorine-doped tin oxide (FTO), SnO₂, In₂O₃, and ZnO.

The material itself of the transparent electrode 62 may not be transparent. For example, the transparent electrode 62 may be formed as a porous layer using a non-transparent material such that the transparent electrode 62 as a whole has a translucent structure.

In the dye-sensitized solar cell 100, a so-called BCE (back contact electrode) structure described in Chem. Mat. 20[15] (2008) 4974-4979 may be used. The BCE structure comprises, instead of the transparent electrode 62, an electrode that is formed of a non-transparent material so as to be spaced apart from the substrate on the light incident side.

The transparent electrode 62 may be formed as a single layer formed of any of the above materials or a stacked layer comprising a plurality of layers.

A first substrate 52 having a transparent electrode 62 formed in advance and acquired from the market may be used.

No particular limitation is imposed on the thickness of the photoelectric conversion layer 70. To further improve light transmittance, conversion efficiency and others, the thickness of the photoelectric conversion layer 70 is preferably, for example, about 0.5 µm to about 20 µm.

Preferably, the sensitizing dye 40 has an absorption wavelength in the visible wavelength range and the infrared range. To allow firm adsorption on the porous structure body 10, the sensitizing dye 40 comprises, in its molecule, preferably an interlocking group such as a carboxylic acid group, a carboxylic anhydride group, an alkoxy group, a hydroxyl group, a hydroxy alkyl group, a sulfonic acid group, an ester group, a mercapto group, or a phosphonyl group and more preferably a carboxylic acid group and a carboxylic anhydride group. The interlocking group is a group providing an electric bond that facilitates migration of electrons between the excited sensitizing dye 40 and the porous structure body 10.

Examples of the sensitizing dye 40 may include ruthenium bipyridine dyes, azo dyes, quinone dyes, quinonimine dyes, quinacridone dyes, squarylium dyes, cyanine dyes, merocyanine dyes, triphenylmethane dyes, xanthene dyes, porphyrin dyes, phthalocyanine dyes, perylene dyes, indigo dyes, and naphthalocyanine dyes.

The counter electrode 64 may be formed using the same material as that for the transparent electrode 62 described above. Specific examples of the material of the counter electrode 64 may include metals (e.g., platinum, gold, silver, copper, aluminum, rhodium, and indium) and conductive metal oxides (e.g., ITO and SnO₂). The thickness of the counter electrode 64 is preferably about 3 nm to about 10 µm. Particularly, when the material is a metal, the thickness is preferably about 5 µm or less and more preferably about 3 µm or less.

The electrolyte 80 used may be, for example, a liquid obtained by dissolving lithium iodide and iodine in a mixed solvent of acetonitrile and ethylene carbonate (volume ratio = 1:4).

The dye-sensitized solar cell 100 may be configured as either a super-straight type dye-sensitized solar cell or a sub-straight type dye-sensitized solar cell.

### (2) Embodiment 2 of dye-sensitized solar cell

Referring to FIG. 2B, embodiment 2 of the dye-sensitized solar cell will be described. FIG. 2B is a schematic illustration showing a cut-end face of the dye-sensitized solar cell. The same components as those in embodiment 1 of the dye-sensitized solar cell will be denoted by the same numerals, and their detailed description will be omitted.

As shown in FIG. 2B, the dye-sensitized solar cell 100 according to embodiment 2 comprises a first substrate 52, a transparent electrode 62 provided on the first substrate 52, and a photoelectric conversion layer 70 provided on the transparent electrode 62. The photoelectric conversion layer 70 is combined with the transparent electrode 62, thus forming a photoelectrode.

The photoelectric conversion layer 70 comprises the above-described porous structure body 10 as a functional material. The porous structure body 10 used in embodiment 2 is preferably a porous structure body 10 in which CNT, for example, is used as the first target material 20 and titanium oxide, for example, is used as the second target material, namely, the material of the aggregate body 30.

The dye-sensitized solar cell 100 further comprises a second substrate 54 and a counter electrode 64 provided on the second substrate 54. The counter electrode 64 is provided so as to face the photoelectric conversion layer 70.

In embodiment 2, a plurality of porous structure body 10 are arranged with their one ends in the elongation direction fixed to the transparent electrode 62. The plurality of porous structure body 10 extend substantially parallel to each other toward the counter electrode 64 such that their elongation direction is substantially parallel to the thickness direction of the first substrate 52.

No particular limitation is imposed on the embodiment of arrangement of the plurality of porous structure body 10. The plurality of porous structure body 10 may be arranged, for example, in a matrix form such that the plurality of porous structure body 10 are spaced at regular intervals. Many pieces of the sensitizing dye 40 are supported on the surface (pores) of each of the porous structure body 10.

The photoelectric conversion layer 70 and the counter electrode 64 are provided so as to be spaced apart from each other. The gap between the photoelectric conversion layer 70 and the counter electrode 64 is filled with an electrolyte 80. The gap filled with the electrolyte 80 is sealed by a sealing portion 90 that is provided in contact with the transparent electrode 62 and the counter electrode 64 so as to surround the gap.

### 5. Method for producing dye-sensitized solar cell

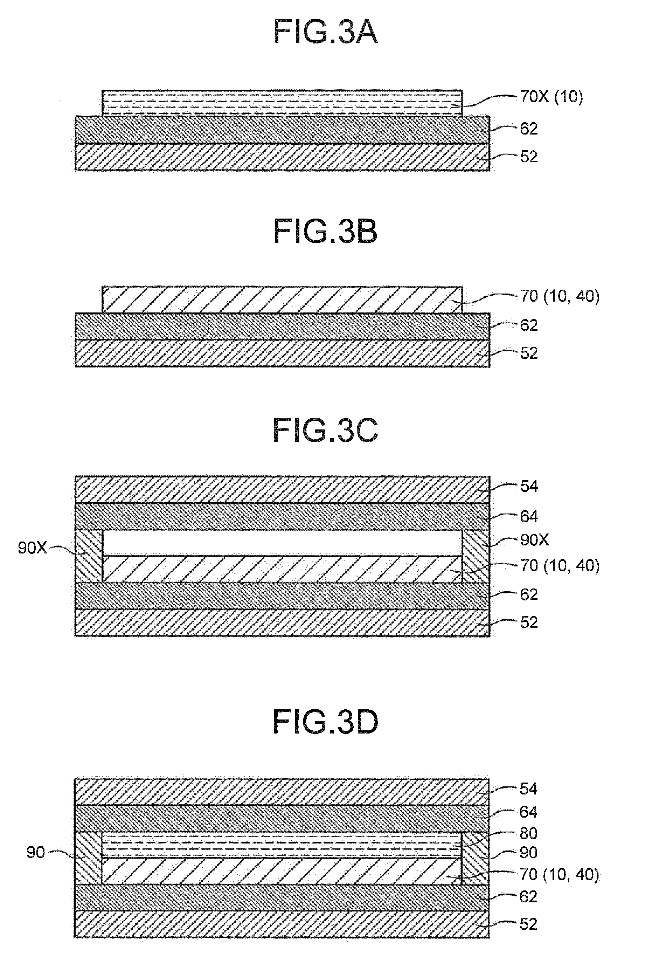

An example of the method for producing a dye-sensitized solar cell will be described with reference to FIGs. 3A, 3B, 3C, and 3D. FIGs. 3A, 3B, 3C, and 3D are schematic illustrations showing a step of producing the dye-sensitized solar cell.

### 5.1. Example 1 of method for producing dye-sensitized solar cell

Example 1 of the method for producing the dye-sensitized solar cell 100 comprises the steps of: preparing a substrate comprising a transparent electrode 62; obtaining a complex by binding the multimer of the fusion protein to the first target material 20, wherein the multimer of the fusion protein has an internal cavity and is formed from the fusion protein that contains a first peptide portion capable of binding to the first target material 20, a second peptide portion capable of binding to the second target material, and a polypeptide portion capable of forming a multimer having an internal cavity, thus forming a combination body and binding the combination body to the second target material or the precursor of the second target material; placing a material comprising the complex on the transparent electrode 62; burning the material on the transparent electrode, thus consuming the multimer of the fusion protein, whereby a structure comprising a porous structure body 10 is formed on the transparent electrode 62, the porous structure body 10 comprising an aggregate body 30 that comprises the second target material, is located so as to surround the first target material 20, and adheres to the first target material 20, the aggregate body 30 having a plurality of first pores 32 unevenly distributed near the first target material 20 and a plurality of second pores 34 scattered over the aggregate body 30; supporting a sensitizing dye on the porous structure body, thus forming a photoelectrode; and pouring an electrolyte and sealing the substrate having the photoelectrode and a counter electrode 64.

### (1) Step of preparing substrate comprising transparent electrode

First, the first substrate 52 comprising the transparent electrode 62 is prepared. In this step, a substrate having a transparent electrode 62 formed thereon in advance may be acquired from the market, as described above. As described above, a transparent electrode 62 may be deposited and formed on a first substrate 52 available from the market by sputtering, vacuum deposition and the like.

The prepared first substrate 52 comprising the transparent electrode 62 may be subjected to any suitable treatment step such as heat treatment or other surface treatment. For example, it is preferable to perform a step comprising immersing the first substrate 52 comprising the transparent electrode 62 in a 40 aqueous titanium tetrachloride solution heated to 70°C to 80°C for about 30 minutes, washing the resultant first substrate 52 with water or ethanol, and drying the first substrate 52. By performing this step, the photoelectric conversion efficiency can be further improved.

### (2) Step of placing material comprising complex on transparent electrode

Next, the first target material 20 and the multimer of the fusion protein having an internal cavity and prepared by the step described above are prepared. The multimer is composed of the fusion protein containing the first peptide portion capable of binding to the first target material 20, the second peptide portion capable of binding to the second target material, and the polypeptide portion capable of forming a multimer having an internal cavity.

The multimer of the fusion protein is bound to the first target material (e.g., CNT) to form a combination body. The combination body is mixed and bound to the second target material or the precursor of the second target material (e.g., titanium(IV) bis(ammonium lactato)dihydroxide, which is a precursor of titanium oxide) to prepare a complex comprising the second target material or the precursor of the second target material, the multimer of the fusion protein, and the first target material (e.g., CNT). Then, for example, a paste-like material comprising the complex is placed on the transparent electrode 62. For example, a paste-like material mixed with the complex, the second target material and/or the precursor of the second target material, and a dispersion medium added as needed is applied to the first substrate 52 by any suitable step such as a well-known coating method to form a film.

The final concentration of the complex comprising the second target material or the precursor of the second target material, the multimer of the fusion protein, and the first target material is preferably less than 1% by weight. The final concentration of the complex may be more preferably in the range of 0.06% by weight to 0.5% by weight, still more preferably in the range of 0.1% by weight to 0.3% by weight, yet more preferably in the range of 0.15% by weight to 0.25% by weight, and particularly preferably in the range of 0.15% by weight to 0.2% by weight.

The "final concentration of the complex" means the concentration of the complex comprising the second target material or the precursor of the second target material, the multimer of the fusion protein, and the first target material and the complex comprising the second target material or the precursor of the second target material, the multimer of the fusion protein, and the first target material only in the second target material or the precursor of the second target material in the paste-like material. More specifically, the "final concentration of the complex" means the concentration of the complex in solids remaining after the solvent is removed from the paste-like material, in other words, the ratio of the complex comprised in the aggregate body obtained in the burning step with the assumption that the complex is not consumed in the burning step.

When the final concentration of the complex falls within the above range, the electric characteristics of the formed dye-sensitized solar cell can be further improved, and the photoelectric conversion efficiency can thereby be further improved.

### (3) Step of forming structure comprising porous structure body on transparent electrode

As in the step of producing the porous structure body 10 described above, a material layer deposited on, for example, the transparent electrode 62 is burned to consume the multimer of the fusion protein. When a precursor of the second target material is used, burning can convert the precursor of the second target material to the second target material. For example, when applied titanium(IV) bis(ammonium lactato)dihydroxide, which is a precursor of titanium oxide, is burned, titanium oxide is obtained. Alternatively, titanium butoxide may be used as the precursor of the second target material. In this case, titanium oxide serving as the second target material can be obtained by burning titanium butoxide. Moreover, zinc hydroxide or zinc sulfate may be used as the precursor of the second target material. In this case, zinc oxide serving as the second target material can be similarly obtained by burning zinc hydroxide or zinc sulfate.

In this step, the porous structure body 10 having the aggregate body 30 located so as to surround the first target material 20 and adhering to the first target material 20, in other words, a layer structure comprising the porous structure body 10 (a photoelectric conversion layer), can be formed on the transparent electrode 62.

A modification of the above steps (2) and (3) will be described. This modification is an example in which a material comprising a pre-formed porous structure body 10 is supplied to a first substrate 52 comprising a transparent electrode 62, thus forming a structure comprising the porous structure body 10.

First, for example, an isolated porous structure body 10 formed as described above is prepared. Then a material comprising the porous structure body 10, the second target material and/or a precursor of the second target material is placed on the transparent electrode 62. For example, this step may comprise the step of applying a mixture of the porous structure body 10, the second target material and/or the precursor of the second target material, and a medium such as ethanol to the transparent electrode 62, thus forming a film. This step of forming a film may comprise the step of mixing the porous structure body 10, the second target material and/or the precursor of the second target material, and a paste comprising a component such as a dispersion medium and applying the obtained mixture, thus forming the film.

Then the formed layer structure comprising the porous structure body 10 is subjected to heat treatment. This heat treatment step may comprise a drying step performed at a temperature of about 120°C for about 5 minutes.

The heat treatment step may further comprise a burning step performed after the drying step. For example, when a precursor of the second target material is used, the burning step may be the step of converting the precursor of the second target material to the second target material. The burning step may be performed, for example, as the step of burning at a temperature of about 500°C for about 30 minutes.

Through the above steps, the structure comprising the porous structure body 10 (e.g., a layer structure such as a photoelectric conversion layer) can be formed on the transparent electrode 62.

### (4) Step of supporting sensitizing dye on porous structure body to form photoelectrode

Next, as shown in FIG. 3A, the sensitizing dye 40 described above is adsorbed on the formed layer 70X comprising the porous structure body 10. This step can be performed, for example, as the step of immersing the layer 70X comprising the porous structure body 10 in a liquid comprising the sensitizing dye 40 dissolved or dispersed therein or applying this liquid to the layer comprising the porous structure body 10.

No particular limitation is imposed on the solvent in which the sensitizing dye 40 is dissolved or dispersed. Examples of the solvent may include ethanol, acetone, diethyl ether, tetrahydrofuran, acetonitrile, chloroform, hexane, benzene, and ethyl acetate.

No particular limitation is imposed on the temperatures and pressures of the liquid and atmosphere when the layer 70X comprising the porous structure body 10 is immersed in the liquid comprising the sensitizing dye 40 dissolved or dispersed therein. The sensitizing dye 40 may be adsorbed, for example, at about room temperature and atmospheric pressure. The time of immersion may be appropriately controlled according to the type of the sensitizing dye 40 used, the type of solvent used, the concentration of the solution and others.

In this step, a photoelectric conversion layer 70 in which the sensitizing dye 40 is adsorbed on the porous structure body 10 can be obtained as shown in FIG. 3B. The photoelectrode comprising the transparent electrode 62 and the photoelectric conversion layer 70 integrally joined to each other is thereby formed on the first substrate 52.

### (5) Step of pouring electrolyte and sealing photoelectrode and counter electrode

Next, a second substrate 54 having a counter electrode 64 formed thereon is prepared. The counter electrode 64 and the photoelectric conversion layer 70 are provided and stacked so as to face each other with a spacing therebetween.

Next, the electrolyte 80 is poured into the gap between the photoelectric conversion layer 70 and the counter electrode 64 to fill the gap with the electrolyte 80.

As shown in FIG. 3C, first, a sealing material 90X is provided so as to form the gap between the photoelectric conversion layer 70 and the counter electrode 64. The sealing material 90X is provided in contact with the transparent electrode 62 and the counter electrode 64 so as to surround the gap with, for example, an injection port being present so that the electrolyte 80 can be injected into the gap. The sealing material 90X may be provided on the photoelectric conversion layer 70 in advance or may be provided on the counter electrode 64 in advance and bonded to the photoelectric conversion layer 70 with the counter electrode 64 facing the photoelectric conversion layer 70. The sealing material 90X used may be any known plastic adhesive comprising an ultraviolet curable resin, a thermosetting resin and the like.

Next, as shown in FIG. 3D, the electrolyte 80 is injected into the gap formed by the photoelectric conversion layer 70 (the first substrate 52), the counter electrode 64 (the second substrate 54), and the sealing material 90X to fill the gap with the electrolyte 80. Then the injection port used to inject the electrolyte 80 is sealed with, for example, the sealing material 90X. The sealing material 90X is subjected to any suitable treatment such as ultraviolet irradiation treatment or heat treatment suitable for the selected material to form the sealing material 90X into the sealing portion 90. The region (gap) between the photoelectric conversion layer 70 and the counter electrode 64 is thereby filled with the electrolyte 80 and sealed with the sealing portion 90. In other words, the photoelectric conversion layer 70 and the counter electrode 64 are sealed by the sealing portion 90 in the region filled with the electrolyte 80.

Through the above steps, a dye-sensitized solar cell is produced.

### 5.2. Example 2 of method for producing dye-sensitized solar cell

Example 2 of the production method is a method for producing a dye-sensitized solar cell having the configuration described with reference to FIG. 2B.

Example 2 of the method for producing the dye-sensitized solar cell 100 comprises the steps of: preparing a substrate comprising a transparent electrode 62; arranging at least one CNT used as the first target material 20 on the transparent electrode 62 so as to extend in the thickness direction of the substrate; forming a complex by binding the multimer of the fusion protein to the CNT, whrein the multimer of the fusion protein has an internal cavity and is formed from the fusion protein that contains a first peptide portion capable of binding to the CNT, a second peptide portion capable of binding to the precursor of the second target material, and a polypeptide portion capable of forming a multimer having an internal cavity, thus forming a combination and then binding the combination to the second target material or the precursor of the second target material; burning the complex to consume the multimer of the fusion protein, whereby a porous structure body 10 is formed on the transparent electrode 62, the porous structure body 10 comprising an aggregate body 30 that comprises the second target material, is located so as to surround the CNT, and adheres to the CNT, the aggregate body 30 having a plurality of first pores 32 unevenly distributed near the CNT and a plurality of second pores 34 scattered over the aggregate body 30; supporting the sensitizing dye 40 by the porous structure body 10, thus forming a photoelectrode; and pouring the electrolyte 80 and sealing the photoelectrode and a counter electrode 64.

Example 2 of the production method is different from the example 1 of the production method only in terms of the step (2) of placing the material comprising the complex on the transparent electrode. In Example 2 of the production method, steps (2'-1) and (2'-2) are performed instead of step (2) in example 1 of the production method.

Only steps (2'-1) and (2'-2) performed in example 2 of the production method will be described, and detailed description of the same steps as those in example 1 of the production method will be omitted.

### (2'-1) Step of arranging at least one CNT on transparent electrode so as to extend in thickness direction of substrate

This step may be performed, for example, according to a method of producing CNTs disclosed in JP 2001-181842 A.

This step may be performed, for example, as (i) the step of causing a suspension of CNTs to flow along a ceramic filter to arrange the CNTs on the surface of the filter and transferring the arranged CNTs onto the transparent electrode 62, (ii) the step of causing an inorganic material to be held in a plurality of proteins having internal cavities, arranging the plurality of proteins holding the inorganic material on the transparent electrode 62, removing the proteins, and growing CNTs using the inorganic material remaining on the transparent electrode 62 as a seed, or (iii) the step of adsorbing an inorganic material on a substrate, growing carbon nanotubes using the inorganic material as a seed to obtain a carbon nanotube-arranged substrate, and transferring the carbon nanotubes on the carbon nanotube-arranged substrate to the transparent electrode 62.

As the step of forming CNTs on the transparent electrode, the above step (iii) will be described. First, a protein that can hold in its internal cavities an inorganic material (e.g., an iron nanoparticle) used as a seed after the protein forms a protein multimer is prepared. For example, ferritin protein fused with a titanium-binding peptide (minTBP-1) (TBF) can be used as such a protein.

Next, the inorganic material is held in the protein multimer (inorganic material-encapsulating protein). In one specific example, iron oxide nanoparticles are encapsulated in the internal cavities of the TBF multimer.

Then the inorganic material used as the seed is provided in a region in which CNTs are to be provided. In other words, the inorganic material is adsorbed on the substrate.

Preferably, this step is performed as a step in which a substrate having a silicon oxide film formed thereon is used and the inorganic material-encapsulating protein is arranged and adsorbed on the silicon oxide film. In this step, for example, the protein multimer encapsulating the inorganic material in its internal cavities is first adsorbed on the silicon oxide film in the substrate having the silicon oxide film formed thereon. In one specific example, a solution (buffer solution) of the TBF multimer encapsulating iron oxide nanoparticles is placed on the silicon oxide film to adsorb the nanoparticles thereon.

Next, only the inorganic material is allowed to remain on the silicon oxide film. More specifically, for example, UV-O₃ treatment is performed to remove only the protein component. A substrate in which only the inorganic material is adsorbed on the surface of the silicon oxide film can thereby be obtained. This step may be performed as needed. The substrate on which the TBF multimer encapsulating iron oxide nanoparticles is adsorbed may be used for the step of growing CNTs described later without removing only the protein component from the substrate.

Next, CNTs are grown (synthesized) on the silicon oxide film to form a CNT forest in which the CNTs stand close together on the surface of the silicon oxide film.

The CNT forest can be formed as follows. First, the substrate in which an inorganic material or a TBF multimers encapsulating the inorganic material is adsorbed on the surface of the silicon oxide film is subjected to heat treatment in a vacuum. The heat treatment may be performed in the temperature range of, for example, 500°C to 800°C and preferably in the temperature range of 600°C to 700°C.

Next, the heated substrate is left (allowed) to stand in a hydrogen gas (H₂) atmosphere. This step may be performed at a hydrogen flow rate in the range of preferably 10 seem to 200 seem and a reaction pressure in the range of, for example, 60 Pa to 1.5 kPa (1,500 Pa) and preferably 200 Pa to 1.5 kPa. In this step, the inorganic material adsorbed on the silicon oxide film is activated.

Next, the substrate having the silicon oxide film with the activated inorganic material adsorbed thereon is left (allowed) to stand in, for example, a mixed atmosphere of acetylene gas (C₂H₂) / hydrogen gas for a prescribed time to grow CNTs on the silicon oxide film. A CNT forest is thereby formed, and a carbon nanotube-arranged substrate is obtained.

This step may be performed at a hydrogen flow rate in the range of preferably 10 seem to 200 seem, an acetylene flow rate in the range of, for example, 10 seem to 200 seem and preferably 10 seem to 50 seem, a reaction pressure in the range of, for example, 60 Pa to 1.5 kPa and preferably 200 Pa to 1.5 kPa, an acetylene partial pressure in the range of, for example, 19 Pa to 400 Pa and preferably 19 Pa to 350 Pa, and a reaction temperature in the range of, for example, 500°C to 800°C and preferably 600°C to 700°C.

When the step (ii) described above is performed to form a CNT forest, for example, the step until the step of adsorbing the inorganic material on the transparent electrode 62 is performed under the same conditions as those in the above step (iii). Then the substrate having the transparent electrode 62 in which the inorganic material or the TBF multimer encapsulating the inorganic material is adsorbed on the surface of the transparent electrode 62 is heated in the range of 500°C to 600°C, whereby the CNT forest can be formed.

Next, the carbon nanotubes in the formed carbon nanotube-arranged substrate are transferred to the transparent electrode 62. This step may be performed, for example, using a method described in Sci. Rep. 2:368 doi:10.1038/srep00368 (2012), namely, a dry transfer method.

More specifically, first, the carbon nanotube-arranged substrate is subjected to H₂O etching to weaken the chemical bonding force between the inorganic material (catalyst particles) and synthesized carbon nanotubes on the carbon nanotube-arranged substrate. Next, the CNT forest on the carbon nanotube-arranged substrate is provided so as to face the transparent electrode 62. Then the carbon nanotube-arranged substrate and the substrate having the transparent electrode 62 formed thereon are laid on top of the other such that the CNT forest comes into contact with the transparent electrode 62. Next, the carbon nanotube-arranged substrate is pressed from its surface opposite to the side on which the CNT forest is formed to press the carbon nanotube-arranged substrate against the transparent electrode 62. In the above step, the CNT forest can be transferred to the transparent electrode 62.

Preferably, the above-described step of adsorbing the inorganic material on the substrate is a step in which at least two types of inorganic material-encapsulating proteins with different sizes are arranged and adsorbed on the substrate. The "size" means the diameter of the inner cavity comprised in an inorganic material-encapsulating protein.

The diameter of the inner cavity of an inorganic material-encapsulating protein is generally within the range of about 4 nm to about 20 nm. Therefore, it is preferable to combine at least two types of inorganic material-encapsulating proteins having different sizes within the above range. No particular limitation is imposed on the difference in size, so long as the sizes of at least two types of inorganic material-encapsulating proteins are mutually different. The combination of at least two types of inorganic material-encapsulating proteins is preferably a combination in which the size of at least one type of inorganic material-encapsulating protein is about twice the size of another at least one type of inorganic material-encapsulating protein. Preferred examples of the combination of at least two types of inorganic material-encapsulating proteins may include a combination of a TBF multimer and a CDT multimer.

Preferably, to synthesize a CNT forest comprising single-walled CNTs (SWNTs), at least two types of inorganic material-encapsulating proteins with different sizes are used, and a substrate with these inorganic material-encapsulating proteins adsorbed on the surface of a silicon oxide film is subjected to heat treatment in a vacuum.

The heat treatment may be performed in the temperature range of, for example, 500°C to 800°C and preferably 600°C to 700°C.

For example, when two types of inorganic material-encapsulating proteins with different sizes are used, the ratio of the amounts of the two types of protein multimers with different sizes (e.g., a TBF multimer and a CDT multimer) that are adsorbed on the surface of the silicon oxide film (the amount of the protein with a smaller size / the amount of the protein with a larger size) is within the range of preferably 0.5 to 2.0.

Next, the heated silicon oxide film is left (allowed) to stand in a hydrogen gas (H₂) atmosphere. This step may be performed at a hydrogen flow rate in the range of preferably 10 seem to 200 seem and a reaction pressure in the range of, for example, 60 Pa to 1.5 kPa (1,500 Pa) and preferably 200 Pa to 1.5 kPa. In this step, the inorganic material adsorbed on the silicon oxide film is activated.

Next, the substrate having the silicon oxide film with the activated inorganic material adsorbed thereon is left (allowed) to stand in, for example, a mixed atmosphere of acetylene gas (C₂H₂) / hydrogen gas for a prescribed time to grow CNTs on the silicon oxide film, whereby a CNT forest is formed.

This step may be performed at a hydrogen flow rate in the range of preferably 10 seem to 200 seem, an acetylene flow rate in the range of, for example, 10 seem to 200 seem and preferably 10 sccm to 50 seem, a reaction pressure in the range of, for example, 60 Pa to 1.5 kPa and preferably 200 Pa to 1.5 kPa, an acetylene partial pressure in the range of, for example, 19 Pa to 400 Pa and preferably 19 Pa to 350 Pa, and a reaction temperature in the range of, for example, 500°C to 800°C and preferably 600°C to 700°C.

In the above step, when only a TBF multimer, for example, is used, the diameter of the formed CNTs becomes larger, and Multi-Walled CNTs to which a protein is less likely to adhere in a subsequent step are mainly formed. When only a CDT multimer, for example, is used, the formed CNTs are Single-Walled CNTs, but the density of the CNTs on the substrate becomes low.

However, when at least two types of inorganic material-encapsulating proteins with different sizes, for example, a mixture of two types, a TBF multimer with a larger size and a CDT multimer with a smaller size, are used, a CNT forest having a higher density and comprising mainly SWNTs which have a small diameter and to which a protein can easily adhere in a subsequent step can be formed.

### (2'-2) Step of binding fusion protein multimer to CNTs to form combination and binding combination to second target material or precursor of second target material to obtain complex

This step may be performed as the following step. First, the multimer of the fusion protein is mixed with, for example, a buffer solution to prepare a liquid (paste-like) material. Then the liquid material is supplied to the transparent electrode 62 having the grown CNTs arranged thereon using, for example, a coating method. The multimer of the fusion protein is thereby bound to the CNTs to form a combination. Then the second target material or a precursor of the second target material is mixed with, for example, a dispersion medium to obtain a liquid material, and the liquid material is supplied using, for example, a coating method to bind the second target material or the precursor of the second target material to the multimer of the fusion protein bound to the CNTs, whereby a complex is formed. If necessary, the second target material or the precursor of the second target material is deposited around the CNTs.

Next, a film structure deposited in the same manner as in the above-described production step for the porous structure body 10 is dried and burned to consume the multimer of the fusion protein. In this step, a porous structure body 10 having the aggregate body 30 located so as to surround the first target material 20 and adhering to the first target material 20 can be formed.

Then the same steps as steps (4) and (5) described in example 1 of the production method are performed, whereby a dye-sensitized solar cell having the structure described above with reference to FIG. 2B is produced.

### Examples

The present invention will be described with reference to the following Examples, but the present invention is not limited by these Examples.

### Example 1: Production of strain for expressing fusion protein CNHBP-Dps-TBP (CDT)

The metal-encapsulating protein Dps from Listeria innocua, an N-terminus of which is fused with a carbon nanohorn-binding protein (abbreviated as CNHBP and consisting of the amino acid sequence DYFSSPYYEQLF (SEQ ID NO:6); see International Publication No. WO2006/068250) and a C-terminus of which is fused with a titanium oxide-binding protein (abbreviated as TBP and consisting of the amino acid sequence RKLPDA (SEQ ID NO:8); see International Publication No. WO2005/010031) was constructed (abbreviated as CNHBP-Dps-TBP or CDT, SEQ ID NOS:1 and 2) by the following procedure.

First, synthesized DNAs (SEQ ID NO:9 and SEQ ID NO:10) was burned by heating a mixed solution of the synthesized DNAs at 98°C for 30 seconds and rapidly cooling it to 4°C. This DNA solution and pET20 carrying a Dps gene from Listeria innocua (see, K. Iwahori et al., Chem. Lett., 2007, vol. 19, p.3105) were separately digested completely with a restriction enzyme NdeI.

The resulting DNA products were ligated with T4 DNA ligase (Takara Bio Inc., Japan) to obtain the plasmid pET20-CD carrying a gene encoding Dps, the N-terminus of which is fused with CNHBP (CNHBP-Dps, abbreviated as CD). Subsequently, PCR was carried out using pET20-CD as a template, and oligonucleotides consisting of the nucleotide sequences represented by SEQ ID NOS:11 and 12.

The resulting PCR product was purified using Wizard SV Gel and PCR Clean-Up System (Promega, USA), and digested with the restriction enzymes DpnI and BamHI. The PCR product digested with the restriction enzymes was self-ligated using the T4 DNA ligase (Promega, USA).

E. *coli* JM109 (Takara Bio Inc., Japan) was transformed with the self-ligated PCR product to construct JM 109 possessing the expression plasmid (pET20-CDT) carrying the gene encoding Dps (CDT), the N-terminus of which was fused with the carbon nanohorn-binding peptide and the C-terminus of which was fused with the titanium-binding peptide. The plasmid pET20-CDT was purified from the transformant using Wizard Plus Miniprep System (Promega, USA). Finally, BL21 (DE3) (Invitrogen, USA) was transformed with pET20-CDT to make a strain BL21 (DE3)/pET20-CDT for expressing the protein.

### Example 2: Preparation example of fusion protein CDT

BL21 (DE3)/pET20-CDT were cultured in 5 mL of LB medium (containing 100 mg/L of ampicillin) at 37°C. Eighteen hours after starting the cultivation, the cultured medium was inoculated to 3 L of new LB medium (containing 100 mg/L of ampicillin) and cultured with shaking using BMS-10/05 (ABLE, Japan) at 37°C for 24 hours. The resulting microbial cells were collected by centrifugation (5,000 rpm, 5 minutes), and stored at -80°C. A half (6 g) of the cryopreserved microbial cells was suspended in 40 mL of 50 mM Tris-HCl buffer solution (pH 8.0). Subsequently, the microbial cells were disrupted by giving an ultrasonic pulse (200 W, Duty 45%) to the suspension every one second for 12 minutes using Digital Sonifier 450 (Branson, USA). The solution was centrifuged at 15,000 rpm for 15 minutes (JA-20, Beckman Coulter, USA), and a supernatant fraction was collected. The collected solution was heated at 60°C for 20 minutes, and rapidly cooled on ice after the heating. The cooled solution was centrifuged (JA-20) at 17,000 rpm for 10 minutes, and a supernatant (about 20 mL) was collected again. This solution was sterilized using a disc filter (Millex GP, 0.22 µm, Millipore, USA). And, this solution was ultrafiltrated and concentrated using Amicon-Ultra-15 (NMWL. 50000, Millipore, USA) until a liquid amount became 10 mL to obtain a protein solution.

Subsequently, a CDT fraction that was an objective protein was purified from the resulting protein solution using gel filtration chromatography. Specifically, 10 mL of the protein solution was applied to HiPrep 26/60 Sephacryl S-300 High resolution column (GE healthcare, USA) equilibrated with Tris-HCl buffer solution (50 mM Tris-HCl solution containing 150 mM NaCl, pH 8.0), and separated/purified at a flow rate of 1.4 mL/minute to collect fractions corresponding to CDT.

Then the obtained protein solution was ultrafiltrated and concentrated, and the buffer solution in the protein solution was replaced with a 50mM Tris-HCl buffer solution (pH 8.0). 10 mL of the protein solution was injected into a HiLoard 26/10 Q-Sepharose High Performance column (GE healthcare, USA) equilibrated with a 50mM Tris-HCl buffer solution (pH 8.0). Separation/purification was performed at a flow rate of 4.0 mL/minute with a salt concentration gradient using a 50mM Tris-HCl buffer solution (pH 8.0) containing 0mM to 500mM NaCl. CDT fractions were thereby purified, and CDT was obtained. The following examples were carried out using the purified CDT.

### Example 3: Preparation example of CNT/CDT complex

To form a nano-complex of CNT and CDT, a potassium phosphate buffer solution containing CNT and CDT [50mM potassium phosphate (pH 6.0) containing CDT at a final concentration of 0.5 mg/mL and CNT (Sigma, 519308, carbon nanotube, single walled) at a final concentration of 0.3 mg/mL] was prepared. Ultrasonic pulses (200 W, Duty 20%) were applied to the potassium phosphate buffer solution containing CDT on ice using Digital Sonifier 450 (Branson, USA) for 1 second at three-second intervals until the total application time reached 5 minutes. The sonicated CDT-CNT mixed solution was centrifuged (15,000 rpm, 5 minutes), thus obtaining a CNT/CDT complex in which many CDTs were bound to CNT.

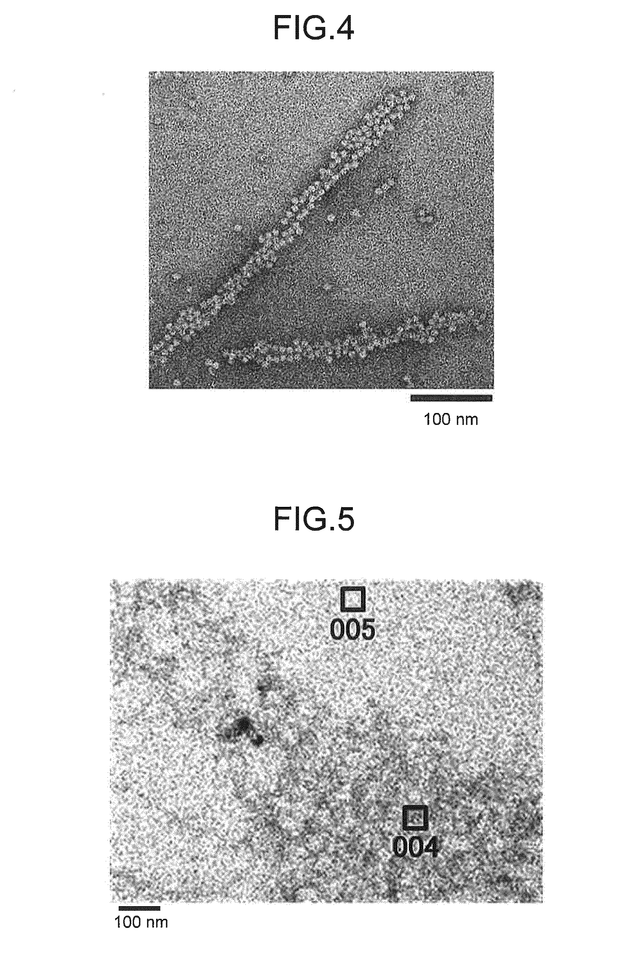

The result is shown in FIG. 4. FIG. 4 shows a transmission electron microscopic image of the complex of CNHBP-Dps-TBP (CDT) and CNT. The image was taken after the complex was stained with 3% phosphotungstic acid. As can be seen from the result, the CNHBP-Dps-TBP (CDT) and CNT form a complex.

### Example 4: Preparation example of CNT/CDT/Ti complex

A titanium precursor, Titanium(IV) bis(ammonium lactato)dihydroxide (SIGMA, 388165), was added to the obtained CNT/CDT complex solution at a final concentration of 2.5% by weight, and the mixture was left to stand at room temperature (24°C). A sample 30 minutes after the start of the reaction and a sample 15 hours after the start of the reaction were centrifuged (15,000 rpm, 5 minutes), and precipitates were collected. The obtained precipitates were washed three times with water and finally suspended in water. Each of the obtained unstained samples was subjected to electron microscopic analysis under a transmission electron microscope (JEM3100-FEF, 300 kV). The result of the electron microscopic analysis on the sample 15 hours after the start of the reaction is shown in FIG. 5. FIG. 5 shows a transmission electron microscopic image of a black precipitate obtained by adding the titanium precursor to the complex of CNHBP-Dps-TBP (CDT) and CNT.

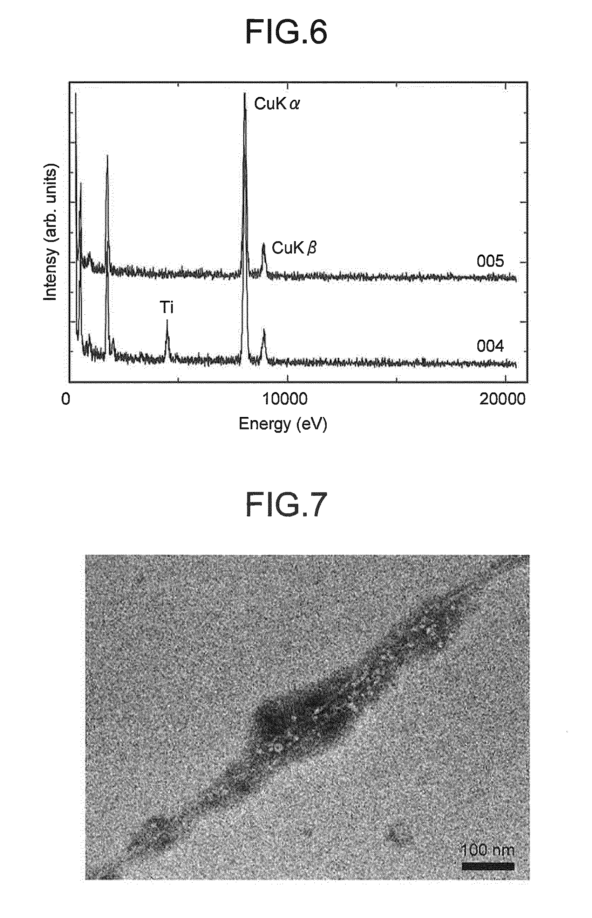

As can be seen from the result, the black structure was observed. The obtained sample (black structure) was subjected to composition analysis by energy dispersive X-ray spectrometry (EDS) using a transmission electron microscope (JEM3100-FEF, 300kV). The results are shown in FIG. 6. FIG. 6 is a diagram showing peaks obtained by the EDS analysis performed on regions surrounded by Box 004 and Box 005 in FIG. 5.

As can be seen from the results, the black structure in the region denoted by Box 004 in FIG. 5 contains titanium atoms. However, no peak for titanium atoms was observed in the region comprising no black structure and denoted by Box 005 in FIG. 5.

Peaks originating from copper were observed in both Box 004 and Box 005. This may be because a component of a copper-made grid on which a sample was placed was detected during electron microscopic observation.

Therefore, it was found that the gray region found in the observed CNT/CDT nano-complex solution with the titanium precursor added thereto contains titanium atoms.

Next, a structure comprised in the CNT/CDT nano-complex solution with the titanium precursor added thereto was stained with 3% phosphotungstic acid (PTA) and subjected to TEM analysis at a high magnification using a transmission electron microscope (JEM2200-FS, 200 kV). The result is shown in FIG. 7. FIG. 7 shows a transmission electron microscopic image of a black precipitate obtained by adding the titanium precursor to the complex of CNHBP-Dps-TBP (CDT) and CNT.

As can be seen from the result, a rod-shaped structure having a length of 1 µm in its elongation direction and a diameter in the range of 50 nm to 100 nm in a direction orthogonal to the elongation direction was found.

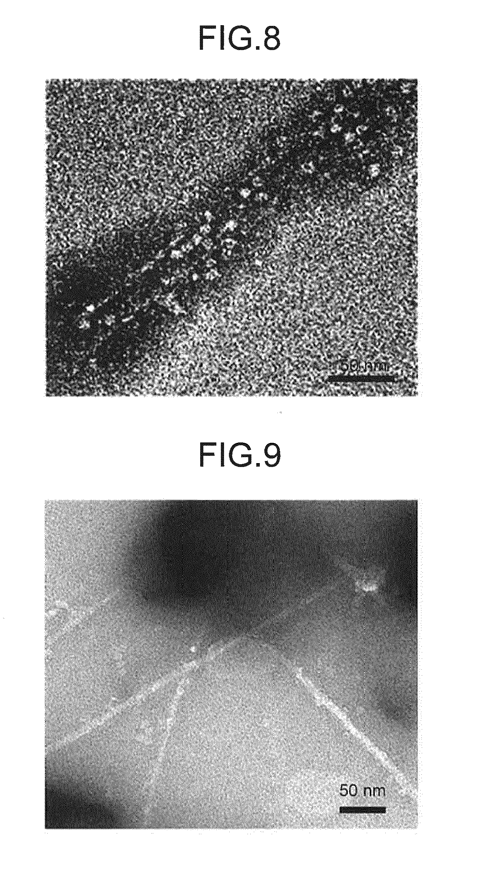

FIG. 8 shows a transmission electron microscopic image of a black precipitate obtained by adding the titanium precursor to the complex of CNHBP-Dps-TBP (CDT) and CNT. As shown in FIG. 8, in the obtained transmission electron microscopic image, a CNT having a diameter of about 5 nm was observed in a black region having a high electron density and containing titanium. In addition, spherical protein CDT with a diameter of about 9 nm was observed around the CNT. In this complex, the distance (S1) between CDT and CNT was 1 nm to 10 nm.

As a control, the titanium precursor was added to a solution containing only CNT, and the mixture was allowed to react in the same manner. More specifically, CNT (Sigma, 519308, carbon nanotube, single walled) was added at a final concentration of 3 mg/mg to a potassium phosphate buffer solution (pH 6.0) with a final concentration of 50 mM. The obtained solution was sonicated on ice using Digital Sonifier 450 (Branson, USA). More specifically, sonication (200 W, Duty 20%) was performed for one second, and the ultrasonic waves were stopped for 3 seconds. This cycle was repeated until the total sonication time reached 5 minutes. If centrifugation is performed after sonication, all the CNTs may be precipitated, and accordingly, centrifugation was not performed. The titanium precursor, titanium(IV) bis(ammonium lactato)dihydroxide, was added at a final concentration of 2.5% by weight to the sonicated solution containing only the CNTs, and the mixture was left to stand at room temperature (24°C). A sample 15 hours after the start of the reaction was centrifuged (15,000 rpm, 5 minutes), and a precipitate was collected. The precipitate was washed three times with water and finally suspended in water. The obtained suspension was stained with 3% PTA and subjected to electron microscopic analysis using a transmission electron microscope (JEM2200-FS, 200 kV). The result is shown in FIG. 9. FIG. 9 shows a transmission electron microscopic image of a black precipitate obtained by adding the titanium precursor to the solution containing CNTs dispersed therein.

As can be seen from the result, in the solution in which only CNTs were dispersed, no black region originating from titanium atoms specifically present around CNTs and observed in FIG. 7 was observed.

In other words, by utilizing the titanium-deposition activity of CDT bound to CNT, the CNT/CDT complex (nano-complex) can be coated with titanium, so that a CNT/CDT/Ti complex (nano-complex) can be synthesized.

### Example 5: Binding between metal particle-encapsulating fusion protein CDT and CNT

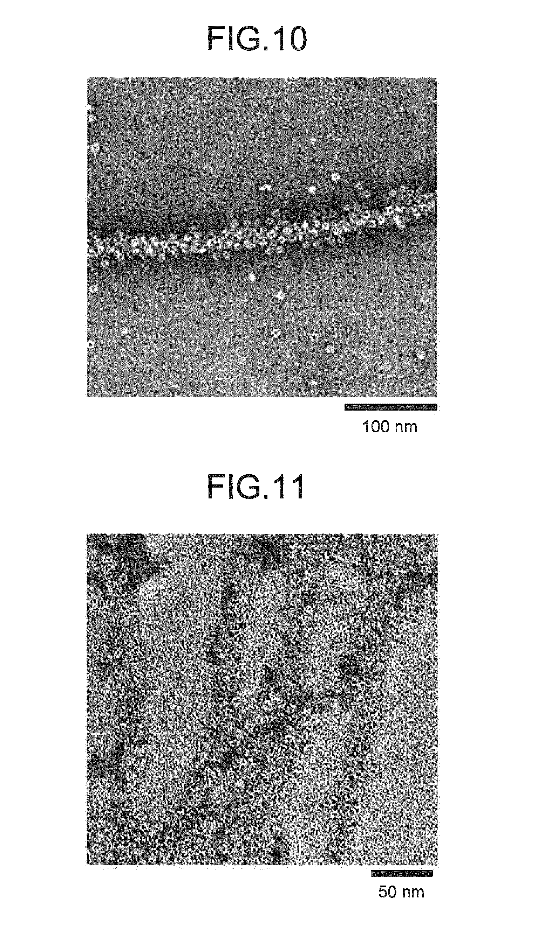

First, an iron oxide nanoparticle was formed in an internal cavity of a CDT multimer. Specifically, 1 mL of HEPES buffer solution containing CDT (80 mM HEPES/NaOH, pH 7.5 containing 0.5 mg/mL of CDT and 1 mM ammonium iron sulfate each at a final concentration) was prepared, and left stand at 4°C for 3 hours. Then, this buffer solution was centrifuged (15,000 rpm, 5 minutes), and a supernatant containing the protein was collected. This supernatant was ultrafiltrated and concentrated using icon-Ultra-15 (NMWL. 50000, Millipore, USA) and the buffer solution in the solution of the CDT multimer having the iron oxide nanoparticle in its internal cavity (Fe-CDT) was replaced with water to obtain a protein solution. A potassium phosphate buffer solution (50 mM potassium phosphate (pH 6.0) containing 0.3 mg/mL Fe-CDT and 0.3 mg/mL CNT each at a final concentration) containing CNT and the CDT multimer having the iron oxide nanoparticle in its internal cavity was prepared using this protein solution. An ultrasonic pulse treatment (200 W, duty 10%) for one second was given to the prepared solution every 3 seconds for total 5 minutes on ice using Digital Sonifier 450 (Branson, USA). The Fe-CDT-CNT mixed solution treated by the ultrasonic pulse was centrifuged (15,000 rpm, 5 minutes) to obtain a CNT/Fe-CDT complex in which many CDT multimers were bound to CNT. The result is shown in FIG. 10. FIG. 10 is a view showing a transmission electron microscopic image of the complex of CNHBP-Dps-TBP (CDT multimer) and CNT having the iron oxide nanoparticle in its internal cavity. The transmission electron microscopic image was obtained by photographing the sample stained with 3% TPA.

As a result, it could be confirmed that the complex (CNT/Fe-CDT complex) of CNT and CNHBP-Dps-TBP (CDT multimer) having the iron oxide nanoparticle in its internal cavity had been obtained.

### Example 6: Preparation example of CNT/Fe-CDT/Ti complex

The titanium precursor, titanium (IV) bis(ammonium lactato)dihydroxide (Sigma-Aldrich, 388165) was added to the obtained CNT/Fe-CDT complex solution such that the final concentration of the precursor became 2.5% by weight, and the mixture was left stand at room temperature (24°C). Samples were centrifuged (15,000 rpm, 5 minutes) 30 minutes and 15 hours after starting the reaction, and pellets were collected. The pellet was washed three times with water, and finally suspended in water to obtain an aqueous solution of CNT/Fe-CDT/Ti.

FIG. 11 is a view showing a transmission electron microscopic image of a black precipitate obtained by adding the titanium precursor to the complex of CNT and CNHBP-Dps-TBP (CDT multimer) having the iron oxide nanoparticle in its internal cavity. When the aqueous solution of CNT/Fe-CDT/Ti not stained was analyzed under TEM, the CDT multimers encapsulating the iron oxide nanoparticles in their internal cavities could be observed in a black rod-shaped structure as shown in FIG. 11. The distance (S1) between CDT and CNT in this complex was 1 nm.

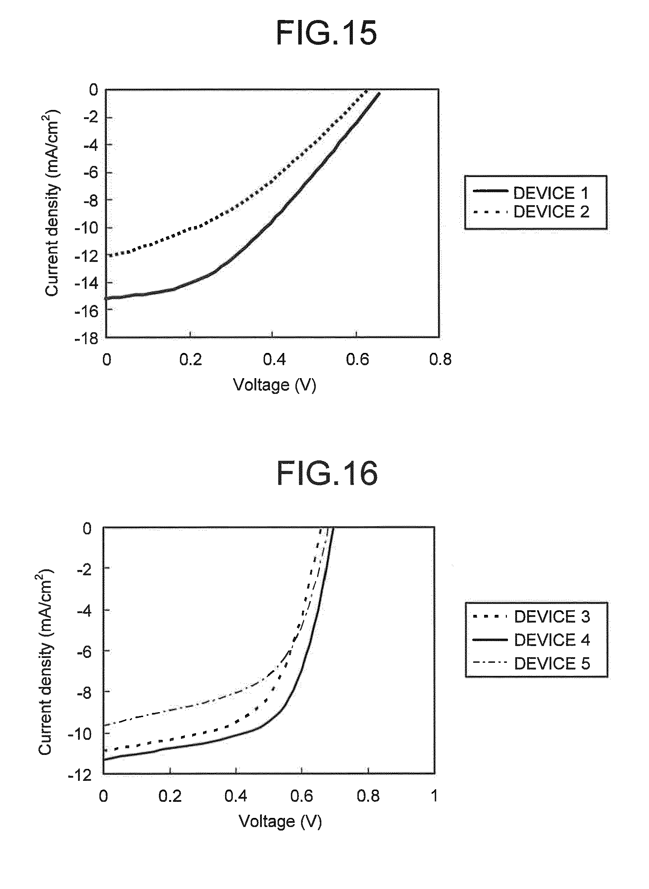

This black rod-shaped structure could be speculated to contain titanium from the result of EDS analysis. A surface area of the titanium nano-rod structure, which appeared to be increased by the CDT multimers, was analyzed from this TEM image. As a result, it could be speculated from the TEM image in FIG. 16 that 64 CDT multimers were encapsulated in the titanium nano-rod structure having a length of 102 nm in a lengthwise direction, and a diameter of 31 nm in a direction orthogonal to the lengthwise direction. The surface area of the titanium nano-rod structure is 1.1×10⁴ (nm²). The surface area of the CDT multimer having a diameter of 9 nm is 254 (nm²). A sum of the surface areas of 64 CDT multimers is 1.6×10⁴ (nm²). That is, the surface area per 100 nm of the length in the lengthwise direction of the titanium nano-rod structure encapsulating the CDT multimers observed in this case is 2.6×10⁴ (nm²), and could be estimated to be 2.4 times larger than 1.1×10⁴ (nm²) that was the surface area per 100 nm of the length in the lengthwise direction of the titanium nano-rod structure which did not encapsulate the CDT multimer.

Since the iron oxide nanoparticle encapsulated in the CDT multimer could be introduced into the titanium film, it is expected to introduce the metal nanoparticle of nickel, cobalt, manganese, phosphorus, uranium, beryllium, aluminium, cadmium sulfide, cadmium selenide, palladium, chromium, copper, silver, gadolium complex, platinum cobalt, silicon oxide, cobalt oxide, indium oxide, platinum, gold, gold sulfide, zinc selenide, and cadmium selenium, which are predicted to be able to encapsulated in the CDT multimer, into a titanium film and a titanium oxide film which coat CNT.

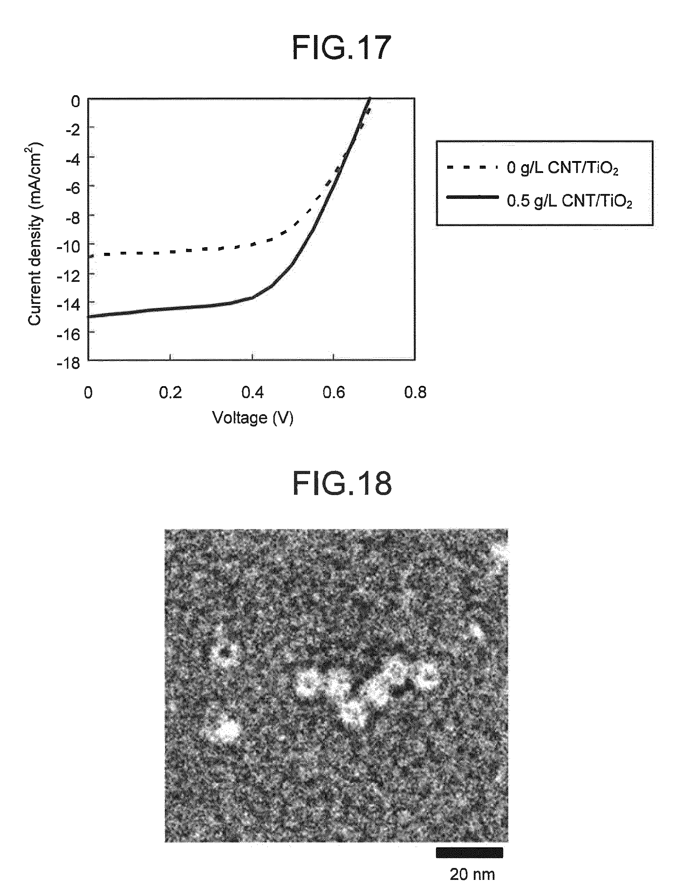

Subsequently, 10 µL of an aqueous solution of CNT/Fe-CDT/Ti was placed on a silicon substrate treated with UV/ozone (115°C, 5 minutes, 1 mL/minute) and coated with an SiO₂ film having a thickness of 10 nm, and treated with heat at 450°C for 30 minutes. Subsequently the complex was left stand at and cooled to room temperature, and analyzed under a scanning electron microscope (SEM). The result is shown in FIG. 17. FIG. 17 is a view showing a scanning electron microscopic image of a structure obtained by heating the complex of CNT and CNHBP-Dps-TBP (CDT multimer) coated with titanium oxide.

As a result, an appearance where a circumference of CNT observed as a fibrous shape was covered with particulate structures and film-shaped structures was observed. Also, from the analysis by EDS, it was suggested that the structures that coated CNT was composed of the titanium oxide.

### Example 7: Preparation example of CNT/TiO₂ complex

First, CDT at a final concentration of 0.3 mg/mL and CNT (carbon nanotube single walled, Sigma, 519308) at a final concentration of 0.3 mg/mL were added to 50 mM potassium phosphate buffer solution (pH 6.0). Subsequently, 40 mL of the obtained solution was sonicated (200 W, 25%) on ice using Digital Sonifier (Branson, USA) in cycles of sonicating the solution for one second and resting the sonication for three seconds, and the sonication treatment was continued for 5 minutes. A thick element having a diameter of 10 mm was used for the sonication. After the sonication, the solution was transferred to a 50 mL tube, and CNT that had not been bound to the CDT multimer was removed by centrifugation at 8,500 rpm for 10 minutes. Titanium (IV) bis(ammonium lactato)dihydroxide (Sigma, 388165) was added at a final concentration of 2.5% by weight to this solution, and the mixture was left stand at room temperature for 2 hours. In consequence, precipitation of an aggregate body was observed. Subsequently, the solution in a 50 mL centrifuge tube was centrifuged at 8,500 rpm for 10 minutes and the precipitate was collected to purify a CNT/CDT/Ti complex. The complex was further washed by adding 40 mL of water and centrifuging it. Finally, 0.8 mL of water was added and the solution was transferred to a 1.5 mL of microtube. Subsequently, 200 µL of the obtained CNT/CDT/Ti solution was placed on a quartz board and heated at temperature ranging from 450°C to 800°C (at each temperature of 500°C, 600°C, 700°C and 800°C) for 30 minutes (temperature rising rate: 50°C/minute).

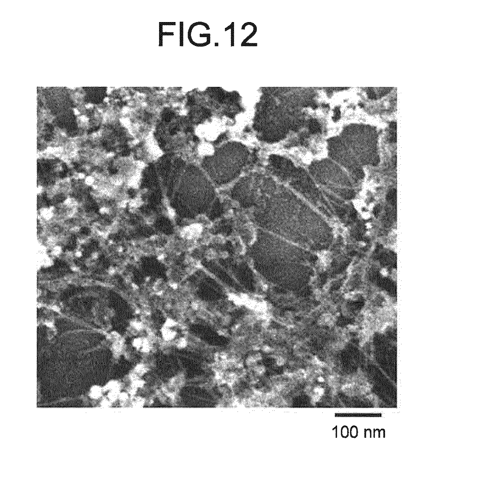

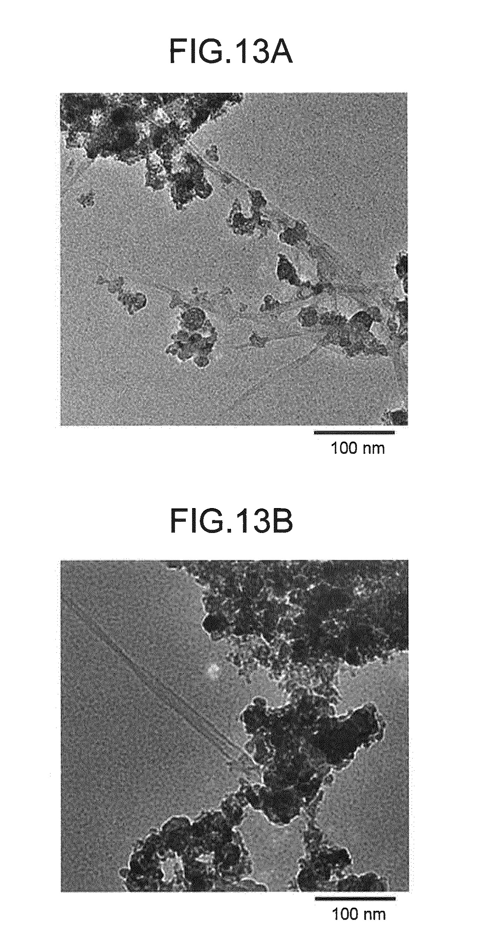

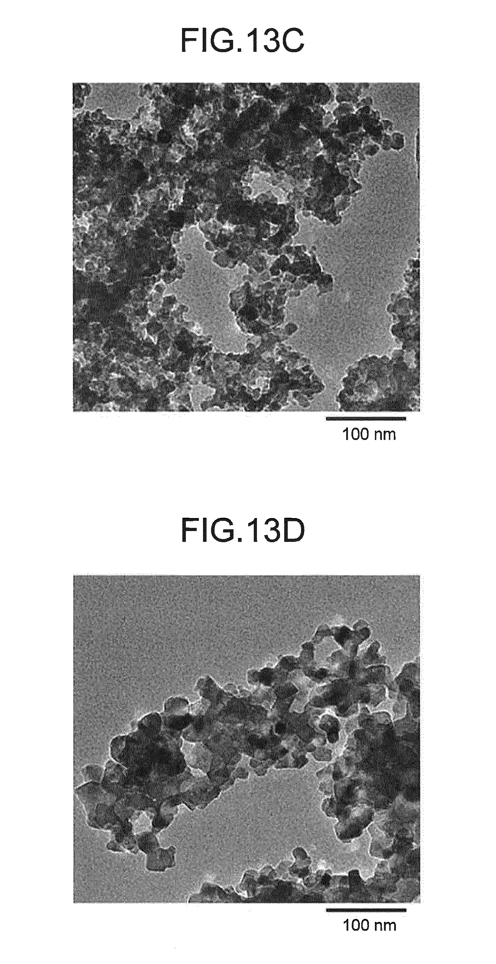

Black powder obtained by the burning at each temperature was stained with 3% PTA, and analyzed under TEM. The results are shown in FIG. 12. FIGS. 13A, 13B, 13C, and 13D are views showing transmission electron microscopic images of the structures obtained by heating the complex of CNT and CNHBP-Dps-TBP (CDT multimer) coated with titanium oxide.

As shown in FIG. 13A, when the complex was burned at 500°C, many linear structures could be observed. As shown in FIG. 13B, when the complex was burned at 600°C, the linear structure could be scarcely observed. As shown in FIGS. 13C and 13D, when the complex was burned at 700°C or above, the linear structure could not be observed at all.

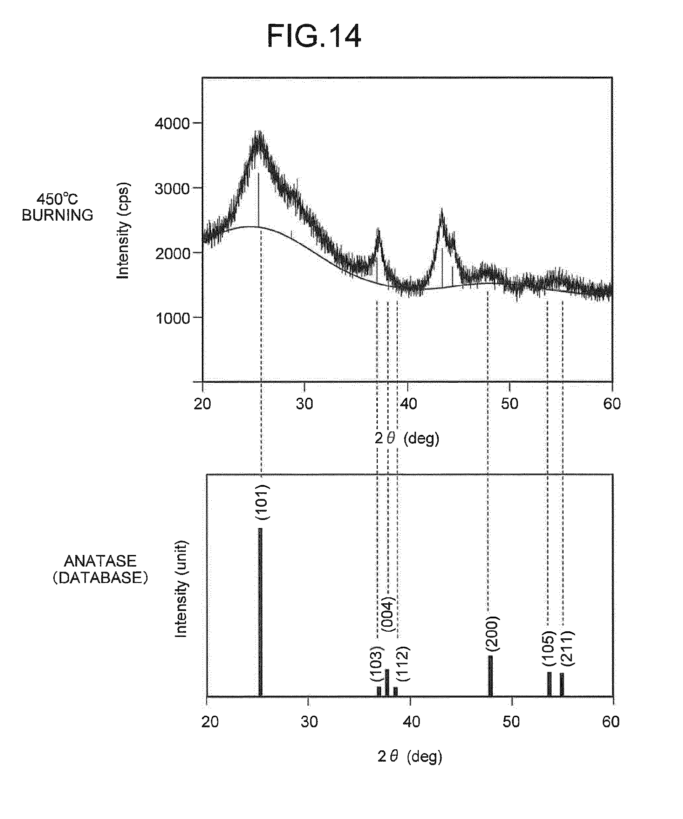

In order to further examine a crystalline condition of the obtained black powder, the black powder obtained by burning at 450°C was analyzed by X ray diffraction (XRD). The result is shown in FIG. 14. FIG. 14 is a view showing the result of the XRD analysis of the structure obtained by burning the complex of CNHBP-Dps-TBP (CDT) and CNT, which coated with titanium oxide at 450°C.

As shown in FIG. 14, peaks specific to (101) and (200) phases of anatase type TiO₂ crystal could be observed.

However, it was estimated that TiO was also included because peaks other than those of the anatase type TiO₂ crystal were also observed. Likewise, the black powders obtained by burning at 500°C and 600°C were analyzed by the XRD analysis, and the similar peak patterns were observed. Thus, it was suggested that the black powder obtained by burning at least at 600°C or below comprised an anatase type TiO₂ crystal having a photocatalytic activity.

### Example 8: Production example 1 of photoelectric conversion element (dye-sensitized solar cell)

The CNT/CDT/Ti complex obtained in Example 4 was used as a material for a photoelectric conversion layer of a photoelectric conversion element (dye-sensitized solar cell) to evaluate its effect on properties of the dye-sensitized solar cell. The production of the dye-sensitized solar cell was carried out by modifying the protocols by Solaronix (David Martineau, Dye Solar Cells for Real, 02.09.2010).

First, a CNT(SWNT)/CDT/Ti corresponding to 1 mL of a reaction solution was synthesized by the above described method, washed with water and suspended in an ethanol solution. The CNT(SWNT)/CDT/Ti complex was blended into a titanium oxide paste (Ti-Nanoxide D, Solaronix), and used as a material of a dye-sensitized solar cell. In order to form a photoelectric conversion layer, pieces of mending tape (3M Company, a thickness of about 100 µm) cut into 5 mm and doubly attached were attached to both ends of an FTO substrate (fluorine-doped tin oxide, Solaronix), respectively that was a transparent electrode substrate cut into 25 mm×25 mm. An interval between the tape pieces was 10 mm.

The titanium oxide paste containing the CNT(SWNT)/CDT/Ti complex was placed between the tape pieces, extended flatly using a slide glass, and left stand at 30°C for 30 minutes to dry the titanium oxide paste. The substrate on which the titanium oxide paste had been placed was placed in a burning furnace and burned at 450°C for 30 minutes. The temperature was raised at 90°C/minute. After the burning, the substrate was naturally cooled to 100°C or below. 1 mL of 0.2 g/L ruthenium (Ru) dye-sensitizing solution (N719, dissolved in dry ethanol, Solaronix) was applied to the burned substrate, which was then left stand at room temperature for 24 hours. The electrode substrate stained red by being left stand for 24 hours was washed with ethanol to remove the dye not adsorbed to the titanium oxide surface, dried using a dryer, and used as a photoelectrode.

A dye-sensitized solar cell was made using a Pt electrode (opposite electrode) obtained by coating the surface of the fluorine-doped tin oxide (FTO) film with platinum (Pt) having a thickness of 50 nm, and the structure comprising the above photoelectrode.

A sealing sheet (SX1170-25, Solaronix) was used as a sealing material, and heating at 120°C for 5 minutes was given using a hotplate. Araldite Rapid (Showa Highpolymer Co., Ltd.) that was an epoxy-based adhesive was applied to an adhered surface not completely sealed and left stand at 30°C for 2 hours to seal completely. Finally, an iodine electrolyte solution (Solaronix) was added to obtain the dye-sensitized solar cell.

The produced dye-sensitized solar cell was evaluated by illuminating with light at an intensity of 100 mW/cm² with a xenon lamp. The results are shown in FIG. 15. FIG. 15 is a graph showing the current-voltage characteristics of the dye-sensitized solar cells. The measurement of the current-voltage characteristics was performed with a delay time of 0 seconds. As is clear from FIG. 15, the current-voltage characteristics of the dye-sensitized solar cell using the porous structure body were very good.

Characteristics of the dye-sensitized solar cells were evaluated. More specifically, their photoelectric conversion efficiency (η(%)), i.e., the ratio of the incident energy of the applied light converted to electric power in the solar cells, open-circuit voltage (Voc (V)), i.e., voltage with no current flow, short-circuit current density (Jsc (mA/cm²)), i.e., current density measured at a voltage of 0 V, and fill factor (FF) in the relation for the photoelectric conversion efficiency η: photoelectric conversion efficiency η = Jsc × Voc × FF, were evaluated. The results are shown in Table 1.

As is evident from Table 1, the short-circuit current density was 12 mA/cm² in the dye-sensitized solar cell [device 2 (-)] comprising a photoelectrode formed of the titanium oxide paste alone. On the other hand, the short-circuit current density was 15 mA/cm² in the dye-sensitized solar cell [device 1 (+)] using the titanium oxide paste in which the CNT (SWNT)/CDT/Ti complex had been blended as the functional material of the photoelectrode. Thus, an amount of the current was increased by 25% by using the CNT (SWNT)/CDT/Ti complex as the functional material of the electrode. Further, the photoelectric conversion efficiency η was increased to 1.4 times by using the titanium oxide paste in which the CNT (SWNT)/CDT/Ti complex had been blended as the functional material of the photoelectrode.

**Table 1**

| | CNT/CDT/Ti | Voc (V) | Jsc (mA/cm²) | FF | η (%) |
|---|---|---|---|---|---|
| Device 1 | + | 0.67 | 15.3 | 0.37 | 4.0 |
| Device 2 | - | 0.64 | 12.3 | 0.36 | 2.8 |

### Example 9: Production example 2 of photoelectric conversion element (dye-sensitized solar cell)

The CNT/CDT/Ti complex obtained in Example 4 was used as the material for the photoelectric conversion layer of the photoelectric conversion element (dye-sensitized solar cell), and effects of the complex on the properties of the dye-sensitized solar cell was evaluated. The production of the dye-sensitized solar cell was carried out by modifying the protocol by Solaronix.

To synthesize a large amount of CNT(SWNT)/CDT/Ti, first, CDT1 at a final concentration of 0.3 mg/mL and CNT (SWNT, Sigma, 519308-250MG, carbon nanotube, single walled) at a final concentration of 0.3 Mg/mL were added to a 50mM potassium phosphate buffer solution (pH 6.0). 50 mL of the obtained solution was sonicated on ice using Digital Sonifier 450 (Branson, USA). More specifically, the solution was subjected to sonication cycles comprising applying ultrasonic waves (200 W, 25%) for 1 second and suspending the application of ultrasonic waves for 3 seconds until the total sonication time reached 5 minutes. A thick element with a diameter of 10 mm was used for the sonication.

After sonication, the solution was transferred to a 50 mL tube and centrifuged at 8,500 rpm for 10 minutes to remove CNT (SWNT) not bound to the CDT1. Ti [BALDH] (Sigma Aldrich) was added to the resultant solution at a final concentration of 2.5% by weight, and the mixture was left to stand at room temperature for 2 hours. Then the mixture was centrifuged in a 50 mL centrifuge tube at 8,500 rpm for 10 minutes, and the precipitate was collected to purify a CNT(SWNT)/CDT1/Ti complex. Then 40 mL of water was added, and the mixture was centrifuged in a 50 mL centrifuge tube at 8,500 rpm for 30 minutes for washing. 1 mL of water was added to the precipitate, and the resultant precipitate was transferred to a 1.5 mL microtube and centrifuged at 15,000 rpm for 20 minutes. The resultant precipitate was dispersed in 300 µL of ethanol to obtain a CNT(SWNT)/CDT1/Ti complex. This procedure was performed twice to obtain the CNT(SWNT)/CDT1/Ti complex from a total of 100 mL of the reaction solution. 200 µL of the solution containing the obtained complex was placed on a quartz board and subjected to heat treatment at 450°C for 30 minutes (temperature rising rate: 50°C/min) to obtain a CNT(SWNT)/TiO₂ complex. The obtained CNT(SWNT)/TiO₂ complex was re-dispersed in 100 µL of ethanol and then collected.

The obtained CNT(SWNT)/TiO₂ complex was kneaded with a titanium oxide paste (Ti-Nanoxide D, SOLARONIX) at a final concentration of 0.5 g/L (device 4) or 5 g/L (device 5), and the mixture was used as a material for a dye-sensitized solar cell. To form a photoelectric conversion layer, pieces of two-ply mending tape (3M Company, thickness: about 100 µm) cut into 5 mm were attached to opposite ends of an FTO substrate (fluorine-doped tin oxide, SOLARONIX) cut into 25 × 25 mm and used as a transparent electrode substrate. The spacing between the pieces of tape was 5 mm.

One of the titanium oxide pastes containing the CNT(SWNT)/TiO₂ complex at the above concentrations was placed between the pieces of tape, spread evenly using a glass slide, and left to stand at 30°C for 30 minutes to dry the titanium oxide paste. The substrate with the titanium oxide paste placed thereon was placed in a burning furnace and burned at 450°C for 30 minutes. The temperature rising rate was set to 90°C/min. After burning, the substrate was naturally cooled to 100°C or below. 1 mL of a 0.2 g/L ruthenium (Ru) sensitizing dye solution (N719, a dehydrated ethanol solution, SOLARONIX) was applied to the burned substrate and left to stand at room temperature for 24 hours. The electrode substrate left to stand for 24 hours and thereby stained red was washed with ethanol to remove the dye not adsorbed on the surface of titanium oxide and left to stand at room temperature for 10 minutes or longer to dry the electrode substrate, and the resultant electrode substrate was used as a photoelectrode.

A dye-sensitized solar cell was made using a Pt electrode (opposite electrode) obtained by coating the surface of the fluorine-doped tin oxide (FTO) film with platinum (Pt) having a thickness of 50 nm, and the structure comprising the above photoelectrode.

A sealing sheet (SX1170-25, Solaronix) was used as a sealing material, and the heating at 120°C for 5 minutes was given using the hotplate. Araldite Rapid (Showa Highpolymer Co., Ltd.) that was the epoxy-based adhesive was applied to the adhered surface not completely sealed and left stand at 60°C for 1 hour to seal completely. Finally, the iodine electrolyte solution (Solaronix) was added to obtain the dye-sensitized solar cell.

The produced dye-sensitized solar cell was evaluated by illuminating with light at an intensity of 100 mWjcm² with the xenon lamp. The results are shown in FIG. 16. FIG. 16 is a graph showing the current-voltage characteristics of the dye-sensitized solar cells. The measurement of the current-voltage characteristics was performed with a delay time of 100 ms. As can be seen from FIG. 16, the current-voltage characteristics of the dye-sensitized solar cells using the porous structure body were very good.

The characteristics of the produced dye-sensitized solar cells (devices 4 and 5) and a dye-sensitized solar cell (device 3) used as a control and containing no CNT(SWNT)/TiO₂ complex in the photoelectrode, that is, their open-circuit voltage Voc (V), short-circuit current density Jsc (mA/cm²), fill factor FF, and photoelectric conversion efficiency η (%), were evaluated. The results are shown in Table 2. More specifically, when the electrode produced using the titanium oxide paste containing the CNT(SWNT)/TiO₂ complex at a concentration of 0.5 g/L was used, the photoelectric conversion efficiency was improved to 4.7%.

**Table 2**

| | CNT/TiO₂ (g/L) | Voc (V) | Jsc (mA/cm²) | FF | η (%) |
|---|---|---|---|---|---|
| Device 3 | 0 | 0.66 | 10.8 | 0.58 | 4.1 |
| Device 4 | 0.5 | 0.70 | 11.3 | 0.60 | 4.7 |
| Device 5 | 5.0 | 0.68 | 9.7 | 0.55 | 3.6 |

### Example 10: Production example 3 of photoelectric conversion element (dye-sensitized solar cell)

The CNT/CDT/Ti complex obtained in Example 4 was used as the material for the photoelectric conversion layer of a photoelectric conversion element (dye-sensitized solar cell), and the influence of the complex on the characteristics of the dye-sensitized solar cell was evaluated. To produce the dye-sensitized solar cell, the protocols by SOLARONIX were modified and used.

To synthesize a large amount of CNT(SWNT)/CDT/Ti, first, CDT1 at a final concentration of 0.3 mg/mL and CNT (SWNT, Sigma, 519308-250MG, carbon nanotube, single walled) at a final concentration of 0.3 mg/mL were added to a 50mM potassium phosphate buffer solution (pH 6.0). 50 mL of the obtained solution was sonicated on ice using Digital Sonifier 450 (Branson, USA). More specifically, the solution was subjected to sonication cycles comprising applying ultrasonic waves (200 W, 25%) for 1 second and suspending the application of ultrasonic waves for 3 seconds until the total sonication time reached 5 minutes. A thick element with a diameter of 10 mm was used for sonication.

After sonication, the solution was transferred to a 50 mL tube and centrifuged at 8,500 rpm for 1 minute to remove CNT (SWNT) not bound to the CDT1. Ti [BALDH] (Sigma Aldrich) was added to the resultant solution at a final concentration of 2.5% by weight, and the mixture was left to stand at room temperature for 2 hours. Then the mixture was centrifuged in a 50 mL centrifuge tube at 8,500 rpm for 10 minutes, and the precipitate was collected to purify a CNT(SWNT)/CDT1/Ti complex. Then 40 mL of water was added, and the mixture was centrifuged in a 50 mL centrifuge tube at 8,500 for 30 minutes for washing. 1 mL of water was added to the precipitate, and the resultant precipitate was transferred to a 1.5 mL microtube and centrifuged at 15,000 rpm for 20 minutes. The resultant precipitate was dispersed in 300 µL of ethanol to obtain a CNT(SWNT)/CDT1/Ti complex. This procedure was performed twice to obtain the CNT(SWNT)/CDT1/Ti complex from a total of 100 mL of the reaction solution. 200 µL of the solution containing the obtained complex was placed on a quartz board and subjected to heat treatment at 450°C for 30 minutes (temperature rising rate: 50°C/min) to obtain a CNT(SWNT)/TiO₂ complex. The obtained CNT(SWNT)/TiO₂ complex was re-dispersed in 100 µL of ethanol and then collected.

The obtained CNT(SWNT)/TiO₂ complex was kneaded with a titanium oxide paste (Ti-Nanoxide D, SOLARONIX) at a final concentration of 0.5 g/L and used as a material for a dye-sensitized solar cell (device 7). To form a photoelectric conversion layer, an FTO substrate (fluorine-doped tin oxide, SOLARONIX) cut into 25 mm × 25 mm and used as a transparent electrode substrate was immersed for 30 minutes in a 40mM aqueous titanium tetrachloride solution heated to 80°C. The substrate was washed sequentially with water and ethanol, and pieces of two-ply mending tape (3M Company, thickness: about 100 µm) cut into 5 mm were attached to opposite ends of the substrate. The spacing between the pieces of tape was 5 mm.

The titanium oxide paste containing the CNT(SWNT)/TiO₂ complex at the above concentration was placed between the pieces of tape, spread evenly using a glass slide, and left to stand at 120°C for 5 minutes to dry the titanium oxide paste. The substrate with the titanium oxide paste placed thereon was placed in a burning furnace and burned at 500°C for 30 minutes. The temperature rising rate was set to 90°C/min. After burning, the substrate was naturally cooled to 100°C or below. 1 mL of a 0.2 g/L ruthenium (Ru) sensitizing dye solution (N719, a dehydrated ethanol solution, SOLARONIX) was applied to the burned substrate and left to stand at room temperature for 24 hours. The electrode substrate left to stand for 24 hours and thereby stained red was washed with ethanol to remove the dye not adsorbed on the surface of titanium oxide and left to stand at room temperature for 10 minutes or longer to dry the electrode substrate, and the resultant electrode substrate was used as a photoelectrode.

A dye-sensitized solar cell was produced using a structure comprising the above photoelectrode and a Pt electrode (counter electrode) prepared by coating the surface of a fluorine-doped tin oxide (FTO) film with platinum (Pt) to a thickness of 50 nm.

A sealing sheet (SX1170-25, SOLARONIX) was used as a sealing material and heated at 120°C for 5 minutes using a hotplate. Araldite Rapid (Showa Highpolymer Co., Ltd.), an epoxy-based adhesive, was applied to the bonding surface not completely sealed and then left to stand at 60°C for 1 hour to seal the bonding surface. Finally, an iodine electrolyte solution (SOLARONIX) was poured to obtain a dye-sensitized solar cell.

The produced dye-sensitized solar cell was irradiated with light at an intensity of 100 mW/cm² using a xenon lamp to evaluate current-voltage characteristics. The results are shown in FIG. 17. FIG. 17 is a graph showing the current-voltage characteristics of dye-sensitized solar cells. The measurement of the current-voltage characteristics was performed with a delay time of 100 ms. As is clear from FIG. 17, the current-voltage characteristics of the dye-sensitized solar cell using the porous structure body were very good.

The characteristics of the produced dye-sensitized solar cell (device 7) and a dye-sensitized solar cell (device 6) used as a control and containing no CNT(SWNT)/TiO₂ complex, namely, their open-circuit voltage Voc (V), short-circuit current density Jsc (mA/cm²)m, fill factor FF, and photoelectric conversion efficiency η (%) were evaluated. The results are shown in Table 3. More specifically, when the electrode produced using the titanium oxide paste containing the CNT(SWNT)/TiO₂ complex at a concentration of 0.5 g/L was used, the photoelectric conversion efficiency was improved to 5.8%.

**Table 3**

| | CNT/TiO₂(g/L) | Voc (V) | Jsc (mA/cm²) | FF | η (%) |
|---|---|---|---|---|---|
| Device 6 | 0 | 0.71 | 10.9 | 0.58 | 4.5 |
| Device 7 | 0.5 | 0.69 | 15.0 | 0.56 | 5.8 |

### Example 11: Production example 3 of photoelectric conversion element (dye-sensitized solar cell)

The CNT/CDT/Ti complex obtained in Example 4 was used as the material for the photoelectric conversion layer of a photoelectric conversion element (dye-sensitized solar cell), and the influence of the complex on the characteristics of the dye-sensitized solar cell was evaluated. To produce the dye-sensitized solar cell, the protocols by SOLARONIX were modified and used.

First, CNT(SWNT)/CDT/Ti was synthesized in an amount corresponding to 1 mL of a reaction solution using the same method as in the above Example, washed with water, and suspended in an ethanol solution. The CNT(SWNT)/CDT/Ti complex was kneaded with a titanium oxide paste (Ti-Nanoxide D, SOLARONIX) at a final concentration of 0.2% by weight (titanium oxide: 10% by weight to 14% by weight, complex: 0.02% by weight to 0.03% by weight) and used as a material for a dye-sensitized solar cell. To form a photoelectric conversion layer, an FTO substrate (fluorine-doped tin oxide, SOLARONIX) cut into 25 × 25 mm and used as a transparent electrode substrate was immersed in a 40mM aqueous titanium tetrachloride solution at 80°C for 30 minutes. Pieces of two-ply mending tape (3M Company, thickness: about 100 µm) cut into 5 mm were attached to opposite ends of the FTO substrate. The spacing between the pieces of tape was 5 mm.

The titanium oxide paste containing the CNT(SWNT)/CDT/Ti complex was placed between the pieces of tape, spread evenly using a glass slide, and left to stand at 30°C for 30 minutes to dry the titanium oxide paste. The substrate with the titanium oxide paste placed thereon was placed in a burning furnace and burned at 450°C for 30 minutes. The temperature rising rate was set to 90°C/min. After burning, the substrate was naturally cooled to 100°C or below. After cooling, the titanium oxide portion on the FTO substrate was cut into a 5 mm x 10 mm piece, and the cut substrate piece was immersed in 1 mL of a 0.2 g/L ruthenium (Ru) sensitizing dye solution (N719, a dehydrated ethanol solution, SOLARONIX) and left to stand at room temperature for 24 hours. The electrode substrate left to stand for 24 hours and thereby stained red was washed with ethanol to remove the dye not adsorbed on the surface of titanium oxide and left to stand at room temperature to dry the electrode substrate, and the resultant electrode substrate was used as a photoelectrode (corresponding to device 8).

A control photoelectrode (corresponding to device 9) was produced using a titanium oxide electrode containing 0.2% by weight of a CNT/titanium oxide complex synthesized by oxidization treatment. Another control photoelectrode (corresponding to device 10) was produced using a titanium oxide electrode containing 0.2% by weight of CNT, and another control photoelectrode (corresponding to device 11) was produced using a titanium oxide electrode containing no CNT. The synthesis of the CNT/titanium oxide complex by oxidization treatment was performed with reference to a method in a reference (W. Wang et al. (2005) Journal of Molecular Catalysis A: Chemical, 235, 194-199.). More specifically, 34 mL (0.1 mol) of a titanium butoxide solution was added to 200 mL of ethanol, and the mixture was stirred at room temperature for 30 minutes. Then 28 mL of nitric acid (35% by weight) was added, and an appropriate amount of CNT was added. The mixture was stirred at room temperature overnight until the mixture became a gel. Then centrifugation was performed, and the precipitate was collected and left to stand at 80°C overnight to dry the precipitate, whereby a CNT/titanium oxide complex was obtained.

A dye-sensitized solar cell was produced using a structure comprising one of the above photoelectrodes and a Pt electrode (counter electrode) prepared by coating the surface of a fluorine-doped tin oxide (FTO) film with platinum (Pt) to a thickness of 50 nm.

A sealing sheet (SX1170-25, SOLARONIX) was used as a sealing material and heated at 120°C for 5 minutes using a hotplate. Araldite Rapid (Showa Highpolymer Co., Ltd.), an epoxy-based adhesive, was applied to the bonding surface not completely sealed and left to stand at 30°C for 2 hours to seal the bonding surface. Finally, an iodine electrolyte solution (SOLARONIX) was poured to obtain a dye-sensitized solar cell.

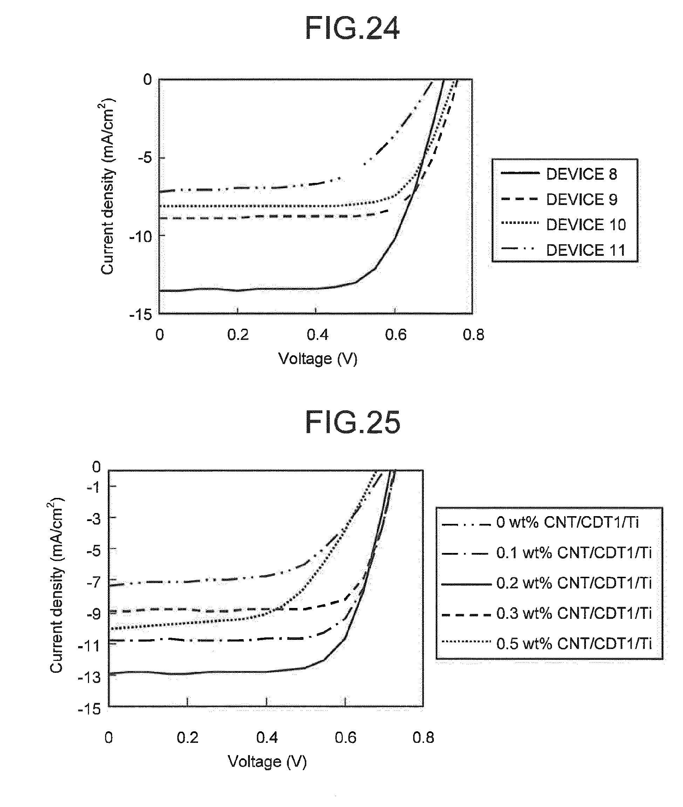

The produced dye-sensitized solar cells (devices 8 to 11) were irradiated with light at an intensity of 100 mW/cm² using a xenon lamp for evaluation. The results are shown in FIG. 24. FIG. 24 is a graph showing the current-voltage characteristics of the dye-sensitized solar cells. The measurement of the current-voltage characteristics was performed with a delay time of 100 ms. As is clear from FIG. 24, the current-voltage characteristics of the dye-sensitized solar cells (devices 8, 9, and 10) comprising the photoelectrodes formed from titanium oxide pastes containing nanomaterials were better than the current-voltage characteristics of the dye-sensitized solar cell (device 11) comprising the photoelectrode formed only from a titanium oxide paste. Particularly, the current-voltage characteristics of the dye-sensitized solar cell (device 8) in which the complex of CNT and titanium oxide synthesized using CDT1 was used as the functional material for the electrode was very good.

The open voltage Voc (V), the short-circuit current density Jsc (mA/cm 2) the fill factor FF, and the photoelectric conversion efficiency η (%) were evaluated as the properties of the dye-sensitized solar cell. The results are shown in Table 4.

As is evident from Table 4, the larger short-circuit current density was measured in the dye-sensitized solar cells comprising the photoelectrode formed from the titanium oxide paste containing a nanomaterial (devices 8, 9, and 10) than in the dye-sensitized solar cell comprising the photoelectrode formed from the titanium oxide paste alone (device 11). In particular, the short-circuit current density was increased by 180% by using the CNT(SWNT)/CDT/Ti complex as the functional material of the electrode. The short-circuit current density in the device using the complex of titanium oxide and CNT synthesized using CDT1 (device 8) as the functional material of the electrode was larger than the short-circuit current density in the device using CNT alone as the functional material of the electrode (device 10) and the short-circuit current density in the device using the complex of titanium oxide and CNT synthesized by the oxidation treatment as the functional material of the electrode (device 9).

The impedance of each of devices 8 and 10 was measured. A potentiostat/galvanometer SP-50 (Bio-Logic) was used for the measurement. In the measurement, frequency was changed from 500 mHz to 10 kHz, and resistance components were determined from current values and the phase of the response of voltage values before and after the change.

The results showed that the sheet resistance of the substrate in device 8 was 18 Ω and the sheet resistance of the substrate in device 10 was 35 Ω. The resistances at the interface between the photoelectrode with the dye and the electrolyte were 10 Ω and 15 Ω. Therefore, when the CNT(SWNT)/CDT/Ti complex was used as the functional material for the electrode, the sheet resistance component was reduced by 59%, and the resistance at the interface between the photoelectrode with the dye and the electrolyte was reduced by 33%, so that the conductivity of the titanium oxide electrode was found to be improved.

First, for a reason why the short-circuit current density was enhanced by introducing CNT into the titanium oxide electrode, this was thought to be because generated carrier passed through CNT and could move to a conductive film (FTO electrode) prior to being bound again to decrease the amount of the current. It was also thought that by forming the complex of CNT and titanium oxide by the CDT1 treatment or the oxidation treatment, CNT and titanium oxide could be adhered tightly and resistance between CNT and titanium oxide was reduced. Thus, it was thought that the carrier generated in the circumference of titanium oxide more efficiently moved to CNT than in the case of introducing untreated CNT into the titanium oxide paste. In the complex of titanium oxide/CNT synthesized by an acid treatment, it has been thought that the structure of CNT is partially broken by the acid and the movement of the carrier in CNT is inhibited. However, if the protein CDT1 is used, the CNT/titanium oxide complex can be synthesized without impairing the structure of CNT. Further, it was thought that the surface area was enhanced by the empty holes derived from CDT1 and the dye could be supported more abundantly. Thus, it was thought that the generated carrier was more abundant, a moving rate of the carrier in CNT was kept more stably, and thus the larger short-circuit current was observed in the complex of titanium oxide/CNT synthesized using CDT1 than in the complex of titanium oxide/CNT synthesized by the acid treatment.

As a result of the enhanced short-circuit current density, the photoelectric conversion efficiency η in the device using the titanium oxide paste in which CNT(SWNT)/CDT/Ti had been blended (device 8) as the functional material of the photoelectrode was 2.2 times larger than the photoelectric conversion efficiency η in the device using the titanium oxide paste not containing CNT (device 11) as the functional material of the photoelectrode. The highest photoelectric conversion efficiency was measured in the device using the titanium oxide paste in which CNT(SWNT)/CDT/Ti had been blended as the functional material of the photoelectrode among the devices produced this time. Therefore, it was found that the performance of the solar cell could be enhanced by using the complex of titanium oxide and CNT synthesized using CDT1 under the mild condition.

It was also confirmed that the higher short-circuit current density and the higher photoelectric conversion efficiency η than in the devices produced in Example 8 could be obtained by treating the FTO substrate with titanium tetrachloride or blending the CNT(SWNT)/CDT/Ti complex at a final concentration of 0.2% by weight into the titanium oxide electrode.

**Table 4**

| | Voc (V) | Jsc (mA/cm²) | FF | η (%) |
|---|---|---|---|---|
| Device 8 | 0.73 | 13.1 | 0.68 | 6.5 |
| Device 9 | 0.76 | 8.9 | 0.73 | 5.0 |
| Device 10 | 0.75 | 8.2 | 0.72 | 4.5 |
| Device 11 | 0.71 | 7.3 | 0.58 | 3.0 |

### Example 12: Production example 4 of photoelectric conversion element (dye-sensitized solar cell)

The CNT/CDT/Ti complex obtained in Example 4 was used as the material for the photoelectric conversion layer of a photoelectric conversion element (dye-sensitized solar cell), and the influence of the complex on the characteristics of the dye-sensitized solar cell was evaluated. To produce the dye-sensitized solar cell, the protocols by SOLARONIX were modified and used.

First, CNT(SWNT)/CDT1/Ti was synthesized using the same method as in the above Example, washed with water, and suspended in an ethanol solution. The CNT(SWNT)/CDT/Ti complex was kneaded with a titanium oxide paste (Ti-Nanoxide D, SOLARONIX) at a final concentration of 0.1% by weight to 1% by weight. More specifically, the CNT(SWNT)/CDT/Ti complex was kneaded with the titanium oxide paste such that the final concentration of the CNT(SWNT)/CDT/Ti complex was 0.1% by weight (corresponding to device 13), 0.15% by weight (corresponding to device 14), 0.2% by weight (corresponding to device 15), 0.25% by weight (corresponding to device 16), 0.3% by weight (corresponding to device 17), 0.5% by weight (corresponding to device 18), 1.0% by weight (corresponding to device 19), 5.0% by weight (corresponding to device 20), or 0.06% by weight (corresponding to device 21). The resultant pastes were used as the materials for dye-sensitized solar cells. As a control, a titanium oxide paste containing no CNT(SWNT)/CDT1/Ti complex was also used (corresponding to device 12). To form a photoelectric conversion layer, an FTO substrate (fluorine-doped tin oxide, SOLARONIX) cut into 25 mm × 25 mm and used as a transparent electrode substrate was immersed in a 40mM aqueous titanium tetrachloride solution at 80°C for 30 minutes. Pieces of two-ply mending tape (3M Company, thickness: about 100 µm) cut into 5 mm were attached to opposite ends of the FTO substrate. The spacing between the pieces of tape was 5 mm.

One of the titanium oxide pastes containing the CNT(SWNT)/CDT1/Ti complex at the above concentrations was placed between the pieces of tape, spread evenly using a glass slide, and left to stand at 30°C for 30 minutes to dry the titanium oxide paste. The substrate with the titanium oxide paste placed thereon was placed in a burning furnace and burned at 450°C for 30 minutes. The temperature rising rate was set to 90°C/min. After burning, the substrate was naturally cooled to 100°C or below. After cooling, the titanium oxide portion on the FTO substrate was cut into a 5 mm x 10 mm piece, and the substrate was immersed in 1 mL of a 0.2 g/L ruthenium (Ru) sensitizing dye solution (N719, a dehydrated ethanol solution, SOLARONIX) and left to stand at room temperature for 24 hours. The electrode substrate left to stand for 24 hours and thereby stained red was washed with ethanol to remove the dye not adsorbed on the surface of titanium oxide and dried at room temperature, and the resultant electrode substrate was used as a photoelectrode.

A dye-sensitized solar cell was produced using a structure comprising the above photoelectrode and a Pt electrode (counter electrode) prepared by coating the surface of a fluorine-doped tin oxide (FTO) film with platinum (Pt) to a thickness of 50 nm.

A sealing sheet (SX1170-25, SOLARONIX) was used as a sealing material and heated at 120°C for 5 minutes using a hotplate. Araldite Rapid (Showa Highpolymer Co., Ltd.), an epoxy-based adhesive, was applied to the bonding surface not completely sealed and left to stand at 30°C for 2 hours to seal the bonding surface. Finally, an iodine electrolyte solution (SOLARONIX) was poured to obtain a dye-sensitized solar cell.

The produced dye-sensitized solar cells (devices 12 to 21) were irradiated with light at an intensity of 100 mW/cm² using a xenon lamp for evaluation. The results of evaluation of devices 12, 13, 15, 17, and 18 are shown in FIG. 25. FIG. 25 is a graph showing the current-voltage characteristics of the dye-sensitized solar cells. The measurement of the current-voltage characteristics was performed with a delay time of 100 ms. As is clear from FIG. 25, the current-voltage characteristics of the dye-sensitized solar cells using the titanium oxide electrodes formed by adding the CNT(SWNT)/CDT1/Ti complex at a final concentration of 0.1% by weight to 0.5% by weight were favorable.

The characteristics of the dye-sensitized solar cells, namely, their open-circuit voltage Voc (V), short-circuit current density Jsc (mA/cm²), fill factor FF, and photoelectric conversion efficiency η (%), were evaluated. The results are shown in Table 5.

As is clear from Table 5, improvement in photoelectric conversion efficiency was observed in the dye-sensitized solar cells (devices 13, 14, 15, 16, 17, 18, and 19) using the titanium oxide electrodes formed by adding the CNT(SWNT)/CDT1/Ti complex at a final concentration of 0.06% by weight to 0.5% by weight. Particularly, in the dye-sensitized solar cell (device 15) using the titanium oxide paste containing the CNT(SWNT)/CDT/Ti complex at a final concentration of 0.2% by weight, the highest photoelectric conversion efficiency, 6.6%, was measured.

As described above, when a titanium oxide electrode is formed by adding a prescribed amount of the CNT(SWNT)/CDT1/Ti complex, the performance of the dye-sensitized solar cell can be improved.

**Table 5**

| | CNT/CDT1/Ti (wt%) | Voc (V) | Jsc (mA/cm²) | FF | **η** (%) |
|---|---|---|---|---|---|
| Device 12 | 0.0 | 0.705 | 7.3 | 0.579 | 2.97 |
| Device 13 | 0.1 | 0.730 | 10.8 | 0.720 | 5.68 |
| Device 14 | 0.15 | 0.728 | 12.9 | 0.679 | 6.39 |
| Device 15 | 0.2 | 0.718 | 12.9 | 0.714 | 6.62 |
| Device 16 | 0.25 | 0.723 | 11.6 | 0.725 | 6.05 |
| Device 17 | 0.3 | 0.728 | 8.9 | 0.785 | 4.91 |
| Device 18 | 0.5 | 0.690 | 10.0 | 0.561 | 3.87 |
| Device 19 | 1.0 | 0.723 | 12.2 | 0.314 | 2.78 |
| Device 20 | 5.0 | 0.710 | 7.16 | 0.492 | 3.34 |
| Device 21 | 0.06 | 0.731 | 6.27 | 0.476 | 4.34 |

### Example 13: Preparation of fusion protein CcDT

### (1) Production of strain for expressing CcDT

A mutant protein having the similar nature as that of CDT was constructed using Dps derived from *Corynebacterium glutamicum.*

PCR was carried out using genomic DNA from *Corynebacterium glutamicum* as the template, and oligonucleotides consisting of the nucleotide sequences of SEQ ID NO:24 and SEQ ID NO:25 as the primers.

The obtained PCR product was purified using Wizard SV Gel and PCR Clean-Up System (Promega, USA) and digested with the restriction enzymes NdeI and EcoRI.

Meanwhile, the plasmid pET20b (Merck, Germany) was digested with the restriction enzymes NdeI and BamHI.

The PCR product and the plasmid digested with the restriction enzymes were ligated using the T4 ligase (Promega, USA). *E. coli* JM109 (Takara Bio Inc., Japan) was transformed with the resulting DNA to construct JM109 possessing an expression plasmid (pET20-CcDT) carrying the gene encoding Dps derived from *Corynebacterium glutamicum,* the N-terminus of which was fused with the carbon nanohorn-binding peptide (CNHBP), and the C-terminus of which was fused with the titanium-binding peptide (TBP) (CcDT, SEQ ID NOS:28 and 29).

pET20-CcDT was purified from this transformant using Wizard Plus Minipreps System (Promega, USA). Finally, BL21 (DE3) (Invitrogen, USA) was transformed with pET20-CcDT to obtain the strain BL21 (DE3)/pET20-CcDT for expressing the protein.

### (2) Purification of CcDT

In order to obtain the CcDT protein, BL21 (DE3)/pET20-CcDT was cultured in 1 mL of LB medium (containing 100 mg/L of ampicillin) at 37°C. Eighteen hours after starting the cultivation, the resulting culture medium was inoculated to 100 mL of new LB medium (containing 100 mg/L of ampicillin), and cultured with shaking using a 500 mL flask at 37°C for 24 hours. The resulting microbial cells were collected by centrifugation (6,000 rpm, 5 minutes), and suspended in 5 mL of 50 mM Tris-HCl buffer solution (pH 8.0). The microbial solution was sonicated to disrupt the microbial cells. The resulting solution was centrifuged at 6,000 rpm for 15 minutes and a supernatant fraction was collected. The collected solution was heated at 60°C for 20 minutes and then rapidly cooled on ice. The cooled solution was centrifuged at 6,000 rpm for 15 minutes and a supernatant (about 5 mL) was collected again. This solution was sterilized using the disc filter (Millex GP. 0.22 µm, Millipore, USA). And, the solution was ultrafiltrated and concentrated using icon-Ultra-15 (NMWL. 50000, Millipore, USA), and the buffer solution in which the protein was dissolved was replaced with Tris-HCl buffer solution (50 mM Tris-HCl pH 8.0) to obtain 2.5 mL of a protein solution.

To purify CcDT from the resulting protein solution, anion exchange chromatography was used. Specifically, 2.5 mL of the protein solution was applied to HiLoard 26/10 Q-Sepharose High Performance column (GE healthcare, USA) equilibrated with 50 mM Tris-HCl buffer solution (pH 8.0). The separation/purification was carried out at a flow rate of 4.0 mL/minute by making a concentration gradient of the salt from 0 mM to 500 mM NaCl in 50 mM Tris-HCl buffer solution (pH 8.0), and fractions containing CcDT were collected.

### Example 14: Confirmation of formation of CcDT multimer

The obtained CcDT was stained with 3% PTA (phosphotungstic acid), and analyzed under the transmission electron microscope. The result is shown in Fig 18. FIG. 18 is a view of a transmission electron microscopic image of obtained CcDT.

As shown in Fig.18, as a result, it was found that CcDT formed a cage-like multimer having a diameter of about 9 nm, as is similar to CDT.

### Example 15: Confirmation of binding between fusion protein CcDT and carbon nanotube

The binding between CcDT and CNT was measured by QCM method. First, the surface of the gold sensor was washed by placing 50 µL of a washing solution [in which 98% (w/v) sulfuric acid and 30% (w/v) hydrogen peroxide water were mixed at 3:1] on the gold sensor for the measurement for five minutes and then washing it out with water. CNT was mixed at a final concentration of 1 mg/mL with 1% SDS solution, and the mixture was sonicated for 30 minutes to prepare a CNT solution. 2 µL of this CNT solution was mounted on the gold electrode and dried naturally at room temperature. After the drying, the sensor was washed twice with water to wash out CNT not bound to the sensor. The sensor was further washed once with phosphate buffer solution A (50 mM potassium phosphate buffer solution containing 0.001% (w/v) Tween 20, pH 7.0).

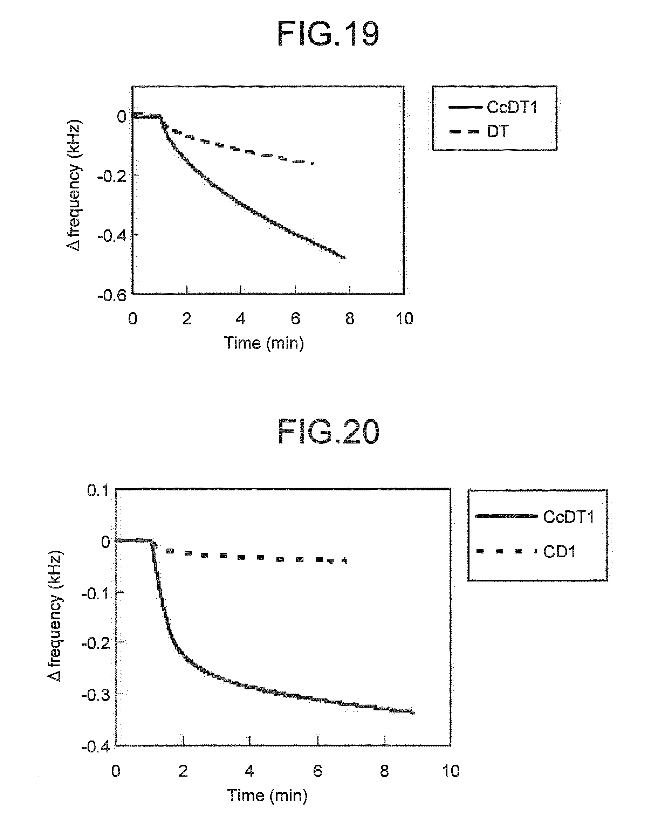

A CNT sensor was attached to the main body (Affinix QNµ, Initium), and the frequency value was stabilized by dropping the phosphate buffer solution A on the CNT sensor and leaving it stand at room temperature for 30 minutes to one hour. After stabilizing the frequency value, 500 µL of a reaction solution containing CcDT at a final concentration of 1 mg/L was added, and the change of the frequency was measured. The result is shown in Fig 19. Fig 19 is a graph showing the result of confirmation of binding between CcDT and CNT.

As shown in Fig 19, as a result, it was observed that CcDT having CNHBP (CcDT1: solid line) could bind to CNT more abundantly than DT having no CNHBP (DT: dashed line). Therefore, it was found that CcDT has the stronger ability to bind to CNT than DT. It was speculated that this ability of CcDT to bind to CNT depended on CNHBP.

### Example 16: Confirmation of binding between fusion protein CcDT and titanium oxide

The binding between CcDT and titanium oxide was measured by QCM method. First, the surface of the titanium oxide sensor was washed by placing 50 µL of the washing solution [in which 98% (w/v) sulfuric acid and 30% (w/v) hydrogen peroxide water were mixed at 3:1] on the sensor for the measurement for five minutes and then washing it out with water. The titanium oxide sensor was attached to the main body (Affinix QNp., Initium), and the frequency value was stabilized by dropping the phosphate buffer solution B (50 potassium phosphate buffer solution, pH 7.0) on the titanium oxide sensor and leaving it stand at room temperature for 30 minutes to one hour. After stabilizing the frequency value, 500 µL of a reaction solution containing CcDT was added at a final concentration of 1 mg/L, and the change of the frequency was measured. The result is shown in Fig 20. Fig 20 is a graph showing the result of confirmation of binding between CcDT and titanium oxide.

As shown in Fig 20, as a result, it was observed that CcDT having TBP (CcDT1: solid line) was bound to titanium oxide more abundantly than CD having no TBP (CD: dashed line). Therefore, it was found that CcDT had the stronger ability to bind to titanium oxide than CD. It was speculated that this ability of CcDT to bind to titanium oxide depended on TBP.

### Example 17: Production of strain for expressing fusion protein CNHBP-Dps-ZnO1' (CDZ)

The metal-encapsulating protein Dps from Listeria innocua, the N-terminus of which is fused with the carbon nanohorn-binding peptide (abbreviated as CNHBP and consisting of the amino acid sequence DYFSSPYYEQLF (SEQ ID NO:6), see International Publication No. WO2006/068250), and the C-terminus of which is fused with a zinc oxide-precipitating peptide (abbreviated as ZnO1' and consisting of the amino acid sequence EAHVMHKVAPRPGGGSC (SEQ ID NO:30), see Umetsu et al., Adv. Mater., 17, 2571-2575 (2005)) was constructed (abbreviated as CNHBP-Dps-ZnO1' or CDZ, SEQ ID NOS:31 and 32) by the following procedure.

First, PCR was carried out using pET20-CDT as the template DNA, and the oligonucleotides consisting of the nucleotide sequence represented by SEQ ID NO:11 and the nucleotide sequence of tttGGATCCttaAcaACTAccTccAccAggAcGTggAgcAacTttAtgcatTacAtgTg cTtcttctaatggagcttttc (SEQ ID NO:33) as the primers. The obtained PCR product was purified using Wizard SV Gel and PCR Clean-Up System (Promega, USA) and digested with the restriction enzymes DpnI and BamHI. The PCR product digested with the restriction enzymes was self-ligated using the T4 ligase (Promega, UAS). E. coli BL21 (DE3) (Nippon Gene, Japan) was transformed with the self-ligated PCR product to construct BL21 (DE3) possessing the expression plasmid carrying the gene encoding Dps (CDZ), the N-terminus and the C-terminus of which were fused to the carbon nanohorn-binding peptide and the zinc oxide-precipitating peptide, respectively (pET20-CDZ).

### Example 18: Purification of fusion protein CDZ

BL20 (DE3)/pET20-CDZ was inoculated to 100 mL of the LB medium (containing 100 mg/L of ampicillin), and cultured with shaking using a 500 mL flask at 37°C for 24 hours. The resulting microbial cells were collected by centrifugation (6,000rpm, 5 minutes) and suspended in 5 mL of 50 Tris-HCl buffer solution (pH 8.0). The microbial solution was sonicated to disrupt the microbial cells. The resulting solution was centrifuged at 6,000 rpm for 15 minutes to collect a supernatant fraction. The collected solution was heated at 60°C for 20 minutes and then cooled on ice rapidly after the heating. The cooled solution was centrifuged at 6,000 rpm for 15 minutes to collect a supernatant (about 5 mL) again. This solution was sterilized using the disc filter (Millex GP 0.22 µm, Millipore, USA). This solution was ultrafiltrated and concentrated using icon-Ultra-15 (NMWL. 50000, Millipore, USA), and the buffer solution in which the protein was dissolved was replaced with Tris-HCl buffer solution (50 mM Tris-HCl solution, pH 8.0) to obtain 2.5 mL of a protein solution.

Anion exchange chromatography was used for purifying CDZ from the resulting protein solution. Specifically, 2.5 mL of the protein solution was applied to HiLoard 26/10 Q-Sepharose High Performance column (GE healthcare, USA) equilibrated with 50 mM Tris-HCl buffer solution (pH 8.0). The separation/purification was carried out at a flow rate of 4.0 mL/minute by making the concentration gradient of the salt from 0 mM to 500 mM NaCl in 50 Tris-HCl buffer solution (pH 8.0), and fractions containing CDZ were collected. Further, the collected solution was ultrafiltrated and concentrated using icon-Ultra-15 (NMWL. 50000, Millipore, USA), and the buffer solution in which the protein was dissolved was replaced with pure water to obtain a CDZ solution.

### Example 19:Confirmation of formation of CDZ multimer

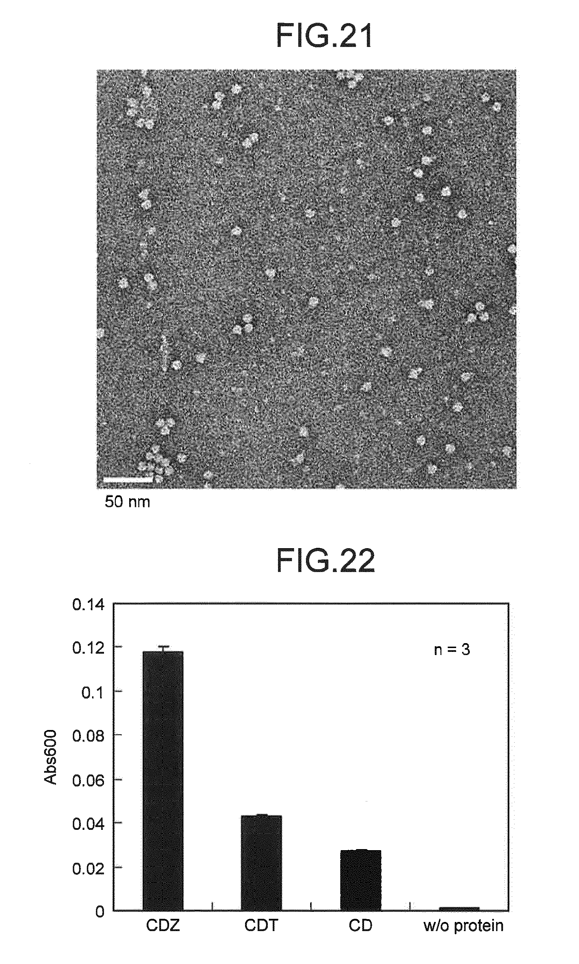

CDZ dissolved in 50 Tris-HCl buffer solution (pH 8.0) before the buffer solution was replaced with pure water was stained with 3% PTA (phosphotungstic acid), and analyzed under the transmission electron microscope. The result is shown in Fig 21. FIG. 21 is a view showing a transmission electron microscopic image of CDZ. As a result, it was found that CDZ formed the multimer forming an inner cavity having a diameter of about 9 nm, as is similar to CDT.

### Example 20: Facilitation of white precipitate formation from aqueous solution of zinc sulfate by CDZ

First, CDZ, CDT, or CD dissolved in pure water was added at a final concentration of 0.1 mg/mL to an aqueous solution of 0.1 M zinc sulfate. After the solution was left stand at room temperature for one hour, a turbidity of the solution was measured using the light at 600 nm. The results are shown in FIG. 22. FIG. 22 is a graph showing facilitation of formation of a white precipitate from an aqueous solution of zinc sulfate by CDZ. The white precipitate occurred prominently in the solution containing CDZ. It was thought that this white precipitate was zinc hydroxide or zinc oxide. On the other hand, no precipitate occurred at all in the solution containing no protein. From above, it was suggested that CDZ had an activity to precipitate the zinc compound. Herein, it is known that zinc hydroxide becomes zinc oxide by being heated at about 125°C.

### Example 21: Confirmation of binding ability of CNHBP in CDZ

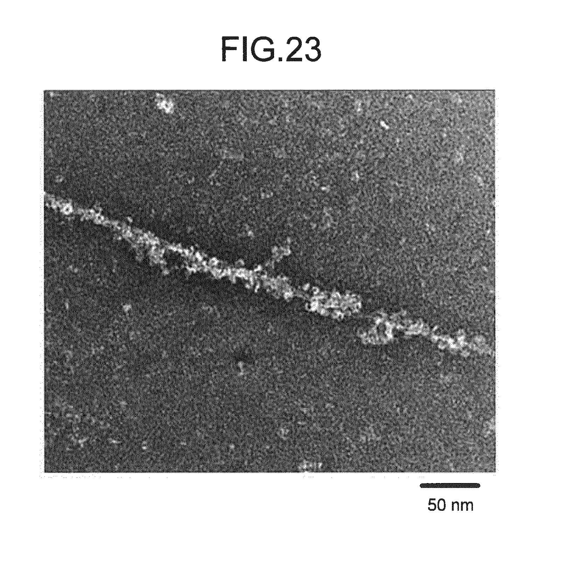

An activity of the carbon nanohorn-binding peptide (CNHBP) fused to the N-terminus of CDZ was examined. CDZ and CNT (Sigma, 519308, carbon nanotube single walled) were added at a final concentration of 0.3 mg/mL to potassium phosphate buffer solution (50 mM, pH 6.0). The ultrasonic pulse (200 W, duty 20%) for one second with an interval of 3 seconds was given to this solution for 5 minutes using Digital Sonifier 450 (Branson, USA). The sonicated CDZ/CNT mixed solution was centrifuged (15,000 rpm, 5 minutes), a protein/CNT complex comprised in a supernatant was stained with 3% PTA and observed under the transmission electron microscope (JEM-2200FS, 200 kV). The result is shown in Fig 23. Fig 23 is a view showing a transmission electron microscopic image of a complex of CDZ and CNT.

As a result, an appearance where CDZ was bound to the circumference of CNT could be observed in the solution containing CDZ having CNHBP at its N-terminus. Therefore, it was found that CDZ retained an activity of binding to CNT.

### Example 22: Formation of CNT forest

With the aim of producing a forest-like CNT(SWNT)/TiO₂ electrode, synthesis of CNTs arranged in a forest form (formation of a CNT forest) used as a base of the electrode was tried.

First, iron nanoparticles used as a catalyst for formation of a CNT forest were prepared using a ferritin protein fused with a titanium-binding peptide (minTBP-1) (TBF, T. Hayashi, et al., (2006) Nano Lett. 6 515.). More specifically, 1 mL of a HEPES buffer solution containing purified TBF (80mM HEPES-NaOH (pH 7.5) containing TBF at a final concentration of 0.5 mg/mL and ammonium iron sulfate at a final concentration of 2 mM) was prepared and left to stand at 4°C for 3 hours. After the buffer solution was left to stand, it was centrifuged (15,000 rpm, 5 minutes), and a supernatant containing the protein was collected. Then the supernatant was ultrafiltrated and concentrated using Amicon-Ultra-15 (NMWL.50000, Millipore, USA), and the buffer solution in the solution of the TBF multimer (Fe-TBF) with iron oxide nanoparticles encapsulated in its internal cavities was replaced with a Tris-HCl buffer solution (50 mM, pH 8.0) to obtain a protein solution.

Then the iron oxide nanoparticles synthesized using the TBF multimer were adsorbed on a silicon substrate. More specifically, a silicon substrate in which a 3 nm-thick silicon oxide film was mounted on a polysilicon wafer was cut into a 7 mm square piece. The cut silicon substrate was subjected to ultrasonic cleaning with acetone for 2 minutes and methanol for 2 minutes and finally washed five times with water. The washed silicon substrate was dried with nitrogen gas and subjected to UV-O₃ treatment for 10 minutes. 10 µL of the Fe-TBF solution (1.0 mg/mL Fe-TBF, 50mM Tris-HCl buffer solution, pH8.0) was placed on the treated silicon substrate and left to stand for 10 minutes to allow the Fe-TBF to adsorb on the silicon substrate. The Fe-TBF solution not adsorbed on the silicon substrate was removed by centrifugation. The silicon substrate was subjected to UV-O₃ treatment for 50 minutes to remove only the protein component in the Fe-TBF adsorbed on the silicon substrate, and a silicon substrate with iron oxide nanoparticles adsorbed on its surface was thereby obtained.

The silicon substrate with the iron oxide nanoparticles adsorbed on its surface was placed in a CVD furnace (manufactured by VIC International, Inc.) to form a CNT forest. More specifically, the silicon substrate was placed in a quartz tube in the CVD furnace and heated at 600°C to 650°C in a vacuum. The heated silicon substrate was left to stand in a hydrogen gas atmosphere (hydrogen flow rate: 200 sccm, reaction pressure: 1.5 kPa) for 5 minutes to activate the iron oxide nanoparticles on the silicon substrate. Then the substrate was left to stand in an acetylene gas/hydrogen gas mixture atmosphere (acetylene flow rate: 10 sccm, hydrogen flow rate: 200 sccm, reaction pressure: 1.5 kPa) for 20 minutes.

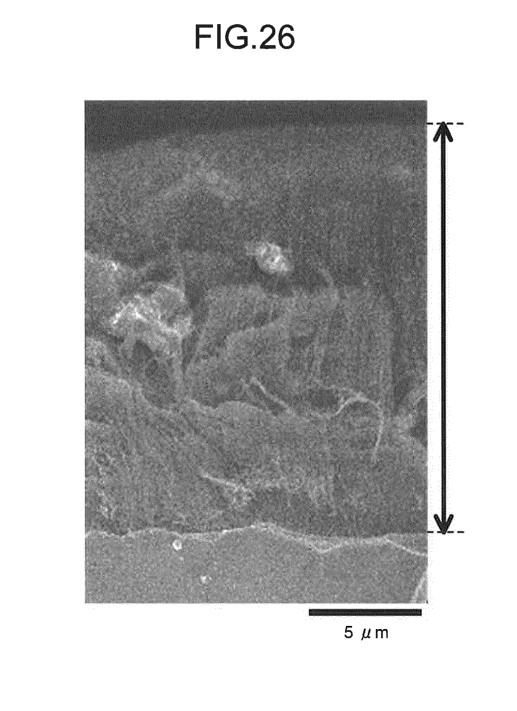

The silicon substrate treated in the CVD furnace was subjected to SEM analysis without staining, and a CNT forest comprising CNTs having a length of about 15 µm was found. The result is shown in FIG. 26. FIG. 26 is a photograph of the silicon substrate subjected to SEM analysis without staining. In FIG. 26, a region indicated by a two-directional arrow is a region in which the CNT forest is formed.

A CNT forest could also be synthesized under the conditions shown in Table 6 other than the above conditions using, as a catalyst, the iron oxide nanoparticles synthesized using the TBF multimer. A CNT forest could also be formed under the same conditions using a substrate not subjected to UV-O₃ treatment, namely from which the protein component in the Fe-TBF adsorbed on the silicon substrate had not been removed. Table 6 is a table showing examples of the conditions under which the formation (growth) of a CNT forest was found.

**Table 6**

| | | | | | | | | | |
|---|---|---|---|---|---|---|---|---|---|
| REACTION TEMPERATURE | °C | 700 | 600 | 700 | 600 | 600 | 600 | 650 | 600 |
| H₂ FLOW RATE | sccm | 100 | 25 | 25 | 25 | 10 | 200 | 200 | 200 |
| C₂H₂ FLOW RATE | sccm | 30 | 50 | 50 | 25 | 50 | 10 | 10 | 10 |
| REACTION PRESSURE | Pa | 1.5k | 200 | 200 | 200 | 200 | 1500 | 1500 | 400 |
| RATIO OF C₂H₂/H₂ | | 0.3 | 2 | 2 | 1 | 5 | 0.05 | 0.05 | 0.05 |
| PARTIAL PRESSURE OF C₂H₂ | Pa | 346.2 | 133.3 | 133.3 | 100.0 | 166.7 | 71.4 | 71.4 | 19.0 |

Under the conditions shown in Tables 7 and 8, CNTs could be synthesized on the silicon substrate although the CNTs were not in a forest form. Tables 7 and 8 are tables showing examples of the conditions under which CNTs were found to be synthesized. In each of the above cases, the silicon substrate with iron oxide nanoparticles adsorbed on its surface was heated to the temperature for CNT synthesis and then left to stand at the same pressure and hydrogen flow rate as those used for CNT synthesis for 5 minutes to activate the iron oxide nanoparticles adsorbed on the silicon substrate. Then the substrate was treated for 20 minutes under any of the conditions shown in Tables 7 and 8 to grow CNTs on the silicon substrate. The temperature was increased and decreased in a vacuum.

**Table 7**

| | | | | | | | |
|---|---|---|---|---|---|---|---|
| REACTION TEMPERATURE | °C | 500 | 500 | 550 | 550 | 650 | 700 |
| H₂ FLOW RATE | sccm | 25 | 200 | 200 | 100 | 200 | 200 |
| C₂H₂ FLOW RATE | sccm | 50 | 10 | 10 | 30 | 10 | 10 |
| REACTION PRESSURE | Pa | 200 | 1.5k | 1.5k | 1.5k | 400 | 400 |
| RATIO OF C₂H₂/H₂ | | 2 | 0.05 | 0.05 | 0.3 | 0.05 | 0.05 |
| PARTIAL PRESSURE OF C₂H₂ | Pa | 133.3 | 71.4 | 71.4 | 346.2 | 19.0 | 19.0 |

**Table 8**

| | | | | | | | |
|---|---|---|---|---|---|---|---|
| REACTION TEMPERATURE | °C | 700 | 750 | 800 | 900 | 800 | 900 |
| H₂ FLOW RATE | sccm | 200 | 100 | 25 | 25 | 100 | 25 |
| C₂H₂ FLOW RATE | sccm | 10 | 30 | 50 | 50 | 200 | 10 |
| REACTION PRESSURE | Pa | 1500 | 1.5k | 200 | 200 | 600 | 60 |
| RATIO OF C₂H₂/H₂ | | 0.05 | 0.3 | 2 | 2 | 2 | 0.4 |
| PARTIAL PRESSURE OF C₂H₂ | Pa | 71.4 | 346.2 | 1000.0 | 1000.0 | 400.0 | 17.1 |

### Example 23: Synthesis of complex of synthesized CNT and CDT

To obtain a forest-like CNT(SWNT)/TiO₂ electrode by processing the above-obtained CNT forest, CDT must be adsorbed on the CNTs comprised in the CNT forest. Therefore, the CNTs were separated from the silicon substrate having the CNT forest formed thereon. Then the detailed structure of the obtained CNTs was observed, and the formation of the complex of CNT and CDT was observed. More specifically, the silicon substrate treated at an acetylene flow rate of 10 sccm, a hydrogen flow rate of 200 sccm, a reaction pressure of 1.5 kPa, and a reaction temperature of 600°C and having the CNT forest formed thereon was placed in a 70% aqueous ethanol solution and subjected to sonication for 1 minute (1s ON/1s OFF, 20% Duty) to separate the CNTs from the silicon substrate. Next, formation of a complex with CDT was tried using the obtained CNT solution by a conventional method. More specifically, a potassium phosphate buffer solution containing CNTs and CDT [50mM potassium phosphate (pH 6.0) containing CDT at a final concentration of 0.1 mg/mL and CNTs at a final concentration of 0.1 mg/mL] was prepared. Ultrasonic pulses (200 W, Duty 20%) were applied to the potassium phosphate buffer solution containing CDT on ice using Digital Sonifier 450 (Branson, USA) for 1 second at three second intervals until the total application time reached 5 minutes.

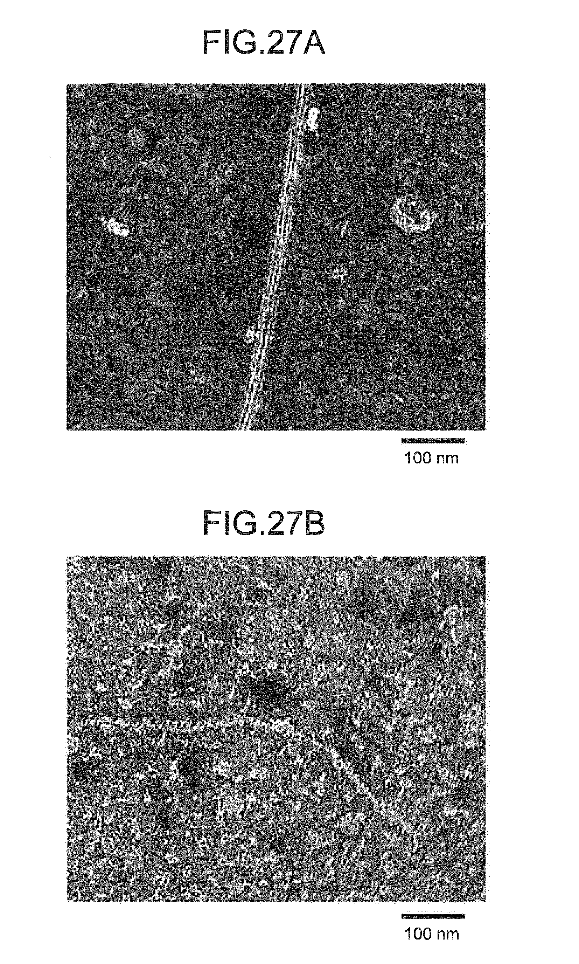

The results are shown in FIGs. 27A and 27B. FIGS. 27A and 27B are photographs of transmission electron microscopic images of the mixed solution of CDT and CNTs. The photographs were taken after the sample was stained with 3% phosphotungstic acid. The CNTs synthesized under the above conditions were found to comprise a mixture of thick CNTs shown in FIG. 27A and thin CNTs shown in FIG. 27B. It was observed that CDT could be bound to a thin CNT (see FIG. 27B). The column-like CNTs synthesized by the above-described method can form a complex with CDT. Therefore, it was suggested that a column-like CNT(SWNT)/TiO₂ complex can be synthesized using CDT from column-like CNTs, as in bulk-like CNTs.

### Example 24: Synthesis of thin CNTs

Since CDT bound to the synthesized thin CNTs was observed, formation of a CNT forest composed of thin CNTs was tried. A previous study has pointed out that the thickness of synthesized CNTs varies according to the size of iron nanoparticles used as a catalyst (M. Kumar and Y. Ando (2010) J. Nanoscience Nanotechnol., 10, 3739). Therefore, formation of a CNT forest was tried using CDT capable of encapsulating, in its internal cavities, iron oxide nanoparticles smaller than those for TBF. More specifically, CDT encapsulating iron oxide nanoparticles in its internal cavities and TBF encapsulating iron oxide nanoparticles in its internal cavities were mixed at 1:0, 1:1, 1:2, or 2:1 and suspended in a tris buffer solution (50 mM, pH 8.0) such that the total amount of the proteins was 1 mg/mL. Then a silicon substrate in which a 3 nm-thick silicon oxide film was mounted on a polysilicon wafer was cut into a 7 square piece. The cut silicon substrate was subjected to ultrasonic cleaning with acetone for 2 minutes and methanol for 2 minutes and finally washed five times with water. The washed silicon substrate was dried with nitrogen gas and subjected to UV-O₃ treatment for 10 minutes. 10 µL of a buffer solution containing CDT encapsulating iron oxide nanoparticles in its internal cavities and TBF encapsulating iron oxide nanoparticles in its internal cavities was placed on the treated silicon substrate and left to stand for 10 minutes to allow the proteins to adsorb on the silicon substrate. The protein solution not adsorbed on the silicon substrate was removed by centrifugation. Then the silicon substrate was subjected to UV-O₃ treatment for 50 minutes to remove only the protein components on the silicon substrate.

The silicon substrate with the iron oxide nanoparticles adsorbed on its surface was placed in a CVD furnace to form a CNT forest. More specifically, the silicon substrate was placed in a quartz tube in the CVD furnace and heated at 600°C in a vacuum. The heated substrate was left to stand in a hydrogen gas atmosphere (hydrogen flow rate: 200 sccm, reaction pressure: 1.5 kPa) for 5 minutes to activate the iron oxide nanoparticles on the substrate. Then the substrate was left to stand in an acetylene gas/hydrogen gas mixture atmosphere (acetylene flow rate: 10 sccm, hydrogen flow rate: 200 sccm, reaction pressure: 1.5 kPa) for 20 minutes.

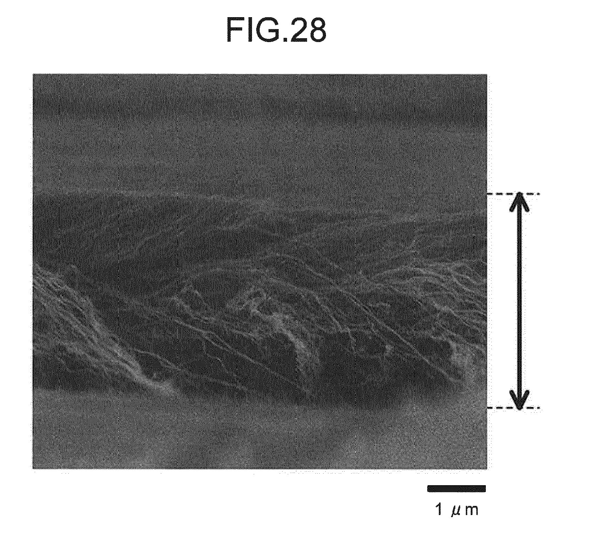

When a solution containing a mixture of CDT encapsulating iron oxide nanoparticles in its internal cavities and TBF encapsulating iron oxide nanoparticles in its internal cavities at 1:1, 1:2, or 2:1 was used, formation of a CNT forest was confirmed. The result is shown in FIG. 28. FIG. 28 is a photograph obtained by SEM analysis on CNTs synthesized using CDT and TBF at a ratio of 1:1. In FIG. 28, a region indicated by a two-directional arrow is a region in which the CNT forest is formed. Next, the substrate having the CNT forest formed thereon was immersed in a 70% aqueous ethanol solution and subjected to sonication for 1 minute (1s ON/1s OFF, 20% Duty) to separate the CNTs from the silicon substrate.

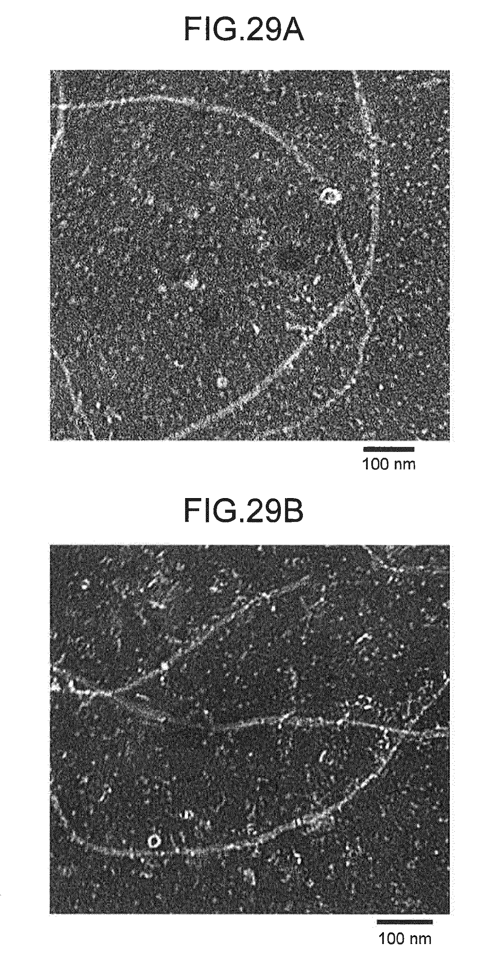

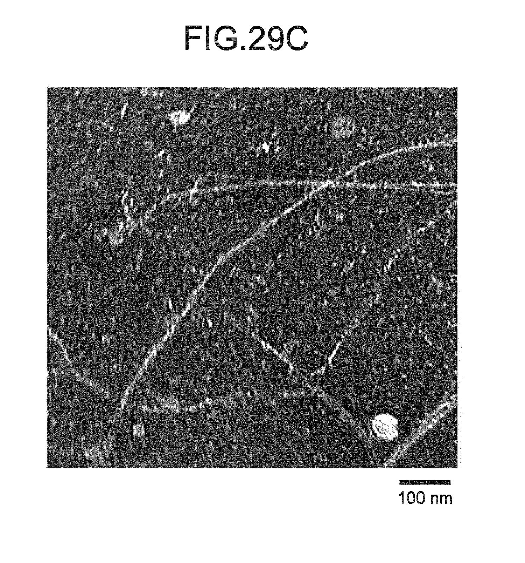

The thickness of the obtained CNTs was analyzed using a TEM. The results are shown in FIGs. 29A, 29B, and 29C. As shown in FIGs. 29A, 29B, and 29C, FIG. 29A is a photograph of CNTs synthesized using CDT and TBF at a ratio of 1:1. FIG. 29B is a photograph of CNTs synthesized using CDT and TBF at a ratio of 1:2. FIG. 29C is a photograph of CNTs synthesized using CDT and TBF at a ratio of 2:1.

It was found that all the CNT forests formed using the catalyst mixtures of CDT encapsulating iron oxide nanoparticles in its internal cavities and TBF encapsulating iron oxide nanoparticles in its internal cavities contain a large number of thin CNTs.

### Example 25: Binding of protein to CNTs

Whether or not CNHBP-Dps (CD) could bind to the CNTs synthesized on the silicon substrate using CDT and TBF at a ratio of 1:2 was examined. CNHBP-Dps has a structure in which a titanium-binding peptide TBP is removed from CNHBP-Dps-TBP and was prepared as follows.

BL21(DE3)/pET20-CD was cultured in 5 mL of LB medium (containing 100 mg/L of ampicillin) at 37°C. Eighteen hours after the start of cultivation, the obtained culture solution was inoculated into 3 L of new LB medium (containing 100 mg/L of ampicillin) and cultured with shaking using BMS-10/05 (ABLE, Japan) at 37°C for 24 hours. The obtained microbial cells were collected by centrifugation (5,000 rpm, 5 minutes) and stored at -80°C. Half (6 g) the amount of the cryopreserved microbial cells was suspended in 40 mL of a 50mM Tris-HCl buffer solution (pH 8.0). Then ultrasonic pulses (200 W, Duty 45%) were applied to the suspension at one-second intervals for 12 minutes using Digital Sonifier 450 (Branson, USA) to disrupt the microbial cells. The solution containing the disrupted microbial cells was centrifuged at 15,000 rpm for 15 minutes (JA-20, Beckman Coulter, USA), and a supernatant fraction was collected. The collected supernatant fraction was heated at 60°C for 20 minutes and rapidly cooled on ice after heating. The cooled supernatant fraction was centrifuged (JA-20) at 17,000 rpm for 10 minutes, and a supernatant (about 20 mL) was again collected. The supernatant was sterilized using a disc filter (Millex GP, 0.22 µm, Millipore, USA). Then the sterilized supernatant was ultrafiltrated and concentrated using icon-Ultra-15 (NMWL. 50000, Millipore, USA) until the amount of the solution became 10 mL to thereby obtain a protein solution. Then NaCl was added to the protein solution at a final concentration of 0.5 M, and the resultant mixture was centrifuged (JA-20) at 6,000 rpm for 5 minutes. Then the supernatant was discarded, and the precipitate was suspended in a 50mM Tris-HCl buffer solution (pH 8.0). This procedure was repeated three times to purify CD.

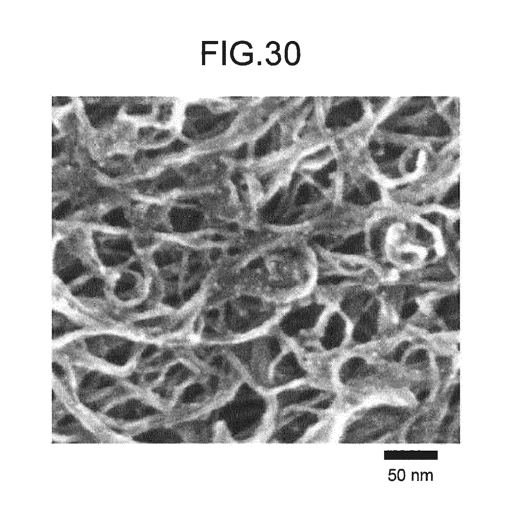

Next, to encapsulate metal particles in the CD, 1 mL of a HEPES buffer solution containing the CD (80mM HEPES-NaOH (pH 7.5) containing CDT at a final concentration of 0.5 mg/mL and ammonium iron sulfate at a final concentration of 1mM) was prepared and left to stand at 4°C for 3 hours. After the buffer solution was left to stand, it was centrifuged (15,000 rpm, 5 minutes), and a supernatant was collected to obtain a solution containing CD encapsulating iron oxide nanoparticles in its internal cavities. The obtained solution was ultrafiltrated and concentrated using icon-Ultra-15 (NMWL.50000, Millipore, USA), and the buffer solution in the solution of the CD multimer (Fe-CD) having iron oxide nanoparticles in its internal cavities was replaced with water to thereby obtain a protein solution. To form a complex of the Fe-CD and the CNT synthesized on the silicon substrate, 10 µL of the aqueous solution containing the Fe-CD (30% by volume, containing ethanol) was added to the CNT synthesized on the silicon substrate, and the resultant substrate was left to stand at room temperature for 10 minutes. Then the substrate was washed with water and dried in a desiccator. The resultant substrate was subjected to SEM analysis. The result is shown in FIG. 30. FIG. 30 is a photograph obtained by SEM analysis on the formed complex of CNT and Fe-CD.

As shown in FIG. 30, when the CDT and TBF encapsulating iron oxide nanoparticles were mixed at a ratio of 1:2, a complex of the formed CNT and Fe-CD was observed. A protein cannot be observed directly by SEM analysis. However, when a protein encapsulating metal nanoparticles such as iron nanoparticles is used, the positions of the protein can be indirectly observed by observing the metal nanoparticles inside the protein. Particularly, it is known that, with the Fe-CD used in the above case, the small nanoparticles having a diameter of about 5 nm are observed as white dots.

This suggests that the CNT-binding peptides used for CD and CDT can recognize the CNT synthesized in the above steps. This also suggests that metal nanoparticles be allowed to adhere to CNT using a protein.

### EXPLANATIONS OF LETTERS OR NVMERALS

- 10: POROUS STRUCTURE BODY
- 20: FIRST TARGET MATERIAL
- 30: AGGREGATE BODY
- 32: FIRST PORE
- 34: SECOND PORE
- 36: METAL PARTICLE
- 38: THIRD PORE
- 40: SENSITIZING DYE
- 52: FIRST SUBSTRATE
- 54: SECOND SUBSTRATE
- 62: TRANSPARENT ELECTRODE
- 64: COUNTER ELECTRODE
- 70: PHOTOELECTRIC CONVERSION LAYER
- 80: ELECTROLYTE
- 90: SEALING PORTION
- 100: DYE-SENSITIZED SOLAR CELL

## Claims

1. A porous structure body comprising: a first target material; and an aggregate body formed by aggregation of a second target material, the aggregate body adhering to the first target material and being located so as to surround the first target material, wherein
the aggregate body has a plurality of first pores unevenly distributed near the first target material in the aggregate body and a plurality of second pores scattered over the aggregate body.

2. The porous structure body according to claim 1, wherein the first target material is a carbon material selected from the group consisting of carbon nanotubes, carbon nanohorns, graphene sheets, fullerenes, and graphite.

3. The porous structure body according to claim 1 or 2, wherein the second target material is titanium oxide or zinc oxide.

4. The porous structure body according to any one of claims 1 to 3, further comprising metal particles put in the first pores and different from the second target material.

5. The porous structure body according to claim 4, wherein the metal particles are nanoparticles of a metal selected from the group consisting of iron oxide, nickel, cobalt, manganese, phosphorus, uranium, beryllium, aluminum, cadmium sulfide, cadmium selenide, palladium, chromium, copper, silver, a gadolium complex, platinum cobalt, silicon oxide, cobalt oxide, indium oxide, platinum, gold, gold sulfide, zinc selenide, and cadmium selenide.

6. The porous structure body according to claim 5, wherein the metal particles are iron oxide nanoparticles.

7. The porous structure body according to any one of claims 1 to 5, wherein the first pores have a diameter within a range of 5 nm to 15 nm.

8. The porous structure body according to any one of claims 1 to 7, wherein distances to each the first pore from a surface of the first target material are identical and within a range of 1 nm to 500 nm.

9. An electronic device comprising, as a functional material, the porous structure body according to any one of claims 1 to 8.

10. The electronic device according to claim 9, wherein the electronic device is a solar cell.

11. The electronic device according to claim 10, wherein the solar cell comprises the porous structure body as a material for an electrode or as a functional material placed on the electrode.

12. The electronic device according to claim 11, wherein the electronic device is a dye-sensitized solar cell.

13. A method for producing a porous structure body, comprising the steps of:
preparing a complex in which a first target material and a second target material or a precursor of the second target material are bound to a multimer of a fusion protein, the complex being prepared by binding the multimer of the fusion protein to the first target material and binding the second target material or the precursor of the second target material to the multimer of the fusion protein, wherein the multimer of the fusion protein has an internal cavity and is formed from the fusion protein that contains a first peptide portion capable of binding to the first target material, a second peptide portion capable of binding to the second target material, and a polypeptide portion capable of forming a multimer having an internal cavity; and
forming an aggregate body by burning the complex to consume the multimer of the fusion protein, the aggregate body adhering to the first target material and being located so as to surround the first target material, the aggregate body having a plurality of first pores unevenly distributed near the first target material and a plurality of second pores scattered over the aggregate body.

14. The method for producing the porous structure body according to claim 13,
wherein the step of preparing the complex is the step of preparing the complex in which the first target material and the precursor of the second target material are bound to the multimer of the fusion protein, and the step of forming the aggregate body is the step of burning the complex to consume the multimer of the fusion protein and to convert the precursor of the second target material to the second target material, thereby forming the aggregate body.

15. The method for producing the porous structure body according to claim 14, wherein the step of preparing the complex is the step of binding the precursor of the second target material to the multimer of the fusion protein and depositing the second target material around the first target material.

16. The method for producing the porous structure body according to any one of claims 13 to 15, wherein the first target material is a carbon material selected from the group consisting of carbon nanotubes, carbon nanohorns, graphene sheets, fullerenes, and graphite.

17. The method for producing the porous structure body according to any one of claims 13 to 16, wherein the second target material is titanium oxide or zinc oxide.

18. The method for producing the porous structure body according to any one of claims 13 to 17,
wherein the multimer of the fusion protein further comprises metal particles different from the first target material, each of the metal particles being encapsulated in the internal cavity thereof, and the aggregate body formed further comprises the metal particles in the first pores.

19. The method for producing a porous structure body according to claim 18, wherein the metal particles are nanoparticles of a metal selected from the group consisting of iron oxide, nickel, cobalt, manganese, phosphorus, uranium, beryllium, aluminum, cadmium sulfide, cadmium selenide, palladium, chromium, copper, silver, a gadolium complex, platinum cobalt, silicon oxide, cobalt oxide, indium oxide, platinum, gold, gold sulfide, zinc selenide, and cadmium selenide.

20. The method for producing the porous structure body according to claim 19, wherein the metal particles are iron oxide nanoparticles.

21. A method for producing an electronic device, comprising the step of forming a functional structure using the porous structure body according to any one of claims 1 to 8 as a functional material.

22. A method for producing a dye-sensitized solar cell, comprising the steps of:
preparing a substrate comprising a transparent electrode;
obtaining a complex by binding a multimer of a fusion protein to a first target material, wherein the multimer of the fusion protein has an internal cavity and is formed from the fusion protein that contains a first peptide portion capable of binding to the first target material, a second peptide portion capable of binding to the second target material, and a polypeptide portion capable of forming a multimer having an internal cavity, thereby forming a combination body and binding the combination body to a second target material or a precursor of the second target material;
placing a material comprising the complex on the transparent electrode;
burning the material on the transparent electrode to consume the multimer of the fusion protein, thereby forming a structure having a porous structure body on the transparent electrode, the porous structure body comprising an aggregate body that comprises the second target material, adheres to the first target material, and is located so as to surround the first target material, the aggregate body having a plurality of first pores unevenly distributed near the first target material and a plurality of second pores scattered over the aggregate body;
supporting a sensitizing dye by the porous structure body, thereby forming a photoelectrode; and
pouring an electrolyte and sealing the photoelectrode and a counter electrode.

23. The method for producing the dye-sensitized solar cell according to claim 22, wherein the structure comprising the porous structure body is formed such that a final concentration of the complex is less than 1% by weight.

24. The method for producing the dye-sensitized solar cell according to claim 23, wherein the structure comprising the porous structure body is formed such that the final concentration of the complex is within a range of 0.06% by weight to 0.5% by weight.

25. A method for producing a dye-sensitized solar cell, comprising the steps of:
preparing a substrate comprising a transparent electrode;
forming a porous structure body comprising an aggregate body by binding a multimer of a fusion protein, wherein the multimer of the fusion protein has an internal cavity and is formed from the fusion protein that contains a first peptide portion capable of binding to the first target material, a second peptide portion capable of binding to the second target material, and a polypeptide portion capable of forming a multimer having an internal cavity, to a first target material, thereby forming a combination body, binding the combination body to a second target material or a precursor of the second target material, thereby forming a complex body, and burning the complex body to consume the multimer of the fusion protein, the aggregate body comprising the second target material, adhering to the first target material, and being located so as to surround the first target material, the aggregate body having a plurality of first pores unevenly distributed near the first target material and a plurality of second pores scattered over the aggregate body;
placing a material comprising the porous structure body and the second target material or the precursor of the second target material on the transparent electrode;
heating the material on the transparent electrode, thereby forming a structure body comprising the porous structure body by the transparent electrode;
supporting a sensitizing dye by the porous structure body, thereby forming a photoelectrode; and
pouring an electrolyte and sealing the photoelectrode and a counter electrode.

26. The method for producing the dye-sensitized solar cell according to any one of claims 22 to 25, wherein the first target material is a carbon material selected from the group consisting of carbon nanotubes, carbon nanohorns, graphene sheets, fullerenes, and graphite.

27. A method for producing a dye-sensitized solar cell, comprising the steps of:
preparing a substrate comprising a transparent electrode;
arranging at least one carbon nanotube used as a first target material on the transparent electrode so as to extend in a direction of a thickness of the substrate;
forming a complex by binding a multimer of a fusion protein to the carbon nanotube, wherein the multimer of the fusion protein has an internal cavity and is formed from the fusion protein that contains a first peptide portion capable of binding to the carbon nanotube, a second peptide portion capable of binding to a precursor of the second target material, and a polypeptide portion capable of forming a multimer having an internal cavity, thereby forming a combination body and binding the combination body to a second target material or a precursor of the second target material;
burning the mixture to consume the multimer of the fusion protein, whereby a structure body having a porous structure body comprising an aggregate body is formed on the transparent electrode, the aggregate body comprising the second target material, adhering to the carbon nanotube, and being located so as to surround the carbon nanotube, the aggregate body having a plurality of first pores unevenly distributed near the carbon nanotube and a plurality of second pores scattered over the aggregate body;
supporting a sensitizing dye by the porous structure body, thereby forming a photoelectrode; and
pouring an electrolyte and sealing the photoelectrode and a counter electrode.

28. The method for producing a dye-sensitized solar cell according to claim 27, wherein the step of arranging the at least one carbon nanotube used as the first target material on the transparent electrode so as to extend in the direction of the thickness of the substrate comprises the step of adsorbing an inorganic material on a substrate, the step of growing a carbon nanotube from the inorganic material as a seed, thereby obtaining a carbon nanotube-arranged substrate, and the step of transferring the carbon nanotube on the carbon nanotube-arranged substrate to the transparent electrode.

29. The method for producing the dye-sensitized solar cell according to claim 28, wherein the step of adsorbing the inorganic material on the substrate is the step of adsorbing and arranging at least two types of inorganic material-encapsulating proteins with different sizes on the substrate.

30. The method for producing the dye-sensitized solar cell according to claim 29, wherein the step of adsorbing the inorganic material on the substrate is the step of adsorbing and arranging the inorganic material-encapsulating proteins on a silicon oxide film provided on the substrate used.

31. The method for producing the dye-sensitized solar cell according to claim 29 or 30, wherein the inorganic material-encapsulating proteins comprise at least one type of protein selected from ferritin protein, Dps protein, CDT protein, and modified proteins thereof.

32. The method for producing the dye-sensitized solar cell according to any one of claims 22 to 31, wherein the second target material is titanium oxide or zinc oxide.

33. A dye-sensitized solar cell obtainable by the method for producing according to any one of claims 22 to 32.

34. A dye-sensitized solar cell comprising:
a substrate comprising a transparent electrode;
a photoelectric conversion layer provided on the transparent electrode, the photoelectric conversion layer comprising a porous structure body, the porous structure body supporting a sensitizing dye, the porous structure body comprising a first target material and an aggregate body comprising a second target material, the aggregate body adhering to the first target material and being located so as to surround the first target material, the aggregate body having a plurality of first pores unevenly distributed near the first target material and a plurality of second pores scattered over the aggregate body; and
a sealing portion that seals the dye-sensitized solar cell such that the photoelectrode faces a counter electrode and the photoelectrode and the counter electrode are in contact with an electrolyte.

35. A dye-sensitized solar cell comprising:
a substrate comprising a transparent electrode;
a photoelectric conversion layer provided on the transparent electrode, the photoelectric conversion layer comprising a porous structure body, the porous structure body supporting a sensitizing dye, the porous structure body comprising at least one carbon nanotube used as a first target material and an aggregate body comprising a second target material, the at least one carbon nanotube being arranged to extend in a direction of a thickness of the substrate, the aggregate body adhering to the carbon nanotube and being located so as to surround the carbon nanotube, the aggregate body having a plurality of first pores unevenly distributed near the carbon nanotube and a plurality of second pores scattered over the aggregate body; and
a sealing portion that seals the dye-sensitized solar cell such that the photoelectrode faces a counter electrode and the photoelectrode and the counter electrode are in contact with an electrolyte.

36. The dye-sensitized solar cell according to claim 33, wherein the first target material is a carbon material selected from the group consisting of carbon nanotubes, carbon nanohorns, graphene sheets, fullerenes, and graphite.

37. The dye-sensitized solar cell according to any one of claims 34 to 36, wherein the second target material is titanium oxide or zinc oxide.

38. A method for producing a carbon nanotube-arranged substrate in which at least one carbon nanotube is arranged to extend in a thickness direction, the method comprising the steps of:
adsorbing and arranging at least two types of inorganic material-encapsulating proteins with different sizes on the substrate; and
growing a carbon nanotube from an inorganic material encapsulated in the inorganic material-encapsulating proteins as a seed.

39. The method for producing a carbon nanotube-arranged substrate according to claim 38, wherein the step of adsorbing and arranging the at least two types of inorganic material-encapsulating proteins with different sizes on the substrate is performed by adsorbing the inorganic material-encapsulating proteins on a silicon oxide film provided on the substrate used.

40. The method for producing the carbon nanotube-arranged substrate according to claim 38 or 39, wherein the inorganic material-encapsulating proteins comprise at least one type of protein selected from ferritin protein, Dps protein, CDT protein, and modified proteins thereof.


### Sequence Listing

SEQUENCE LISTING

<110>  AJINOMOTO CO., INC.

<120>  Porous composite and method for manufacturing thereof

<130>  PAMA-24547

<150>  JP 2011-278816
<151>  2011-12-20

<150>  JP 2011-173229
<151>  2011-08-08

<160>  33

<170>  PatentIn version 3.5

<210>  1
<211>  531
<212>  DNA
<213>  Artificial Sequence

<220>
<223>  Nucleotide sequence encoding an amino acid sequence of fusion
       protein


<220>
<221>  CDS
<222>  (1)..(531)

<400>  1
atg gac tac ttc tct tct ccg tac tac gaa cag ctg ttt atg aaa aca        48
Met Asp Tyr Phe Ser Ser Pro Tyr Tyr Glu Gln Leu Phe Met Lys Thr
1               5                   10                  15

atc aac tca gta gac aca aag gaa ttt ttg aat cat caa gta gcg aat        96
Ile Asn Ser Val Asp Thr Lys Glu Phe Leu Asn His Gln Val Ala Asn
            20                  25                  30

tta aac gta ttc aca gta aaa att cat caa att cat tgg tat atg aga       144
Leu Asn Val Phe Thr Val Lys Ile His Gln Ile His Trp Tyr Met Arg
        35                  40                  45

ggc cac aac ttc ttc act tta cat gaa aaa atg gat gat tta tat agc       192
Gly His Asn Phe Phe Thr Leu His Glu Lys Met Asp Asp Leu Tyr Ser
    50                  55                  60

gaa ttc ggt gaa caa atg gat gaa gta gca gaa cgt tta ctt gcc att       240
Glu Phe Gly Glu Gln Met Asp Glu Val Ala Glu Arg Leu Leu Ala Ile
65                  70                  75                  80

ggt gga agc cca ttc tcg act tta aaa gag ttt tta gaa aat gcg agt       288
Gly Gly Ser Pro Phe Ser Thr Leu Lys Glu Phe Leu Glu Asn Ala Ser
                85                  90                  95

gta gaa gaa gct cct tat aca aaa cct aaa act atg gat caa tta atg       336
Val Glu Glu Ala Pro Tyr Thr Lys Pro Lys Thr Met Asp Gln Leu Met
            100                 105                 110

gaa gac tta gtt ggt aca tta gaa tta ctt aga gac gaa tat aaa caa       384
Glu Asp Leu Val Gly Thr Leu Glu Leu Leu Arg Asp Glu Tyr Lys Gln
        115                 120                 125

ggc att gag cta act gac aaa gaa ggc gac gat gta aca aac gat atg     432
Gly Ile Glu Leu Thr Asp Lys Glu Gly Asp Asp Val Thr Asn Asp Met
    130                 135                 140

cta att gca ttt aaa gct agc att gac aaa cat atc tgg atg ttc aaa     480
Leu Ile Ala Phe Lys Ala Ser Ile Asp Lys His Ile Trp Met Phe Lys
145                 150                 155                 160

gca ttc ctt gga aaa gct cca tta gaa atg cgc aaa ctt ccg gat gcg     528
Ala Phe Leu Gly Lys Ala Pro Leu Glu Met Arg Lys Leu Pro Asp Ala
                165                 170                 175

taa                                                                 531


<210>  2
<211>  176
<212>  PRT
<213>  Artificial Sequence

<220>
<223>  Synthetic Construct

<400>  2

Met Asp Tyr Phe Ser Ser Pro Tyr Tyr Glu Gln Leu Phe Met Lys Thr
1               5                   10                  15


Ile Asn Ser Val Asp Thr Lys Glu Phe Leu Asn His Gln Val Ala Asn
            20                  25                  30


Leu Asn Val Phe Thr Val Lys Ile His Gln Ile His Trp Tyr Met Arg
        35                  40                  45


Gly His Asn Phe Phe Thr Leu His Glu Lys Met Asp Asp Leu Tyr Ser
    50                  55                  60


Glu Phe Gly Glu Gln Met Asp Glu Val Ala Glu Arg Leu Leu Ala Ile
65                  70                  75                  80


Gly Gly Ser Pro Phe Ser Thr Leu Lys Glu Phe Leu Glu Asn Ala Ser
                85                  90                  95


Val Glu Glu Ala Pro Tyr Thr Lys Pro Lys Thr Met Asp Gln Leu Met
            100                 105                 110


Glu Asp Leu Val Gly Thr Leu Glu Leu Leu Arg Asp Glu Tyr Lys Gln
        115                 120                 125


Gly Ile Glu Leu Thr Asp Lys Glu Gly Asp Asp Val Thr Asn Asp Met
    130                 135                 140


Leu Ile Ala Phe Lys Ala Ser Ile Asp Lys His Ile Trp Met Phe Lys
145                 150                 155                 160

Ala Phe Leu Gly Lys Ala Pro Leu Glu Met Arg Lys Leu Pro Asp Ala
                165                 170                 175

<210>  3
<211>  471
<212>  DNA
<213>  Listeria innocua

<220>
<221>  CDS
<222>  (1)..(471)

<400>  3
atg aaa aca atc aac tca gta gac aca aag gaa ttt ttg aat cat caa      48
Met Lys Thr Ile Asn Ser Val Asp Thr Lys Glu Phe Leu Asn His Gln
1               5                   10                  15

gta gcg aat tta aac gta ttc aca gta aaa att cat caa att cat tgg      96
Val Ala Asn Leu Asn Val Phe Thr Val Lys Ile His Gln Ile His Trp
            20                  25                  30

tat atg aga ggc cac aac ttc ttc act tta cat gaa aaa atg gat gat      144
Tyr Met Arg Gly His Asn Phe Phe Thr Leu His Glu Lys Met Asp Asp
        35                  40                  45

tta tat agc gaa ttc ggt gaa caa atg gat gaa gta gca gaa cgt tta      192
Leu Tyr Ser Glu Phe Gly Glu Gln Met Asp Glu Val Ala Glu Arg Leu
    50                  55                  60

ctt gcc att ggt gga agc cca ttc tcg act tta aaa gag ttt tta gaa      240
Leu Ala Ile Gly Gly Ser Pro Phe Ser Thr Leu Lys Glu Phe Leu Glu
65                  70                  75                  80

aat gcg agt gta gaa gaa gct cct tat aca aaa cct aaa act atg gat      288
Asn Ala Ser Val Glu Glu Ala Pro Tyr Thr Lys Pro Lys Thr Met Asp
                85                  90                  95

caa tta atg gaa gac tta gtt ggt aca tta gaa tta ctt aga gac gaa      336
Gln Leu Met Glu Asp Leu Val Gly Thr Leu Glu Leu Leu Arg Asp Glu
            100                 105                 110

tat aaa caa ggc att gag cta act gac aaa gaa ggc gac gat gta aca      384
Tyr Lys Gln Gly Ile Glu Leu Thr Asp Lys Glu Gly Asp Asp Val Thr
        115                 120                 125

aac gat atg cta att gca ttt aaa gct agc att gac aaa cat atc tgg      432
Asn Asp Met Leu Ile Ala Phe Lys Ala Ser Ile Asp Lys His Ile Trp
    130                 135                 140

atg ttc aaa gca ttc ctt gga aaa gct cca tta gaa taa                  471
Met Phe Lys Ala Phe Leu Gly Lys Ala Pro Leu Glu
145                 150                 155

<210>  4
<211>  156
<212>  PRT
<213>  Listeria innocua

<400>  4

Met Lys Thr Ile Asn Ser Val Asp Thr Lys Glu Phe Leu Asn His Gln
1               5                   10                  15

Val Ala Asn Leu Asn Val Phe Thr Val Lys Ile His Gln Ile His Trp
            20                  25                  30

Tyr Met Arg Gly His Asn Phe Phe Thr Leu His Glu Lys Met Asp Asp
        35                  40                  45

Leu Tyr Ser Glu Phe Gly Glu Gln Met Asp Glu Val Ala Glu Arg Leu
    50                  55                  60

Leu Ala Ile Gly Gly Ser Pro Phe Ser Thr Leu Lys Glu Phe Leu Glu
65                  70                  75                  80

Asn Ala Ser Val Glu Glu Ala Pro Tyr Thr Lys Pro Lys Thr Met Asp
                85                  90                  95

Gln Leu Met Glu Asp Leu Val Gly Thr Leu Glu Leu Leu Arg Asp Glu
            100                 105                 110

Tyr Lys Gln Gly Ile Glu Leu Thr Asp Lys Glu Gly Asp Asp Val Thr
        115                 120                 125

Asn Asp Met Leu Ile Ala Phe Lys Ala Ser Ile Asp Lys His Ile Trp
    130                 135                 140

Met Phe Lys Ala Phe Leu Gly Lys Ala Pro Leu Glu
145                 150                 155

<210>  5
<211>  36
<212>  DNA
<213>  Artificial Sequence

<220>
<223>  Nucleotide sequence encoding the amino acid sequence of carbon
       nanohorn binding peptide (CNHBP)

<220>
<221>  CDS
<222>  (1)..(36)

<400>  5
gac tac ttc tct tct ccg tac tac gaa cag ctg ttt                    36
Asp Tyr Phe Ser Ser Pro Tyr Tyr Glu Gln Leu Phe
1               5                   10

<210>  6
<211>  12
<212>  PRT
<213>  Artificial Sequence

<220>
<223>  Synthetic Construct

<400>  6

Asp Tyr Phe Ser Ser Pro Tyr Tyr Glu Gln Leu Phe
1               5                   10


<210>  7
<211>  18
<212>  DNA
<213>  Artificial Sequence

<220>
<223>  Nucleotide sequence encoding the amino acid sequence of titanium
       binding peptide (TBP)


<220>
<221>  CDS
<222>  (1)..(18)

<400>  7
cgc aaa ctt ccg gat gcg                                                18
Arg Lys Leu Pro Asp Ala
1               5


<210>  8
<211>  6
<212>  PRT
<213>  Artificial Sequence

<220>
<223>  Synthetic Construct

<400>  8

Arg Lys Leu Pro Asp Ala
1               5


<210>  9
<211>  39
<212>  DNA
<213>  Artificial Sequence

<220>
<223>  Synthetic DNA

<400>  9
tatggactac ttctcttctc cgtactacga acagctgtt                             39


<210>  10
<211>  39
<212>  DNA
<213>  Artificial Sequence

<220>
<223>  Synthetic DNA

<400>  10
taaacagctg ttcgtagtac ggagaagaga agtagtcca                             39

<210>  11
<211>  26
<212>  DNA
<213>  Artificial Sequence

<220>
<223>  Primer

<400>  11
tttggatccg aattcgagct ccgtcg                                            26


<210>  12
<211>  50
<212>  DNA
<213>  Artificial Sequence

<220>
<223>  Primer

<400>  12
tttggatcct tacgcatccg gaagtttgcg catttctaat ggagcttttc                  50


<210>  13
<211>  12
<212>  PRT
<213>  Artificial Sequence

<220>
<223>  Peptide capable of binding to a carbon material

<400>  13

His Ser Ser Tyr Trp Tyr Ala Phe Asn Asn Lys Thr
1               5                   10


<210>  14
<211>  7
<212>  PRT
<213>  Artificial Sequence

<220>
<223>  Peptide capable of binding to a carbon material

<400>  14

Tyr Asp Pro Phe His Ile Ile
1               5


<210>  15
<211>  19
<212>  PRT
<213>  Artificial Sequence

<220>
<223>  Peptide capable of binding to a metal material

<400>  15

Ser Ser Lys Lys Ser Gly Ser Tyr Ser Gly Ser Lys Gly Ser Lys Arg
1               5                   10                  15

Arg Ile Leu

<210> 16
<211> 12
<212> PRT
<213> Artificial Sequence

<220>
<223> Peptide capable of binding to a metal material

<400> 16

Arg Lys Leu Pro Asp Ala Pro Gly Met His Thr Trp
1               5                   10

<210> 17
<211> 6
<212> PRT
<213> Artificial Sequence

<220>
<223> Peptide capable of binding to a metal material

<400> 17

Arg Ala Leu Pro Asp Ala
1               5

<210> 18
<211> 12
<212> PRT
<213> Artificial Sequence

<220>
<223> Peptide capable of binding to a silicon material

<400> 18

Met Ser Pro His Pro His Pro Arg His His His Thr
1               5                   10

<210> 19
<211> 9
<212> PRT
<213> Artificial Sequence

<220>
<223> Peptide capable of binding to a silicon material

<400> 19

Arg Arg Arg Leu Ser Cys Arg Leu Leu
1               5

<210> 20
<211> 12
<212> PRT
<213> Artificial Sequence

<220>
<223>  Peptide capable of binding to a silicon material

<400>  20

Lys Pro Ser His His His His His Thr Gly Ala Asn
1               5                   10


<210>  21
<211>  35
<212>  PRT
<213>  Artificial Sequence

<220>
<223>  Fusion peptide (P1R5 peptide)

<400>  21

Ser Ser Lys Lys Ser Gly Ser Tyr Ser Gly Ser Lys Gly Ser Lys Arg
1               5                   10                  15


Arg Ile Leu Gly Gly Gly Gly His Ser Ser Tyr Trp Tyr Ala Phe Asn
            20                  25                  30


Asn Lys Thr
        35


<210>  22
<211>  33
<212>  DNA
<213>  Artificial Sequence

<220>
<223>  Primer

<400>  22
tttcatatgt atatctcctt cttaaagtta aac                                  33


<210>  23
<211>  31
<212>  DNA
<213>  Artificial Sequence

<220>
<223>  Primer

<400>  23
tttcatatga tgaaaacaat caactcagta g                                    31


<210>  24
<211>  63
<212>  DNA
<213>  Artificial Sequence

<220>
<223>  Primer

<400>  24

tttcatatgg actacttctc ttctccgtac tacgaacagc tgtttatggc aaactacaca      60

gtc                                                                   63


<210>  25
<211>  51
<212>  DNA
<213>  Artificial Sequence

<220>
<223>  Primer

<400>  25
tttgaattct tacgcatccg gaagtttgcg catctcttgg atgtttccgt c              51


<210>  26
<211>  558
<212>  DNA
<213>  Artificial Sequence

<220>
<223>  Nucleotide sequence encoding an amino acid sequence of fusion
       protein


<220>
<221>  CDS
<222>  (1)..(558)

<400>  26
atg gac tac ttc tct tct ccg tac tac gaa cag ctg ttt atg gca aac      48
Met Asp Tyr Phe Ser Ser Pro Tyr Tyr Glu Gln Leu Phe Met Ala Asn
1               5                   10                  15

tac aca gtc cct gga atc aac gag aat gac gca aag cag ctt att gat      96
Tyr Thr Val Pro Gly Ile Asn Glu Asn Asp Ala Lys Gln Leu Ile Asp
            20                  25                  30

gga ctg cag gag cgt ctc acc gac tac aac gat ctt cac ctc atc ttg      144
Gly Leu Gln Glu Arg Leu Thr Asp Tyr Asn Asp Leu His Leu Ile Leu
        35                  40                  45

aag cac gtg cac tgg aac gtc act ggc ccc aac ttc att gct gtt cac      192
Lys His Val His Trp Asn Val Thr Gly Pro Asn Phe Ile Ala Val His
    50                  55                  60

gaa atg ctc gac cca cag gtt gac ctt gtt cgt ggc tat gct gac gaa      240
Glu Met Leu Asp Pro Gln Val Asp Leu Val Arg Gly Tyr Ala Asp Glu
65                  70                  75                  80

gtt gca gag cgc att tcc acc ctc gga ggc gca cca gtt gga acc cca      288
Val Ala Glu Arg Ile Ser Thr Leu Gly Gly Ala Pro Val Gly Thr Pro
                85                  90                  95

gaa ggc cac gtt gct gac cgc acc cca ctg caa tat gag cgc aat gcc      336
Glu Gly His Val Ala Asp Arg Thr Pro Leu Gln Tyr Glu Arg Asn Ala
            100                 105                 110

gga aat gtc caa gca cac ctc act gac ctc aat cgc gtg tac acc caa      384
Gly Asn Val Gln Ala His Leu Thr Asp Leu Asn Arg Val Tyr Thr Gln
        115                 120                 125

gtg ctg acc gga gtt cgc gag tcc atg gca tca gcc ggc cca gtg gat      432

Val Leu Thr Gly Val Arg Glu Ser Met Ala Ser Ala Gly Pro Val Asp
    130                 135                 140

cca gta act gaa gac atc tac atc agc cag gcc gcg gag ctg gag aaa       480
Pro Val Thr Glu Asp Ile Tyr Ile Ser Gln Ala Ala Glu Leu Glu Lys
145                 150                 155                 160

ttc cag tgg ttc atc cgc gca cac att gtt gat gta gac gga aac atc       528
Phe Gln Trp Phe Ile Arg Ala His Ile Val Asp Val Asp Gly Asn Ile
                165                 170                 175

caa gag atg cgc aaa ctt ccg gat gcg taa                               558
Gln Glu Met Arg Lys Leu Pro Asp Ala
            180                 185

<210>  27
<211>  185
<212>  PRT
<213>  Artificial Sequence

<220>
<223>  Synthetic Construct

<400>  27

Met Asp Tyr Phe Ser Ser Pro Tyr Tyr Glu Gln Leu Phe Met Ala Asn
1               5                   10                  15

Tyr Thr Val Pro Gly Ile Asn Glu Asn Asp Ala Lys Gln Leu Ile Asp
            20                  25                  30

Gly Leu Gln Glu Arg Leu Thr Asp Tyr Asn Asp Leu His Leu Ile Leu
        35                  40                  45

Lys His Val His Trp Asn Val Thr Gly Pro Asn Phe Ile Ala Val His
    50                  55                  60

Glu Met Leu Asp Pro Gln Val Asp Leu Val Arg Gly Tyr Ala Asp Glu
65                  70                  75                  80

Val Ala Glu Arg Ile Ser Thr Leu Gly Gly Ala Pro Val Gly Thr Pro
                85                  90                  95

Glu Gly His Val Ala Asp Arg Thr Pro Leu Gln Tyr Glu Arg Asn Ala
            100                 105                 110

Gly Asn Val Gln Ala His Leu Thr Asp Leu Asn Arg Val Tyr Thr Gln
        115                 120                 125

Val Leu Thr Gly Val Arg Glu Ser Met Ala Ser Ala Gly Pro Val Asp
    130                 135                 140

Pro Val Thr Glu Asp Ile Tyr Ile Ser Gln Ala Ala Glu Leu Glu Lys
145                 150                 155                 160

Phe Gln Trp Phe Ile Arg Ala His Ile Val Asp Val Asp Gly Asn Ile
                165                 170                 175

Gln Glu Met Arg Lys Leu Pro Asp Ala
            180                 185

<210>  28
<211>  495
<212>  DNA
<213>  Corynebacterium glutamicum

<220>
<221>  CDS
<222>  (1)..(495)

<400>  28
atg gca aac tac aca gtc cct gga atc aac gag aat gac gca aag cag      48
Met Ala Asn Tyr Thr Val Pro Gly Ile Asn Glu Asn Asp Ala Lys Gln
1               5                   10                  15

ctt att gat gga ctg cag gag cgt ctc acc gac tac aac gat ctt cac      96
Leu Ile Asp Gly Leu Gln Glu Arg Leu Thr Asp Tyr Asn Asp Leu His
            20                  25                  30

ctc atc ttg aag cac gtg cac tgg aac gtc act ggc ccc aac ttc att      144
Leu Ile Leu Lys His Val His Trp Asn Val Thr Gly Pro Asn Phe Ile
        35                  40                  45

gct gtt cac gaa atg ctc gac cca cag gtt gac ctt gtt cgt ggc tat      192
Ala Val His Glu Met Leu Asp Pro Gln Val Asp Leu Val Arg Gly Tyr
    50                  55                  60

gct gac gaa gtt gca gag cgc att tcc acc ctc gga ggc gca cca gtt      240
Ala Asp Glu Val Ala Glu Arg Ile Ser Thr Leu Gly Gly Ala Pro Val
65                  70                  75                  80

gga acc cca gaa ggc cac gtt gct gac cgc acc cca ctg caa tat gag      288
Gly Thr Pro Glu Gly His Val Ala Asp Arg Thr Pro Leu Gln Tyr Glu
                85                  90                  95

cgc aat gcc gga aat gtc caa gca cac ctc act gac ctc aat cgc gtg      336
Arg Asn Ala Gly Asn Val Gln Ala His Leu Thr Asp Leu Asn Arg Val
            100                 105                 110

tac acc caa gtg ctg acc gga gtt cgc gag tcc atg gca tca gcc ggc      384
Tyr Thr Gln Val Leu Thr Gly Val Arg Glu Ser Met Ala Ser Ala Gly
        115                 120                 125

cca gtg gat cca gta act gaa gac atc tac atc agc cag gcc gcg gag      432
Pro Val Asp Pro Val Thr Glu Asp Ile Tyr Ile Ser Gln Ala Ala Glu
    130                 135                 140

ctg gag aaa ttc cag tgg ttc atc cgc gca cac att gtt gat gta gac      480
Leu Glu Lys Phe Gln Trp Phe Ile Arg Ala His Ile Val Asp Val Asp
145                 150                 155                 160

gga aac atc caa gag                                                  495
Gly Asn Ile Gln Glu
                165

<210>  29
<211>  165
<212>  PRT
<213>  Corynebacterium glutamicum

<400>  29

Met Ala Asn Tyr Thr Val Pro Gly Ile Asn Glu Asn Asp Ala Lys Gln
1               5                   10                  15

Leu Ile Asp Gly Leu Gln Glu Arg Leu Thr Asp Tyr Asn Asp Leu His
            20                  25                  30

Leu Ile Leu Lys His Val His Trp Asn Val Thr Gly Pro Asn Phe Ile
        35                  40                  45

Ala Val His Glu Met Leu Asp Pro Gln Val Asp Leu Val Arg Gly Tyr
    50                  55                  60

Ala Asp Glu Val Ala Glu Arg Ile Ser Thr Leu Gly Gly Ala Pro Val
65                  70                  75                  80

Gly Thr Pro Glu Gly His Val Ala Asp Arg Thr Pro Leu Gln Tyr Glu
                85                  90                  95

Arg Asn Ala Gly Asn Val Gln Ala His Leu Thr Asp Leu Asn Arg Val
            100                 105                 110

Tyr Thr Gln Val Leu Thr Gly Val Arg Glu Ser Met Ala Ser Ala Gly
        115                 120                 125

Pro Val Asp Pro Val Thr Glu Asp Ile Tyr Ile Ser Gln Ala Ala Glu
    130                 135                 140

Leu Glu Lys Phe Gln Trp Phe Ile Arg Ala His Ile Val Asp Val Asp
145                 150                 155                 160

Gly Asn Ile Gln Glu
                165


<210>  30
<211>  17
<212>  PRT
<213>  Artificial Sequence

<220>
<223>  Amino acid sequence of peptide of mineralizing zinc oxide

<400>  30

Glu Ala His Val Met His Lys Val Ala Pro Arg Pro Gly Gly Gly Ser
1               5                   10                  15

Cys

<210> 31
<211> 561
<212> DNA
<213> Artificial Sequence

<220>
<223> Nucleotide sequence encoding an amino acid sequence of fusion
      protein

<220>
<221> CDS
<222> (1)..(561)

<400> 31
atg gac tac ttc tct tct ccg tac tac gaa cag ctg ttt atg aaa aca      48
Met Asp Tyr Phe Ser Ser Pro Tyr Tyr Glu Gln Leu Phe Met Lys Thr
1               5                   10                  15

atc aac tca gta gac aca aag gaa ttt ttg aat cat caa gta gcg aat      96
Ile Asn Ser Val Asp Thr Lys Glu Phe Leu Asn His Gln Val Ala Asn
            20                  25                  30

tta aac gta ttc aca gta aaa att cat caa att cat tgg tat atg aga      144
Leu Asn Val Phe Thr Val Lys Ile His Gln Ile His Trp Tyr Met Arg
        35                  40                  45

ggc cac aac ttc ttc act tta cat gaa aaa atg gat gat tta tat agc      192
Gly His Asn Phe Phe Thr Leu His Glu Lys Met Asp Asp Leu Tyr Ser
    50                  55                  60

gaa ttc ggt gaa caa atg gat gaa gta gca gaa cgt tta ctt gcc att      240
Glu Phe Gly Glu Gln Met Asp Glu Val Ala Glu Arg Leu Leu Ala Ile
65                  70                  75                  80

ggt gga agc cca ttc tcg act tta aaa gag ttt tta gaa aat gcg agt      288
Gly Gly Ser Pro Phe Ser Thr Leu Lys Glu Phe Leu Glu Asn Ala Ser
                85                  90                  95

gta gaa gaa gct cct tat aca aaa cct aaa act atg gat caa tta atg      336
Val Glu Glu Ala Pro Tyr Thr Lys Pro Lys Thr Met Asp Gln Leu Met
            100                 105                 110

gaa gac tta gtt ggt aca tta gaa tta ctt aga gac gaa tat aaa caa      384
Glu Asp Leu Val Gly Thr Leu Glu Leu Leu Arg Asp Glu Tyr Lys Gln
        115                 120                 125

ggc att gag cta act gac aaa gaa ggc gac gat gta aca aac gat atg      432
Gly Ile Glu Leu Thr Asp Lys Glu Gly Asp Asp Val Thr Asn Asp Met
    130                 135                 140

cta att gca ttt aaa gct agc att gac aaa cat atc tgg atg ttc aaa      480
Leu Ile Ala Phe Lys Ala Ser Ile Asp Lys His Ile Trp Met Phe Lys
145                 150                 155                 160

gca ttc ctt gga aaa gct cca tta gaa gaa gca cat gta atg cat aaa      528
Ala Phe Leu Gly Lys Ala Pro Leu Glu Glu Ala His Val Met His Lys
                165                 170                 175

gtt gct cca cgt cct ggt gga ggt agt tgt taa                          561
Val Ala Pro Arg Pro Gly Gly Gly Ser Cys

180                 185

<210>  32
<211>  186
<212>  PRT
<213>  Artificial Sequence

<220>
<223>  Synthetic Construct

<400>  32

Met Asp Tyr Phe Ser Ser Pro Tyr Tyr Glu Gln Leu Phe Met Lys Thr
1               5                   10                  15

Ile Asn Ser Val Asp Thr Lys Glu Phe Leu Asn His Gln Val Ala Asn
            20                  25                  30

Leu Asn Val Phe Thr Val Lys Ile His Gln Ile His Trp Tyr Met Arg
        35                  40                  45

Gly His Asn Phe Phe Thr Leu His Glu Lys Met Asp Asp Leu Tyr Ser
    50                  55                  60

Glu Phe Gly Glu Gln Met Asp Glu Val Ala Glu Arg Leu Leu Ala Ile
65                  70                  75                  80

Gly Gly Ser Pro Phe Ser Thr Leu Lys Glu Phe Leu Glu Asn Ala Ser
                85                  90                  95

Val Glu Glu Ala Pro Tyr Thr Lys Pro Lys Thr Met Asp Gln Leu Met
            100                 105                 110

Glu Asp Leu Val Gly Thr Leu Glu Leu Leu Arg Asp Glu Tyr Lys Gln
        115                 120                 125

Gly Ile Glu Leu Thr Asp Lys Glu Gly Asp Asp Val Thr Asn Asp Met
    130                 135                 140

Leu Ile Ala Phe Lys Ala Ser Ile Asp Lys His Ile Trp Met Phe Lys
145                 150                 155                 160

Ala Phe Leu Gly Lys Ala Pro Leu Glu Glu Ala His Val Met His Lys
                165                 170                 175

Val Ala Pro Arg Pro Gly Gly Gly Ser Cys
            180                 185

<210>  33
<211>  80
<212>  DNA
<213>  Artificial Sequence

<220>
<223>  Primer

<400>  33
tttggatcct taacaactac ctccaccagg acgtggagca actttatgca ttacatgtgc      60

ttcttctaat ggagcttttc                                                  80